# EUROPÄISCHE PATENTSCHRIFT

(11) **EP 3 092 977 B1**
(45) Veröffentlichungstag und Bekanntmachung des Hinweises auf die Patenterteilung: **11.09.2019**
(21) Anmeldenummer: 16167165.6
(22) Anmeldetag: 26.04.2016
(51) Int. Cl.: A61F 2/28, A61F 2/30

(54) **FLÄCHIGES KNOCHENERSATZMATERIAL UND VERFAHREN ZUR HERSTELLUNG EINES PORÖSEN KÖRPERS**
PLANAR BONE REPLACEMENT MATERIAL AND METHOD FOR PRODUCING A POROUS BODY
MATERIAU PLAT DE REMPLACEMENT OSSEUX ET PROCEDE DE PRODUCTION D'UN CORPS POREUX

(30) Priorität: 13.05.2015 DE 102015107599
(43) Veröffentlichungstag der Anmeldung: 16.11.2016
(73) Patentinhaber: Heraeus Medical GmbH, 61273 Wehrheim (DE)
(72) Erfinder: Vogt, Sebastian Dr., 99092 Erfurt (DE)
(74) Vertreter: Schultheiss & Sterzel Patentanwälte PartG mbB

(56) Entgegenhaltungen:
- EP-A1- 2 832 320
- WO-A1-2014/006519
- DE-B3-102015 110 202
- FR-A1- 2 990 860
- US-A1- 2003 055 511

## Beschreibung

Die Erfindung betrifft ein alloplastisches Knochenersatzmaterial. Die Erfindung betrifft auch ein Verfahren zur Herstellung eines porösen Körpers aus einem alloplastischen Knochenersatzmaterial.

Gegenstand der Erfindung ist somit ein alloplastisches Knochenersatzmaterial, das zum Auffüllen und Stabilisieren von Knochenkavitäten bestimmt ist. Ferner wird ein Verfahren zur Herstellung eines frei geformten porösen Körpers vorgeschlagen.

Knochenersatzmaterialien sind seit langem bekannt und werden umfangreich klinisch genutzt (J. M. Rueger: Knochenersatzmittel, Orthopäde 27 (1998) 72-79.). Die bisher verwendeten Knochenersatzmaterialien sind im Allgemeinen volumenstabil aber nicht formstabil. Eine Ausnahme bildet ein Knochenersatzmaterial, das unter der Bezeichnung "Trabecular metal™" von der Firma Zimmer vertrieben wird und das beispielsweise aus der WO 2013/074 909 A1 bekannt ist. Dieses Material hat eine poröse Struktur, die der Struktur der humanen Spongiosa (Schwammgewebe) nachgebildet ist. Dieses Material besteht aus Tantal und ist in definierten Formen und Größen handelsüblich. Das Material ist im OP nicht in seiner Form und Größe veränderbar. Es kann nicht mit konventionellen Werkzeugen im OP bearbeitet werden. Die jeweilige anatomische Situation des Patienten kann daher nur bedingt berücksichtigt werden. Der medizinische Anwender kann nur versuchen, dass Implantatlager an die vorgegebene Geometrie anzupassen oder ein annähernd passendes Implantat einzusetzend und die bestehenden Lücken mit allogenem Knochenmaterial oder anderem Volumenfüllern zu schließen.

Die EP 2 832 320 A1 offenbart ein Knochenimplantatsystem mit mehreren Knochenimplatatmodulen, wobei das ein erstes Knochenimplatatmodul eine feine Kopplungsstruktur und das zweite Knochenimplatatmodul eine grobe Kopplungsstruktur, so dass diese ineinander greifen können. Aus der WO 2014/006 519 A1 ist ein Produkt zur Herstellung einer porösen Struktur bekannt, bei dem gleichartige Formen aufeinandergelegt und aneinander befestigt werden.

Besonders bei der Rekonstruktion von Pfannendachdefekten im Rahmen von septischen Revisionen von infizierten Hüftgelenksprothesen ist es extrem problematisch, die fehlende Knochensubstanz mechanisch stabil zu ersetzen und zu überbrücken, so dass sogenannte Revisions-Acetabulum-Cups implantiert werden können.

Die Aufgabe der Erfindung besteht also darin, die Nachteile des Stands der Technik zu überwinden. Insbesondere soll ein Knochenersatzmaterial zur Augmentation entwickelt werden, das zur Auffüllung und Überbrückung von Knochendefekten geeignet ist und zu diesem Zweck geformt werden kann. Das Knochenersatzmaterial soll nach dem Ausformen einen formstabilen porösen Körper bilden, ohne dass chemische Aushärtungsreaktionen, wie beispielsweise radikalische Polymerisationen, notwendig sind. Das Knochenersatzmaterial soll nach der Formung eine offene Porosität besitzen und mechanisch stabil sein. Die Porosität und die Größe der Poren sollen dabei dazu ausreichen und dazu geeignet sein, dass menschlicher Knochen eines Patienten, der mit dem Knochenersatzmaterial behandelt wird, in die Poren des Knochenersatzmaterials einwachsen kann. Es wird ferner angestrebt, dass das Knochenersatzmaterial im geformten Zustand möglichst lastragend ist. Weiterhin muss das Knochenersatzmaterial biokompatibel sein, damit es im Körper eines Patienten eingesetzt werden kann.

Die Aufgaben der Erfindung werden gelöst durch ein flächiges alloplastisches Knochenersatzmaterial aufweisend zumindest eine Platte, bevorzugt aufweisend mehrere Platten, zur Augmentation von Knochendefekten, wobei das Knochenersatzmaterial aus einem biokompatiblen Kunststoff, einem biokompatiblen Metall und/oder einer biokompatiblen Metalllegierung besteht, wobei die zumindest eine Platte ein Flächengebilde aufweist und eine Mehrzahl von sich aus dem Flächengebilde der zumindest einen Platte heraus erstreckende Stifte aufweist, wobei die Stifte jeweils mindestens ein Verbindungselement aufweisen, wobei die Stifte elastisch verformbar sind und derart dicht zueinander angeordnet sind, so dass durch Aufeinanderpressen der Flächen mehrerer Platten die gleichen Verbindungselemente verschiedener Platten miteinander verhaken und/oder rasten und die miteinander verhakten und/oder gerasteten Platten einen offenporigen Körper aus miteinander verhakten und/oder gerasteten Platten bilden.

Biokompatible Metalle oder biokompatible Metalllegierungen werden erfindungsgemäß bevorzugt, um daraus die Platten des Knochenersatzmaterials herzustellen.

Bei einem Verhaken greifen Vorsprünge der Verbindungselemente der Stifte der Platten in Vorsprünge, Greifflächen oder Hinterschnitte von Verbindungselementen an Stiften benachbarter Platten, so dass anschließend die Stifte noch durch ein weiteres Zusammendrücken der Platten gegeneinander beweglich sind aber nicht mehr voneinander getrennt werden können. Bei einem Rasten greifen die Verbindungselemente der Stifte der Platten derart in Verbindungselemente von Stiften benachbarter Platten, dass die Platten nicht mehr voneinander getrennt werden können aber auch nicht mehr durch weiteres Zusammendrücken der Platten aufeinander zu bewegt werden können. Die Verbindungselemente können also durch Haken, Nuten, Hinterschnitte, Rastungen und/oder Gegenrastungen beziehungsweise durch Haken, Nuten, Hinterschnitte und/oder Rastelemente gebildet werden.

Die zumindest eine Platte und der aus mehreren Platten gebildete Volumenkörper sind erfindungsgemäß bevorzugt osteokonduktiv.

Theoretisch kann eine einzelne Platte ausreichen, die beispielsweise einfach oder mehrfach gefaltet werden kann, um den gewünschten Volumenkörper herzustellen. Erfindungsgemäß bevorzugt umfasst das Knochenersatzmaterial aber mehrere Platten, die aneinander gefügt werden können. Besonders bevorzugt umfasst das Knochenersatzmaterial mehrere unterschiedlich geformte Platten, die aneinander gefügt werden können. Die Auswahl der Form der Platten richtet sich dabei nach der Behandlungssituation.

Unter dem Begriff "flächig" werden plane Körper und von planen Körpern abgeleitete Körper verstanden, die aus jeweils einem geschlossenen oder bevorzugt aus einem perforierten plattenartigen Grundkörper gebildet sind. Perforierte Flächengebilde sind dabei bevorzugt, weil durch diese Perforationen beziehungsweise die durch die Perforation gebildeten Poren Knochengewebe in die Fläche einwachsen kann. Ganz besonders vorteilhaft und erfindungsgemäße bevorzugt ist es, wenn neben jedem Stift eine Perforation beziehungsweise eine Pore in der Platte angeordnet ist. Dann bildet sich nach der Verhakung und/oder der Rastung von mehreren Platten, das heißt von mehreren flächigen Augmentationsmaterialien ein offen poröser Körper, der bei geeigneter Materialwahl, wie zum Beispiel von Tantal, osteokonduktiv ist.

Bei Druckeinwirkung auf die miteinander in Berührung stehenden Platten des Knochenersatzmaterials bilden diese erfindungsgemäß einen mechanisch stabilen Verbund aus.

Bei erfindungsgemäßen Knochenersatzmaterialien kann vorgesehen sein, dass die Verbindungselemente Pilze, Haken, Hinterschnitte und/oder Rastelemente sind, vorzugsweise Pilze, Haken, Hinterschnitte, Rastungen und/oder Gegenrastungen sind.

Diese Verbindungselemente sind besonders gut zur gegenseitigen Rastung beziehungsweise Verhakung geeignet. Textile Verbindungen, wie beispielsweise Klettverbindungen mit leicht verformbaren Fasern, sind dagegen erfindungsgemäß ungeeignet, da damit keine formstabilen und druckstabilen Körper aufbaubar sind.

Es kann auch vorgesehen sein, dass der Abstand zwischen den Verbindungselementen und dem Flächengebilde der zumindest einen Platte zwischen 0,3 mm und 2 mm beträgt, bevorzugt zwischen 0,5 mm und 1 mm beträgt.

Hierdurch kann sichergestellt werden, dass die zwischen den verhakten oder gerasteten Verbindungselementen frei bleibenden Poren einen ausreichenden Durchmesser für eine offenporige Struktur aufweisen. Es kann dadurch also erreicht werden, dass der entstehende Volumenkörper aus dem Knochenersatzmaterial osteokonduktiv ist.

Ferner kann vorgesehen sein, dass die Stifte der zumindest einen Platte sich senkrecht oder mit einem Winkel zwischen 60° und 90°, bevorzugt mit einem Winkel zwischen 80° und 90°, aus dem Flächengebilde der zumindest einen Platte erstrecken.

Hierdurch wird erreicht, dass sich die Platten später besonders leicht miteinander verbinden lassen. Zudem wird so eine gleichmäßige Belastbarkeit des Knochenersatzmaterials erreicht.

Es wird auch vorgeschlagen, dass die Verbindungselemente an der Mantelfläche der Stifte ausgebildet sind.

Damit kann eine stabile Verbindung der Stifte und damit der Platten untereinander erreicht werden.

Bevorzugte Knochenersatzmaterialien können vorsehen, dass ineinander gepresste Platten irreversibel miteinander verhaken und/oder rasten.

Hierdurch wird sichergestellt, dass sich keine Partikel des fertig geformten Knochenersatzmaterials von dem gebildeten Körper lösen. Dadurch werden Irritationen des behandelten Körpers an der behandelten Stelle verhindert.

Mit einer Weiterbildung der vorliegenden Erfindung wird vorgeschlagen, dass die zumindest eine Platte ohne die hervorragenden Stifte eine Stärke von höchstens 1,5 mm aufweist, bevorzugt einen Stärke zwischen 0,25 mm und 1,5 mm aufweist, besonders bevorzugt einen Stärke zwischen 0,5 mm und 1 mm aufweist.

Die Stärke der zumindest einen Platte kann auch als die Dicke der Platte bezeichnet werden und ist die Abmessung der Platte ohne die Stifte, die senkrecht zur dem Flächengebilde der Platte angeordnet ist. Damit wird erreicht, dass die Platten ausreichend stark gebogen beziehungsweise verformt werden können, um an die Behandlungssituation angepasst zu werden.

Es kann des Weiteren vorgesehen sein, dass die zumindest eine Platte mit einem generativen 3D-Druckverfahren hergestellt wird.

Hierdurch lassen sich die Platten und damit das Knochenersatzmaterial kostengünstig herstellen.

Gemäß einer bevorzugten Ausführungsform kann vorgesehen sein, dass zumindest eines der zumindest einen Verbindungselemente pro Stift stumpf-kegelförmig ausgebildet ist, wobei die Längsachsen der Stifte die Längsachsen der Kegel bilden und wobei der Mantel der Kegel nach der zu dem Flächengebilde der zumindest einen Platte abgewandten Außenseite gerichtet ist.

Hierdurch wird eine besonders stabile Verbindbarkeit der zumindest einen Platte über die als Kegelstümpfe geformten Verbindungselemente erreicht. Zudem wird durch diese Formgebung nach der Implantation des Knochenersatzmaterials verhindert, dass umgebendes Weich- und Knochengewebe verletzt wird.

Es kann ferner vorgesehen sein, dass zumindest eines der zumindest einen Verbindungselemente pro Stift als Haken und/oder als Pilzkopf ausgebildet ist.

Die Haken und/oder die Pilzköpfe sorgen für eine stabile und unlösbare Verbindung der Platten untereinander. Wenn die Verbindungselemente als Pilzköpfe geformt sind, können diese zum Beispiel einen in Richtung des Flächengebildes ausgebildeten Kragen am Pilzkopfrand besitzen, so dass in diesem so erzeugten Hinterschnitt hakenförmige Verbindungselemente von anderen Platten eingreifen können, wodurch eine irreversible, nicht lösbare Verhakung oder Rastung zwischen den Platten entsteht. Es ist auch möglich und erfindungsgemäß bevorzugt, dass zumindest eine Platte verschiedene Verbindungselemente enthält oder verschiedene Stifte mit unterschiedlichen Verbindungselementen aufweist. So kann eine Platte zum Beispiel Haken und Pilzköpfe als Verbindungselemente gleichzeitig besitzen sowohl am gleichen Stift als auch an unterschiedlichen Stiften.

In einer bevorzugten Ausführungsform sind die Verbindungselemente als Pilzköpfe ausgebildet. In einer besonders bevorzugten Ausführungsform sind die Pilzköpfe so gestaltet, dass die Pilzköpfe an der der Oberfläche der zumindest einen Platte zugewandten Seite einen kegelförmigen Hinterschnitt aufweisen. Dadurch können hakenförmige Rastelemente mit diesen Pilzköpfen irreversibel und unlösbar verhakt werden. Bei geeigneter zueinander passender Form der Hinterschnitte zu den Pilzköpfen kann auch ein weiteres Vortreiben der Pilzköpfe verhindert werden, so dass die Pilzköpfe mit den Hinterschnitten rasten.

Besonders bevorzugte Knochenersatzmaterialien können auch vorsehen, dass die Stifte zwischen dem Flächengebilde einer Platte und zumindest einem der zumindest einen Verbindungselemente eine umlaufende Nut als zusätzliches Verbindungselement enthalten, in welche Verbindungselemente anderer Platten einhaken oder einrasten können, bevorzugt derart einrasten, dass keine weitere Bewegung der Verbindungselemente entlang der Stifte möglich ist.

Auch hierdurch wird eine besonders stabile Verbindung von Platten untereinander ermöglicht. Vorteilhaft ist dabei ferner, dass dadurch definierte und freibleibende Hohlräume als offene Poren nach der Rastung der Platten in dem so aus dem Knochenersatzmaterial geformten Körper erreicht werden. Es wird nämlich ein weiteres verschließen der offenporigen Struktur durch ein weiteres vorantreiben der Stifte zwischen den Stiften einer angefügten Platte verhindert und so die Poren offen gehalten.

Mit einer Weiterbildung der vorliegenden Erfindung wird vorgeschlagen, dass zumindest zwei Verbindungselemente hintereinander auf der Mantelfläche der Stifte angeordnet sind, besonders bevorzugt zumindest drei Verbindungselemente hintereinander auf der Mantelfläche der Stifte angeordnet sind.

Hierdurch wird es möglich, Platten mit unterschiedlichen Abständen zueinander zu verhaken und/oder zu rasten. Dadurch wird eine höhere Flexibilität beim Formen des Knochenersatzmaterials erreicht.

Es kann auch vorgesehen sein, dass die zumindest eine Platte in Form eines Quadrats, eines Rechtecks, eines Trapezes, eines Parallelogramms und/oder in Form eines Vielecks ausgebildet ist.

Durch diese Formen können die Körper besonders gut an die zu füllenden beziehungsweise zu behandelnden Knochen aus dem Knochenersatzmaterial angepasst werden.

Des Weiteren kann vorgesehen sein, dass in dem Flächengebilde der zumindest einen Platte durchgehende Poren enthalten sind, wobei die Poren abgerundet sind, insbesondere keine scharfkantigen Konturen aufweisen, wobei vorzugsweise die Poren in dem Flächengebilde der Platte einen freien Querschnitt zwischen 0,25 mm und 1 mm aufweisen, besonders bevorzugt zwischen 0,3 mm und 0,9 mm.

Hierdurch wird sichergestellt, dass die Poren sich in jeder Richtung in den gebildeten Volumenkörper erstrecken, so dass das Knochenwachstum insbesondere in das Flächengebilde der zumindest einen Platte hinein erfolgen kann.

Ebenso kann vorgesehen sein, dass in dem Flächengebilde der zumindest einen Platte durchgehende Poren enthalten sind, wobei die Tiefe der Poren senkrecht zu dem Flächengebilde der zumindest einen Platte zumindest 0,25 mm beträgt, bevorzugt zumindest 0,4 mm beträgt.

Hierdurch kann sichergestellt werden, dass die Poren stabil und gleichförmig vom Knochen umschlossen werden. Die Platten bilden so ein spongiöses Geweben nach, dass einer normalen Knochenstruktur entspricht und mit dieser gut verwachsen kann.

Ferner kann vorgesehen sein, dass die zumindest eine Platte aus biokompatiblem Kunststoff, Edelstahl, Titan, einer Titanlegierung, Tantal, einer Tantallegierung oder aus Kompositen dieser Materialien gebildet ist.

Diese Materialien sind für medizinische Zwecke besonders gut einsetzbar und mit diesen Materialien lassen sich auch die geeigneten elastischen Eigenschaften für die Stifte einstellen. Aus Metall oder Metalllegierungen bestehende Platten können erfindungsgemäß bevorzugt durch selektives Lasersintern oder auch durch Schmelzen mit Elektronenstrahlen hergestellt werden, bevorzugt mit einem 3D-Druckverfahren.

Der biokompatible Kunststoff kann biodegradierbar sein. Dazu können Polylactide, Polylglycolide, Polycaprolactone und Polyester verwendet werden, die aus unterschiedlichen α-Hydroxycarbonsäuren gebildet sind. Als nicht biodegradierbare Kunststoffe kommen Polyamide, Polyimide, Polyetherketon und Polysulfon in Betracht. Platten aus diesen nicht biodegradierbaren und biodegradierbaren Kunststoffen können durch selektives Lasersintern hergestellt werden.

Gemäß einer Weiterbildung der vorliegenden Erfindung kann vorgesehen sein, dass benachbarte Stifte, die auf der gleichen Seite einer ersten Platte der zumindest einen Platte angeordnet sind, einen derartigen Abstand zueinander aufweisen, dass nach einer elastischer Deformation aufgrund einer Verhakung und/oder Rastung mit einem Verbindungselement einer zweiten Platte der zumindest einen Platte die Stifte der erste Platte mindestens zwei Verhakungen und/oder Rastungen mit zumindest zwei weiteren Verbindungselementen der zweiten Platte ermöglichen, bevorzugt mindestens drei Verhakungen und/oder Rastungen mit drei weiteren Verbindungselementen der zweiten Platte ermöglichen.

Durch eine mehrfache Verhakungen und/oder Rastung der Platten kann ein besonders stabiler Körper aus dem Knochenersatzmaterial geformt werden.

Bevorzugt kann ferner vorgesehen sein, dass die Platte oder wenigstens eine der Platten nur auf einer Seite Stifte aufweist und auf der anderen Seite flächig an einem Knochen zu befestigen ist, wobei vorzugsweise hierzu scharfe Spitzen vorgesehen sind, die in den Knochen eingedrückt werden können, oder in dem Flächengebilde der zumindest einen Platte Ösen oder Bohrungen vorgesehen sind, durch die die zumindest eine Platte auf einen Knochen geschraubt oder anders befestigt werden kann.

Eine solche Platte kann zur unmittelbaren flächigen Befestigung am Knochen verwendet werden. Mit den Ösen oder Bohrungen ist es möglich, das Knochenersatzmaterial beziehungsweise die Platte am Knochengewebe anzuschrauben und auf diese Platte beliebig viele weitere Platten des Knochenersatzmaterials aufzubringen und anschließend zu verhaken und/oder zu rasten. Dadurch lassen sich komplexe dreidimensionale Strukturen, wie sie zum Beispiel bei Pfannendachdefekten auftreten, lastragend auffüllen. Auf diese Strukturen können auch Acetabulum-Cups zementfrei oder mit Knochenzement verankert werden.

Auch wenn beide Seiten der zumindest einen Platte Stifte aufweisen, können scharfe Spitzen zur Befestigung an der Knochenoberfläche vorgesehen sein, die Spitzen sollten hierzu die Stifte mit den Verbindungselementen überragen.

Bei Ausführungsformen mit scharfen Spitzen kann das flächige Knochenersatzmaterial durch Einschlagen der scharfen Spitzen in das Knochengewebe verankert werden. Auf diesem aufbauend können dann beliebige Schichten beziehungsweise Platten des Knochenersatzmaterials aufgebracht und verhakt werden.

Es kann auch vorgesehen sein, dass die zumindest eine Platte oder wenigstens eine der zumindest einen Platten auf beiden Seiten Stifte aufweist.

Mit derartigen Platten kann ein schichtweiser Aufbau des Volumenkörpers aus Knochenersatzmaterial erfolgen.

Es wird des Weiteren vorgeschlagen, dass die zumindest eine Platte mit anorganischem oder organischem partikulärem Knochenersatzmaterial und/oder autologer oder auch allogener Spongiosa vermischt sind.

Hiermit kann die Knochenheilung und die Verbindung des Knochenersatzmaterials mit dem Knochen beschleunigt werden.

Bevorzugt kann auch vorgesehen sein, dass die zumindest eine Platte mit einem oder mehreren pharmazeutischen Wirkstoffen aus den Gruppen der Antibiotika, der Bisphosphonate, der Steroide, der nichtsteroidalen Entzündungshemmer, der Wachstumsfaktoren und der Cytostatika beschichtet sind.

Hiermit wird eine pharmakologische Wirkung des Knochenersatzwerkstoffs erreicht, die zur Heilung des mit dem Knochenersatzmaterial behandelten Patienten beiträgt. Aus der Gruppe der Antibiotika sind besonders Gentamicin, Tobramycin, Amikacin, Vancomycin, Teicoplanin, Clindamycin und Daptomycin bevorzugt.

Gemäß einer weiteren Ausführung kann vorgesehen sein, dass die zumindest eine Platte oder wenigstens eine davon als halbkugelförmige Schale oder gewölbte Fläche oder als Bogen oder als Wanne ausgebildet ist.

Hierdurch können die Platten bereits an spezielle Behandlungssituationen angepasst sein und eignen sich so besonders gut zur Ausbildung von gewölbten Strukturen.

Des Weiteren wird mit der Erfindung vorgeschlagen, dass die Poren des aus mehreren Platten gebildeten offenporigen Körpers interkonnektierend und osteokonduktiv sind, wobei vorzugsweise die Poren einen freien Querschnitt zwischen 0,1 mm und 1 mm aufweisen, besonders bevorzugt zwischen 0,25 mm und 0,9 mm.

Hiermit wird sichergestellt, dass der Knochen gut mit den Poren des aus dem Knochenersatzmaterial gebildeten Körpers verwachsen kann.

Es kann auch vorgesehen sein, dass die zumindest eine Platte in dem Flächengebilde plastisch oder elastisch verformbar ist.

Dadurch kann die zumindest eine Platte besonders leicht an verschiedene Behandlungssituationen angepasst werden.

Mit einer Weiterbildung der vorliegenden Erfindung wird vorgeschlagen, dass die Stifte mit Verbindungselementen in Reihen von jeweils drei oder mehreren Stiften angeordnet sind und dass zwischen diesen jeweils drei oder mehrere Reihen ein Streifen von unbesetzter Oberfläche der zumindest einen Platte verbleibt oder dass eine gruppierte oder nestförmige Anordnung von Stiften mit Verbindungselementen vorgesehen ist.

Hierdurch wird Raum für die Verformung der Stifte mit den Verbindungselementen bei der Verhakung und/oder Rastung gegeben.

Gemäß einer bevorzugten Weiterbildung der vorliegenden Erfindung kann auch vorgesehen sein, dass das Knochenersatzmaterial neben der zumindest einen Platte zusätzlich zumindest einen Partikel aufweist, wobei der zumindest eine Partikel einen Kern und mindestens sechs sich vom Kern erstreckende Stifte aufweist, wobei die Stifte jeweils mindestens ein Verbindungselement aufweisen, das analog der Verbindungselemente der zumindest einen Platten ausgebildet ist, so dass die zumindest eine Platte mit dem zumindest einen Partikel durch Aufeinanderpressen die Verbindungselemente der zumindest einen Platte und des zumindest einen Partikels miteinander verhaken und/oder rasten und die miteinander verhakten und/oder gerasteten Platte(n) und Partikel einen offenporigen Körper aus miteinander verhakten und/oder gerasteten Platte(n) und Partikel(n) bilden.

Dadurch wird ein noch variabler nutzbares Knochenersatzmaterial erzielt, das sowohl frei geformt, als auch zur Überbrückung von Spalten und Kavitäten genutzt werden kann.

Die der vorliegenden Erfindung zugrundeliegenden Aufgaben werden auch gelöst durch ein Verfahren zum Formen eines Körpers aus einem erfindungsgemäßen flächigen alloplastischen Knochenersatzmaterial mit mehreren Platten, bei dem mehrere Platten gegeneinander gedrückt werden, wobei die Platten miteinander verhaken und/oder rasten und einen offenporigen Körper ausbilden.

Bei diesem Verfahren kann auch vorgesehen sein, dass die Stifte mit den Verbindungselementen von mindestens zwei Platten in Kontakt miteinander gebracht werden und dass anschließend durch Druck der Platten gegeneinander die Stifte mit den Verbindungselementen miteinander verhakt und/oder gerastet werden.

Bei dem Verfahren kann auch vorgesehen sein, dass zumindest eine der Platten mit einem porösen Volumenkörper eines zweiten Knochenersatzmaterials verbunden werden, indem die Verbindungselemente mit den Poren des zweiten Knochenersatzmaterials rasten und/oder verhaken, und/oder zumindest eine der Platten mit einem partikulären dritten Knochenersatzmaterial aufweisend mehrere Partikel verbunden werden, wobei die Partikel des dritten Knochenersatzmaterials eine Mehrzahl von sich von einem Kern der Partikel erstreckenden Stiften mit Verbindungelementen aufweist, wobei bevorzugt die Stifte und die Verbindungselemente der Partikel des dritten Knochenersatzmaterials die Merkmale der Stifte und Verbindungselemente der zumindest einen Platte des erfindungsgemäßen Knochenersatzmaterials aufweisen.

Der poröse Volumenkörper des zweiten Knochenersatzmaterials kann beispielsweise ein Trabecular metal™ von der Firma Zimmer sein.

Die der zugrundeliegenden Aufgaben werden auch gelöst durch die Verwendung des erfindungsgemäßen flächigen alloplastischen Knochenersatzmaterials als Implantatmaterial in der Unfallchirurgie, Orthopädie oder in der Veterinärmedizin.

Bei Druckeinwirkung auf die miteinander in Berührung stehenden Platten des Knochenersatzmaterials bilden diese erfindungsgemäß einen mechanisch stabilen Verbund aus.

Der Erfindung liegt die überraschende Erkenntnis zugrunde, dass mechanisch rastende beziehungsweise verhakende Platten als alloplastisches Knochenersatzmaterial verwenden werden können. Dabei können die Platten schichtweise oder auch nur teilweise überlappend angeordnet werden, wobei der so geformte poröse Volumenkörper nach Ausbildung der gewünschten dreidimensionalen Struktur fest ist, ohne dass chemische Aushärtungsreaktionen, wie beispielsweise radikalische Polymerisationen, notwendig sind. Die Platten sind bevorzugt in begrenztem Umfang flexibel und können so in eine passende Form gebracht werden und durch Druck miteinander rasten und dabei miteinander verbunden werden. Wenn die geformten Platten miteinander rasten und/oder verhaken stabilisieren sich die Platten untereinander, so dass der entstehende dreidimensionale Volumenkörper aus dem Knochenersatzmaterial fest und formstabil ist. Indem die Platten durch Aneinanderdrücken aus verschiedenen Richtungen mit ausreichender Kraft miteinander verbunden werden, kann sichergestellt werden, dass derart viele Verhakungen und Rastungen erfolgen, dass der erzeugte offenporige Volumenkörper Formstabil und mechanisch belastbar ist. Durch eine geeignete Form und Größe der Platten wird so ein poröses Knochenersatzmaterial gebildet, das für die medizinische Anwendung mechanisch stabil genug ist. Der Knochen kann in die Poren des durch Druck verbundenen Knochenersatzmaterials einwachsen und sich so mit dem Knochenersatzmaterial dauerhaft verbinden.

Überraschend wurde gefunden, dass das erfindungsgemäße flächige Knochenersatzmaterial in Form von Platten an verschiedenartig geformte Oberflächen schichtweise angelegt werden kann und durch einfaches Komprimieren per Hand oder mit Hilfe eines Stößels zu einem porösen, aber homogenen Körper unter Verhakung und/oder Rastung der einzelnen Schichten des flächigen Augmentationsmaterials beziehungsweise Knochenersatzmaterials ausgehärtet werden kann. Damit wird ein Vorteil gegenüber dem bisherigen Knochenersatzmaterial "Trabecular Metal™" erreicht, dessen Form und Größe nicht frei vom medizinischen Anwender bestimmt werden kann. Es ist damit möglich, beliebig geformte Knochendefekte mit einem in situ aushärtenden beziehungsweise festwerdenden Augmentationsmaterial aufzufüllen oder zu überbrücken, ohne dass chemische Reaktionen, wie zum Beispiel radikalische Polymerisationen, notwendig sind. Eine Aushärtung des erfindungsgemäßen Knochenersatzmaterials ist durch einfaches Komprimieren von über die Flächen aneinander liegender Platten möglich. Eine lasttragende Augmentation ist mit dem erfindungsgemäßen flächigen Augmentationsmaterial möglich.

Mechanisch verhakende Systeme nach dem Bauprinzip von Kletten sind seit mehreren Jahrzehnten bekannt. Erstmalig wurde das Prinzip des Klettverschlusses von de Mestral in CH 295 638 A beschrieben. Dieses Prinzip wurde weiterentwickelt und findet bei unterschiedlichsten, reversibel schließenden Klettverschlüssen Verwendung. Beispielhaft für die Weiterentwicklung sind die Schriften DE 1 610 318 A1, DE 1 625 396 A1, US 5 077 870 A und US 4 290 174 A.

Eine interessante Weiterentwicklung erfolgte später, bei der reversibel verklettende Stahlbandsysteme für mechanisch hochbelastbare Anwendungen und für Anwendungen bei hohen Temperaturen entwickelt wurden (DE 10 2004 048 464 A1, DE 10 2006 015 100 A1, DE 10 2006 015 145 A1, DE 10 2006 015 148 A1).

Es wurde im Rahmen der vorliegenden Erfindung überraschend gefunden, dass solche Systeme beziehungsweise derartige Funktionsprinzipien für Knochenersatzmaterialien einsetzbar beziehungsweise auf Knochenersatzmaterialien übertragbar sind. Dabei ist es für die Knochenersatzmaterialien von Vorteil, dass derartige Verbindungen nicht dicht abschließen, sondern Zwischenräume als offenporige Struktur verbleiben. Die sich dadurch im Volumenkörper bildenden interkonnektierenden Poren können mit dem Knochen verwachsen und damit eine stabile Verbindung zwischen dem Knochen und dem Knochenersatzmaterial erzeugen. Hierzu muss sichergestellt werden, dass die Poren in dem Knochenersatzmaterial einen ausreichenden freien Querschnitt aufweisen. Die Poren werden als osteokonduktiv bezeichnet, wenn der Knochen in die Poren einwachsen kann und sich somit mit dem aus dem Knochenersatzmaterial gebildeten Körper verbindet.

Ein beispielhaftes und erfindungsgemäß besonders bevorzugtes Ausführungsbeispiel der vorliegenden Erfindung ist ein flächiges Augmentationsmaterial, bei dem auf mindestens einer Oberfläche eines aus biokompatiblen Metall oder Metalllegierungen gebildeten Flächengebildes drei oder mehrere elastisch-verformbare Stifte angeordnet sind, die an einem Stiftende jeweils mindestens ein Rastelement besitzen und wobei die mindestens drei oder mehreren Stifte so dicht beieinander stehen, dass bei Kontakt der Stifte von mindestens zwei Flächengebilden diese unter Druckeinwirkung miteinander verhaken und/oder rasten und so einen Verbund der flächigen Augmentationsmaterialien ausbilden.

Der Aufbau der Platten wird dabei derart gestaltet und dass durch Zusammenpressen der Platten sich kontaktierende Platten irreversibel miteinander rasten oder verhaken und einen offenporigen Körper aus miteinander verhakten oder gerasteten Platten bilden.

Im Folgenden werden weitere Ausführungsbeispiele der Erfindung anhand von siebenunddreißig schematisch dargestellten Figuren erläutert, ohne jedoch dabei die Erfindung zu beschränken. Dabei zeigt:
Figur 1: eine schematische perspektivische Ansicht auf fünf teilweise unter einander verbundene Platten eines erfindungsgemäßen Knochenersatzmaterials;
Figur 2: fünf schematische Ansichten der beiden unteren Platten nach Figur 1, A) perspektivische Ansicht, B) Seitenansicht, C) Aufsicht auf die Oberseite, D) Sicht auf die Unterseite, E) Querschnittansicht entlang des Schnitts A-A nach Figur 2 B);
Figur 3: drei schematische Ansichten der drei oberen Platten nach Figur 1, A) perspektivische Ansicht, B) Seitenansicht, C) Aufsicht auf die Oberseite;
Figur 4: eine schematische perspektivische Ansicht auf drei unterschiedlich tief ineinander verhakte Platten des erfindungsgemäßen Knochenersatzmaterials nach den Figuren 1 bis 3;
Figur 5: eine schematische Seitenansicht auf zwei über die Verbindungselemente miteinander verhakte Platten des erfindungsgemäßen Knochenersatzmaterials nach den Figuren 1 bis 4;
Figur 6: eine schematische Seitenansicht auf zwei über die Verbindungselemente und über das Flächengebilde miteinander verhakte Platten des erfindungsgemäßen Knochenersatzmaterials nach den Figuren 1 bis 5;
Figur 7: eine schematische Querschnittansicht der zwei über die Verbindungselemente und über das Flächengebilde miteinander verhakten Platten nach Figur 6;
Figur 8: vier schematische Ansichten von miteinander verbundenen Platten eines zweiten alternativen Knochenersatzmaterials, A) perspektivische Ansicht, B) Seitenansicht, C) Aufsicht auf die Oberseite, D) Querschnittansicht entlang des Schnitts C-C nach Figur 8 C);
Figur 9: vier schematische Ansichten von miteinander verbundenen Platten eines dritten alternativen Knochenersatzmaterials, A) perspektivische Ansicht, B) Seitenansicht, C) Aufsicht auf die Oberseite, D) Querschnittansicht entlang des Schnitts B-B nach Figur 9 C);
Figur 10: drei schematische Ansichten von zwei miteinander verbundenen Platten eines vierten alternativen Knochenersatzmaterials, A) perspektivische Ansicht, B) Aufsicht auf die Oberseite, C) Querschnittansicht entlang des Schnitts A-A nach Figur 10 B);
Figur 11: eine schematische perspektivische Ansicht auf zwei über die Verbindungselemente miteinander verbundene Platten des vierten erfindungsgemäßen Knochenersatzmaterials nach Art der Figur 10;
Figur 12: eine schematische perspektivische Ansicht auf zwei nicht miteinander verbundene Platten des vierten erfindungsgemäßen Knochenersatzmaterials nach Art der Figuren 10 und 11;
Figur 13: vier schematische Ansichten des vierten alternativen Knochenersatzmaterials in einer Ausführung mit Fehlstellen A) perspektivische Ansicht von zwei über die Verbindungselemente miteinander verbundenen Platten, B) Seitenansicht einer Grundplatte, C) Aufsicht auf die Oberseite der Grundplatte nach Figur 13 B), D) Querschnittansicht entlang des Schnitts A-A nach Figur 13 C);
Figur 14: eine schematische perspektivische Ansicht auf zwei über die Verbindungselemente miteinander verbundene Platten eines fünften erfindungsgemäßen Knochenersatzmaterials;
Figur 15: drei schematische Ansichten der zwei miteinander verbundenen Platten des fünften alternativen Knochenersatzmaterials nach Figur 14, A) Aufsicht auf die Oberseite, B) Seitenansicht, C) Querschnittansicht entlang des Schnitts B-B nach Figur 15 A);
Figur 16: eine schematische perspektivische Ansicht auf zwei über die Verbindungselemente miteinander verhakte Platten eines sechsten erfindungsgemäßen Knochenersatzmaterials;
Figur 17: eine schematische perspektivische Ansicht auf zwei nicht miteinander verhakte Platten des sechsten erfindungsgemäßen Knochenersatzmaterials nach Figur 16;
Figur 18: eine schematische Seitenansicht auf miteinander verhakte Platten des sechsten erfindungsgemäßen Knochenersatzmaterials nach Figur 16;
Figur 19: eine schematische perspektivische Detailansicht auf zwei miteinander verhakte Haken (links) zwei nicht miteinander verhakte Haken (rechts) des sechsten erfindungsgemäßen Knochenersatzmaterials nach den Figuren 16 bis 18;
Figur 20: eine schematische perspektivische Ansicht auf zwei nicht miteinander verhakte Platten eines siebten erfindungsgemäßen Knochenersatzmaterials;
Figur 21: drei schematische Ansichten von zwei miteinander verbundenen Platten des siebten alternativen Knochenersatzmaterials A) Aufsicht auf die Oberseite, B) Seitenansicht, C) Querschnittansicht entlang des Schnitts B-B nach Figur 21 A);
Figur 22: eine schematische perspektivische Ansicht auf zwei nicht miteinander verhakte Platten eines achten erfindungsgemäßen Knochenersatzmaterials;
Figur 23: drei schematische Ansichten von zwei miteinander verbundenen Platten des achten alternativen Knochenersatzmaterials A) Aufsicht auf die Oberseite, B) Seitenansicht, C) Querschnittansicht entlang des Schnitts C-C nach Figur 23 A);
Figur 24: eine schematische perspektivische Ansicht auf zwei nicht miteinander verhakte Platten einer Modifikation des achten erfindungsgemäßen Knochenersatzmaterials;
Figur 25: drei schematische Ansichten von zwei miteinander verbundenen Platten der Modifikation des achten alternativen Knochenersatzmaterials A) Aufsicht auf die Oberseite, B) Seitenansicht, C) Querschnittansicht entlang des Schnitts C-C nach Figur 25 A);
Figur 26: eine schematische perspektivische Ansicht dreier und sechs paarweise miteinander verbundener Platten des achten alternativen Knochenersatzmaterials und von drei Partikeln eines Knochenersatzmaterials, wobei die Platten mit den Partikeln verbunden werden können;
Figur 27: eine schematische perspektivische Ansicht auf zwei miteinander gerastete Platten eines neunten erfindungsgemäßen Knochenersatzmaterials;
Figur 28: eine schematische perspektivische Ansicht auf die zwei nicht miteinander gerasteten Platten des neunten erfindungsgemäßen Knochenersatzmaterials nach Figur 27;
Figur 29: zwei schematische Ansichten der zwei miteinander gerasteten Platten des neunten alternativen Knochenersatzmaterials nach Figur 27 in einer Aufsicht auf die Oberseite (Figur 29 unten) und in einer Seitenansicht (Figur 29 oben);
Figur 30: eine schematische Querschnittansicht des Schnitts A-A gemäß Figur 29 unten der mit einander gerasteten Platten nach den Figuren 27 und 29;
Figur 31: eine schematische perspektivische Ansicht auf mehrere Platten eines zehnten und elften erfindungsgemäßen Knochenersatzmaterials, die teilweise über ihre Verbindungselemente miteinander verhakt sind;
Figur 32: drei schematische Ansichten von einer Platte eines zwölften alternativen Knochenersatzmaterials, Unten: Aufsicht auf die Oberseite, Mitte: Seitenansicht, Oben: Querschnittansicht entlang des Schnitts A-A nach Figur 32 Unten;
Figur 33: drei schematische Ansichten von einer Platte des zehnten alternativen Knochenersatzmaterials, Unten: Aufsicht auf die Oberseite, Mitte: Seitenansicht, Oben: Querschnittansicht entlang des Schnitts B-B nach Figur 33 Unten;
Figur 34: drei schematische Ansichten von einer Platte des elften alternativen Knochenersatzmaterials, Unten: Aufsicht auf die Oberseite, Mitte: Seitenansicht, Oben: Querschnittansicht entlang des Schnitts C-C nach Figur 34 Unten;
Figur 35: zwei schematische Ansichten von zwei miteinander verbundenen Platten des zwölften alternativen Knochenersatzmaterials nach Figur 32, Unten: Aufsicht auf die Oberseite, Oben: Querschnittansicht entlang des Schnitts D-D nach Figur 35 Unten;
Figur 36: zwei schematische Ansichten von zwei miteinander verbundenen Platten des zehnten alternativen Knochenersatzmaterials nach Figur 33, Unten: Aufsicht auf die Oberseite, Oben: Querschnittansicht entlang des Schnitts E-E nach Figur 36 Unten; und
Figur 37: zwei schematische Ansichten von zwei miteinander verbundenen Platten des elften alternativen Knochenersatzmaterials nach Figur 34, Unten: Aufsicht auf die Oberseite, Oben: Querschnittansicht entlang des Schnitts F-F nach Figur 37 Unten.

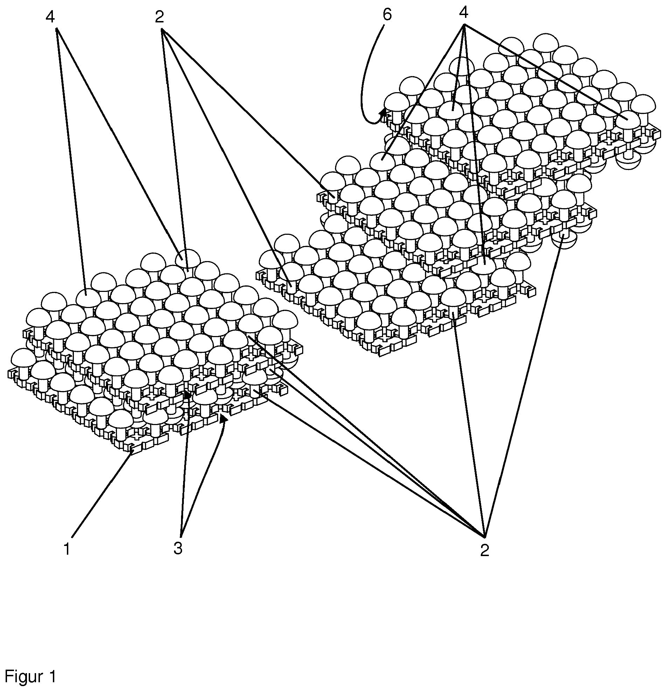

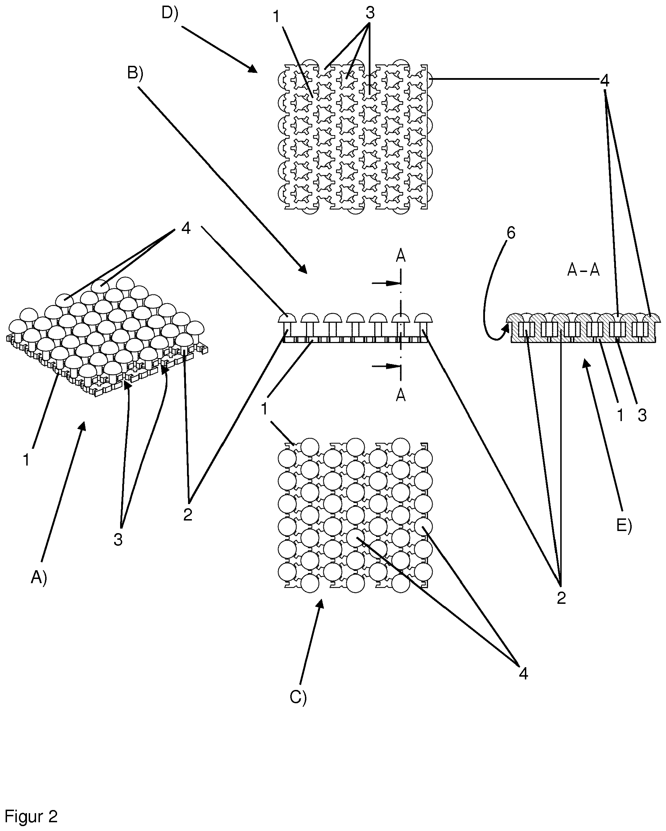

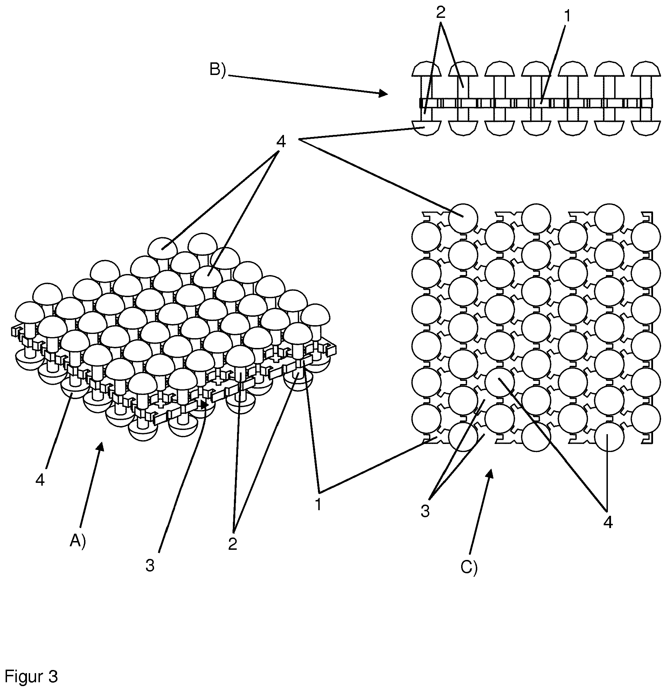

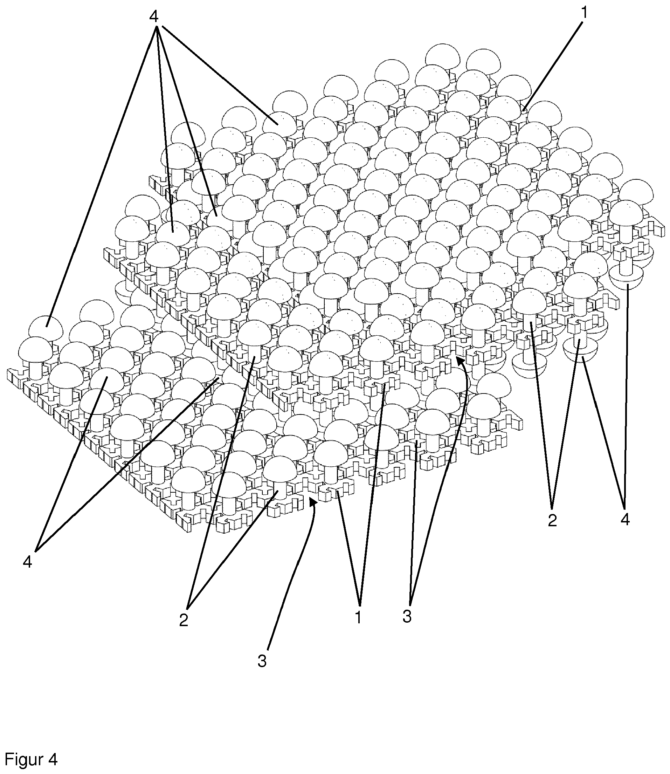

Die Figuren 1 bis 7 zeigen eine erste Ausführungsform der vorliegenden Erfindung, dabei zeigt Figur 1 eine schematische perspektivische Ansicht auf fünf teilweise unter einander zu einem offenporigen Körper beziehungsweise Volumenkörper verbundene Platten eines erfindungsgemäßen Knochenersatzmaterials und Figur 2 zeigt fünf schematische Ansichten der beiden unteren Platten (Grundplatten) nach Figur 1, A) als perspektivische Ansicht, B) als Seitenansicht, C) als Aufsicht auf die Oberseite, D) als Sicht auf Unterseite und E) als Querschnittansicht entlang des Schnitts A-A nach Figur 2 B). Zudem zeigt Figur 3 drei schematische Ansichten der drei oberen Platten (Aufbauplatten) nach Figur 1, A) als perspektivische Ansicht, B) als Seitenansicht und C) als Aufsicht auf Oberseite. Figur 4 zeigt eine schematische perspektivische Ansicht auf drei unterschiedlich tief ineinander verhakte Platten des erfindungsgemäßen Knochenersatzmaterials zur Bildung eines offenporigen Volumenkörpers nach den Figuren 1 bis 3.

Die Platten bestehen aus einem elastischen biokompatiblen Kunststoff oder aus Edelstahl, Titan, einer Titanlegierung, Tantal, einer Tantallegierung können aber auch aus Kompositen dieser Materialien gefertigt sein. Die Platten werden mit einem CAM-Verfahren (CAM - Computer-Aided Manufacturing, Deutsch: rechner-unterstützte Fertigung) beziehungsweise einem 3D-Druckverfahren hergestellt, beispielsweise mit selektivem Laserschmelzen SLM (Selective Laser Melting). Andere Rapid-Prototyping-Verfahren beziehungsweise computergestützte generative Herstellungsverfahren können auch zur Herstellung der Platten verwendet werden, wie beispielsweise Fused Layer Modeling/Manufacturing (FLM), Fused Deposition Modeling (FDM), Laminated Object Modelling (LOM) von Kunststofffolien, Layer Laminated Manufacturing (LLM) von Kunststofffolien, Elektronenstrahlschmelzen (EBM) von Kunststoffen oder Metallen, Multi Jet Modeling (MJM) von Kunststoffen, Selektives Lasersintern (SLS) von Kunststoffen oder Metallen, Stereolithografie (STL oder SLA) von Kunststoffen, Schleif- oder Mehr-Achs-Fräsverfahren oder Digital Light Processing (DLP) von photopolymerisierbaren flüssigen Kunststoffen.

Die Platten weisen jeweils ein plattenförmiges Flächengebilde 1 auf, das die gesamten Platten trägt und in sich verbindet. Das Flächengebilde 1 ist flexibel und elastisch verformbar, so dass auch andere Flächen als Ebenen mit dem Flächengebilde 1 geformt werden können. Von dem Flächengebilde 1 erstrecken sich bei jeder Platte eine Vielzahl von Stiften 2 weg, die senkrecht von der Ebene der Flächengebilde 1 abstehen. In dem Flächengebilde 1 sind zwischen den Stiften 2 eine Vielzahl von durchgehenden Ausnehmungen 3 angeordnet, die, wenn die Platten miteinander zu einem Volumenkörper verbunden sind, eine offene Porosität des Volumenkörpers in einer Richtung senkrecht zu den Flächengebilden 1 bewirken.

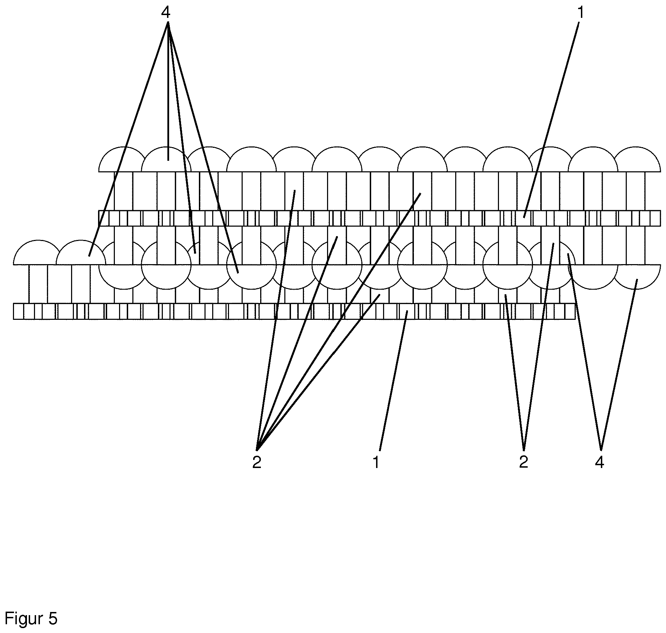

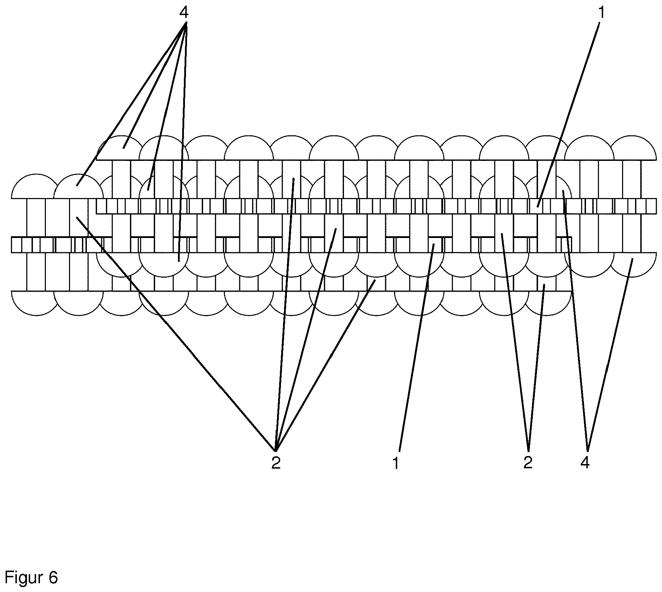

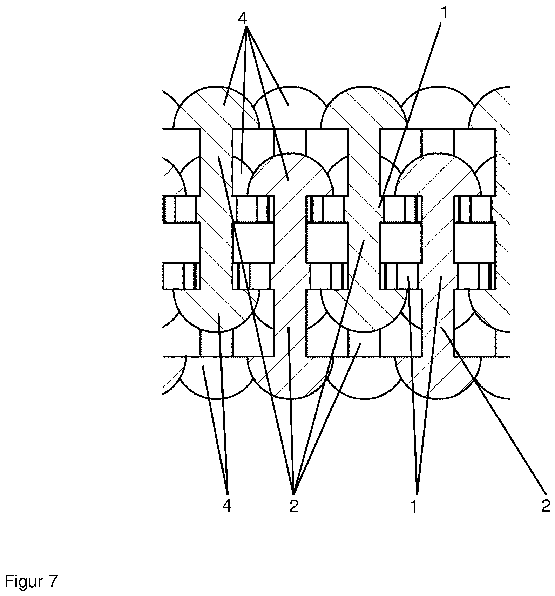

In den Figuren 1 bis 7 sind zwei verschiedene Typen von Platten dargestellt nämlich erstens Grundplatten, die eine flache Unterseite aufweisen und bei denen sich die Stifte 2 nur auf einer Seite der Flächengebilde 1 von dem Flächengebilde 1 aus erstrecken, und zweitens Aufbauplatten, bei denen sich die Stifte 2 von beiden Seiten des Flächengebildes 1 aus erstrecken. Die Grundplatten sind in Figur 1 unten, in Figur 2, in Figur 4 unten und in Figur 5 unten gezeigt. Die Aufbauplatten sind in Figur 1 oben auf, in Figur 3, in Figur 4 oben auf, in Figur 5 oben auf und den Figuren 6 und 7 gezeigt. Die Grundplatten können großflächig anliegend auf dem zu behandelnden Knochen befestigt werden. Die Aufbauplatten können allerdings ebenfalls auf dem zu behandelnden Knochen befestigt werden, wenn auch mit einer geringeren Auflagefläche, so dass theoretisch auf die Grundplatten verzichtet werden kann.

An den ansonsten zylindrischen Stiften 2 sind an dem den Flächengebilden 1 gegenüberliegenden Enden der Stifte 2 Pilze 4 als Verbindungselemente 4 vorgesehen. Die Pilze 4 sind nach außen (von dem Flächengebilde 1 weg) abgerundet und bilden Kugelabschnitte. Es sind aber auch andere Abrundungen, wie beispielsweise Ellipsenabschnitte möglich. An der zum Flächengebilde 1 hin ausgerichteten Seite bilden die Pilze 4 eine ebene Greiffläche 6, die zum Verhaken mit anderen Pilzen 4 eingreifender Platten oder mit den Ausnehmungen 3 eingreifender Platten geeignet sind.

Figur 5 zeigt hierzu eine schematische Seitenansicht auf zwei nur über die Pilze 4 miteinander verhakte Platten des erfindungsgemäßen Knochenersatzmaterials, Figur 6 eine schematische Seitenansicht auf zwei über die Pilze 4 und über die Ausnehmungen 3 des Flächengebildes 1 miteinander gerastete Platten des erfindungsgemäßen Knochenersatzmaterials und Figur 7 eine schematische Querschnittansicht der zwei über die Pilze 4 und über die Ausnehmungen 3 des Flächengebildes 1 miteinander gerastete Platten, wie in Figur 6 gezeigt.

Um ein erfindungsgemäßes Knochenersatzmaterial zu bilden, liegen die Platten bevorzugt aneinander anliegend aber nicht miteinander verhakt oder gerastet vor, so dass also die Pilze 4 der Stifte 2 benachbarter Platten noch nicht ineinander greifen. Zudem können die Platten mit einer Flüssigkeit benetzt vorliegen. In der Flüssigkeit ist bevorzugt zumindest eine pharmazeutisch wirksame Substanz enthalten, die zur Bekämpfung der Infektion oder zum Anregen des Knochenwachstums geeignet ist. Alternativ oder zusätzlich können die Platten mit einer solchen pharmazeutisch wirksamen Substanz beschichtet sein.

Das Knochenersatzmaterial kann geformt werden, indem die Platten über ihre Flächen ineinander gedrückt werden. Dadurch verhaken oder rasten die Platten miteinander und das Knochenersatzmaterial wird in der gewünschten Form verfestigt. Zuvor können die Platten auch durch elastische Verformung der Flächengebilde 1 verformt und an die Behandlungssituation angepasst werden. Nach dem Verhaken oder Rasten mit zumindest einer weiteren (meist dann ebenfalls verformten) Platte stabilisieren sich die beiden so miteinander verbundenen Platten gegenseitig, so dass die gewählte Form verfestigt ist.

Die Platten verbinden sich dabei untereinander in der Art, dass freie Zwischenräume zwischen den miteinander verbundenen Platten im Bereich der Stifte 2 und der Pilze 4 verbleiben, so dass der aus den Platten geformte Volumenköper auch in den Richtungen parallel zur Ebene der Platten offenporig ist. Die Platten haben einen Querschnitt beziehungsweise eine Dicke von etwa 5 mm, so dass die verbleibenden Poren einen freien Querschnitt im Bereich von etwa 0,5 mm aufweisen. Dieser Querschnitt reicht aus, damit sich in den Poren Knochenmaterial ausbilden beziehungsweise einwachsen kann. Der Volumenkörper mit seinen offenen Poren kann also als osteokonduktiv bezeichnet werden. Der aus den Platten geformte Volumenkörper ist daher als Knochenersatzmaterial gut geeignet.

Die Platten sollten fest ineinander gedrückt werden, so dass der Volumenkörper formstabil ist. Die Platten können dabei in einer ersten Stufe mit einander verhaken, indem die Pilze 4 die Stifte 2 verbundener Platten elastisch verformen und durch die elastische Rückstellkraft der Stifte 2 die Pilze 4 die Bewegung benachbarter Platten von dem Flächengebilde 1 weg begrenzen (siehe Figur 5). Die Platten können durch weiteres ineinander drücken der Platten in einer zweiten Stufe mit einander rasten, indem die Pilze 4 durch die Ausnehmungen 3 der Flächengebilde 1 hindurchgedrückt werden. Dabei kann es zu einem Verklemmen der Pilze 4 mit den Ausnehmungen 3 kommen, die eine Bewegung der Pilze 4 gegen die benachbarte Platte verhindert und damit die beiden Platten miteinander rastet. Es ist auch möglich, dass erstens die Kanten der Pilze 4 die Stifte 2 oder die Ausnehmungen 3 oder die Pilze 4 oder zweitens die Kanten der Ausnehmungen 3 die Pilze 4 benachbarter Platten in geringem Umfang plastisch verformen und dadurch eine Rastung der Platten miteinander erfolgt. Zwei Platten können ferner miteinander verbunden werden, indem die Platten an manchen Bereichen über die Pilze 4 miteinander verhakt werden und in anderen Bereichen über die Pilze 4 und Ausnehmungen 3 miteinander gerastet werden. Bevorzugt werden die Abmessungen der Pilze 4, die Tiefe der Ausnehmungen 3 (beziehungsweise die Dicke des Flächengebildes 1) und die Länge der Stifte 2 zwischen dem Flächengebilde 1 und den Pilzen 4 so aufeinander abgestimmt, dass bei einem Verhaken der Platten die von dem Flächengebilde 1 abgewandten Oberflächen der Pilze 4 an der Oberfläche der Flächengebilde 1 benachbarter Platten anliegen und bei einem Rasten der Platten die von dem Flächengebilde 1 abgewandten Oberflächen der Pilze 4 an der Greiffläche 6 der Pilze 4 der benachbarten Platte anliegen. Hierdurch wird erreicht, dass die Platten weder beim Verhaken noch beim Rasten ohne Verformung gegeneinander beweglich sind.

Damit die Greifflächen 6 oder die gegenüberliegenden Kappenoberseiten der Pilze 4 die Ausnehmungen 3 nicht komplett verschießen und damit die Ausnehmungen 3 von den Pilzen 4 leichter verformbar sind, weisen die Ausnehmungen 3 jeweils sechs Schlitze auf, die über den Umfang der Ausnehmungen 3 verteilte sind. Die Breite der Schlitze sollte dazu ausreichen, dass sie osteokonduktiv wirken können.

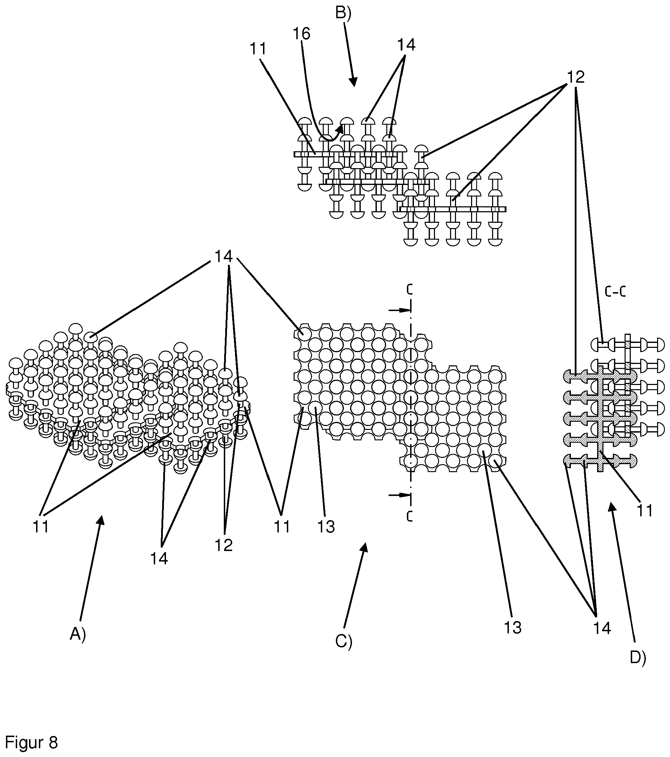

Figur 8 zeigt vier schematische Ansichten von miteinander verbundenen Platten eines zweiten alternativen Knochenersatzmaterials in A) einer perspektivischen Ansicht, B) einer Seitenansicht, C) einer Aufsicht auf die Oberseite und D) einer Querschnittansicht entlang des Schnitts C-C nach Figur 8 C).

Die Platten bestehen aus Edelstahl, Titan, einer Titanlegierung, Tantal, einer Tantallegierung können aber auch aus einem elastischen biokompatiblen Kunststoff oder aus einem Komposit der metallischen Materialien gefertigt sein. Die Platten werden mit einem CAM-Verfahren beziehungsweise einem 3D-Druckverfahren hergestellt, beispielsweise mit selektivem Elektronenstrahlschmelzen (EBM). Andere Rapid-Prototyping-Verfahren beziehungsweise computergestützte generative Herstellungsverfahren können auch zur Herstellung der Platten verwendet werden.

Die Platten weisen jeweils ein plattenförmiges Flächengebilde 11 auf, das die gesamten Platten trägt und in sich verbindet. Das Flächengebilde 11 ist flexibel und elastisch verformbar, so dass auch andere Flächen als Ebenen mit dem Flächengebilde 11 geformt werden können. Von dem Flächengebilde 11 erstrecken sich bei jeder Platte eine Vielzahl von Stiften 12 weg, die senkrecht von der Ebene der Flächengebilde 11 abstehen. In dem Flächengebilde 11 sind zwischen den Stiften 12 eine Vielzahl von durchgehenden Ausnehmungen 13 angeordnet, die, wenn die Platten miteinander zu einem Volumenkörper verbunden sind, eine offene Porosität des Volumenkörpers in einer Richtung senkrecht zu den Flächengebilden 11 bewirken können, wenn nicht die benachbarten Platten anliegen und dadurch die Ausnehmungen 13 abdecken.

In Figur 8 ist nur einer der beiden Typen von Platten dargestellt, nämlich Aufbauplatten, bei denen sich die Stifte 12 von beiden Seiten des Flächengebildes 11 aus erstrecken. Es können auch Grundplatten vorgesehen sein, bei denen eine flache Unterseite vorgesehen ist und bei denen sich die Stifte 12 nur auf einer Seite der Flächengebilde 11 von dem Flächengebilde 11 aus erstrecken. Solche Grundplatten können großflächig anliegend auf dem zu behandelnden Knochen befestigt werden. Die gezeigten Aufbauplatten können allerdings ebenfalls auf dem zu behandelnden Knochen befestigt werden, wenn auch mit einer geringeren Auflagefläche, so dass auf die Grundplatten verzichtet werden kann.

An den ansonsten zylindrischen Stiften 12 sind an dem den Flächengebilden 11 gegenüberliegenden Enden der Stifte 12 und in der Mitte zwischen den Enden der Stifte 12 und den Flächengebilden 11 Pilze 14 als Verbindungselemente 14 vorgesehen. Die Pilze 14 sind nach außen (von dem Flächengebilde 11 weg) abgerundet und bilden Kugelabschnitte. Es sind aber auch andere Abrundungen, wie beispielsweise Ellipsenabschnitte möglich. An der zum Flächengebilde 11 hin ausgerichteten Seite bilden die Pilze 14 eine ebene Greiffläche 16, die zum Verhaken mit anderen Pilzen 14 eingreifender Platten oder mit den Ausnehmungen 13 eingreifender Platten geeignet sind.

Um ein erfindungsgemäßes Knochenersatzmaterial zu bilden, liegen die Platten aneinander anliegend aber nicht miteinander verhakt oder gerastet vor (also nicht so wie in Figur 8 gezeigt), so dass also die Pilze 14 der Stifte 12 benachbarter Platten noch nicht ineinander greifen. Zudem können die Platten mit einer Flüssigkeit benetzt vorliegen. In der Flüssigkeit ist bevorzugt zumindest eine pharmazeutisch wirksame Substanz enthalten, die zur Bekämpfung der Infektion oder zum Anregen des Knochenwachstums geeignet ist. Alternativ oder zusätzlich können die Platten mit einer solchen pharmazeutisch wirksamen Substanz beschichtet sein.

Das Knochenersatzmaterial kann geformt werden, indem die Platten über ihre Flächen ineinander gedrückt werden. Dadurch verhaken oder rasten die Platten miteinander und das Knochenersatzmaterial wird in der gewünschten Form verfestigt. Zuvor können die Platten auch durch elastische Verformung der Flächengebilde 11 verformt und an die Behandlungssituation angepasst werden. Nach dem Verhaken oder Rasten mit zumindest einer weiteren (meist dann ebenfalls verformten) Platte stabilisieren sich die beiden so miteinander verbundenen Platten gegenseitig, so dass die gewählte Form verfestigt ist.

Die Platten verbinden sich dabei untereinander in der Art, dass freie Zwischenräume zwischen den miteinander verbundenen Platten im Bereich der Stifte 12 und der Pilze 14 verbleiben, so dass der aus den Platten geformte Volumenköper in den Richtungen parallel zur Ebene der Platten offenporig ist. Die Platten haben einen Querschnitt beziehungsweise eine Dicke von etwa 9 mm, so dass die verbleibenden Poren einen freien Querschnitt im Bereich von etwa 0,5 mm aufweisen. Dieser Querschnitt reicht aus, damit sich in den Poren Knochenmaterial ausbilden beziehungsweise einwachsen kann. Der Volumenkörper mit seinen offenen Poren kann also als osteokonduktiv bezeichnet werden. Der aus den Platten geformte Volumenkörper ist daher als Knochenersatzmaterial gut geeignet.

Die Platten sollten fest ineinander gedrückt werden, so dass der Volumenkörper formstabil ist. Die Platten können dabei in einer ersten Stufe mit einander verhaken, indem die Pilze 14 die Stifte 12 verbundener Platten elastisch verformen und durch die elastische Rückstellkraft der Stifte 12 die Pilze 14 die Bewegung benachbarter Platten von dem Flächengebilde 11 weg begrenzen. Die Platten können durch weiteres ineinander drücken der Platten in einer zweiten Stufe mit einander rasten, indem die äußeren Pilze 14 durch die Ausnehmungen 13 der Flächengebilde 11 hindurchgedrückt werden, wie in Figur 8 gezeigt. Dabei kann es zu einem Verklemmen der äußeren Pilze 14 mit den Ausnehmungen 13 kommen, die eine Bewegung der Pilze 14 gegen die benachbarte Platte verhindert und damit die beiden Platten miteinander rastet. Es ist auch möglich, dass erstens die Kanten der Pilze 14 die Stifte 12 oder die Ausnehmungen 13 oder die Pilze 14 oder zweitens die Kanten der Ausnehmungen 13 die Pilze 14 benachbarter Platten in geringem Umfang plastisch verformen und dadurch eine Rastung der Platten miteinander erfolgt. Eine erste Verhakung findet auch bereits dann statt, wenn die äußeren Pilze 14 benachbarter Platten ineinander greifen. Zwei Platten können ferner miteinander verbunden werden, indem die Platten an manchen Bereichen über die Pilze 14 miteinander verhakt werden und in anderen Bereichen über die Pilze 14 und Ausnehmungen 13 miteinander gerastet werden. Bevorzugt werden die Abmessungen der Pilze 14, die Tiefe der Ausnehmungen 13 (beziehungsweise die Dicke des Flächengebildes 11) und die Länge der Stifte 12 zwischen dem Flächengebilde 11 und den Pilzen 14 und zwischen den inneren und äußeren Pilzen 14 so aufeinander abgestimmt, dass bei einem Verbinden der Platten die von dem Flächengebilde 11 abgewandten Oberflächen der Pilze 14 an der Oberfläche der Flächengebilde 11 benachbarter Platten anliegen und/oder bei einem Verbinden der Platten die von dem Flächengebilde 11 abgewandten Oberflächen der Pilze 14 an der Greiffläche 16 der Pilze 14 der benachbarten Platte anliegen. Hierdurch wird erreicht, dass die verbundenen Platten nicht ohne Verformung gegeneinander beweglich sind.

Damit die Greifflächen 16 oder die gegenüberliegende Kappenoberseiten der Pilze 14 die Ausnehmungen 13 nicht komplett verschießen und damit die Ausnehmungen 13 von den Pilzen 14 leichter verformbar sind, können die Ausnehmungen 13 mehrere Schlitze aufweisen (nicht gezeigt), die über den Umfang der Ausnehmungen 13 verteilte sind. Die Breite der Schlitze sollte dazu ausreichen, dass sie osteokonduktiv wirken können.

Die Ausführungsform nach Figur 8 unterscheidet sich also von der nach den Figuren 1 bis 7 vor allem dadurch, dass die Stifte 12 zwei Pilze 14 aufweisen die beabstandet zueinander angeordnet sind. Zudem weisen die Ausnehmungen 13 keine zusätzlichen Schlitze auf.

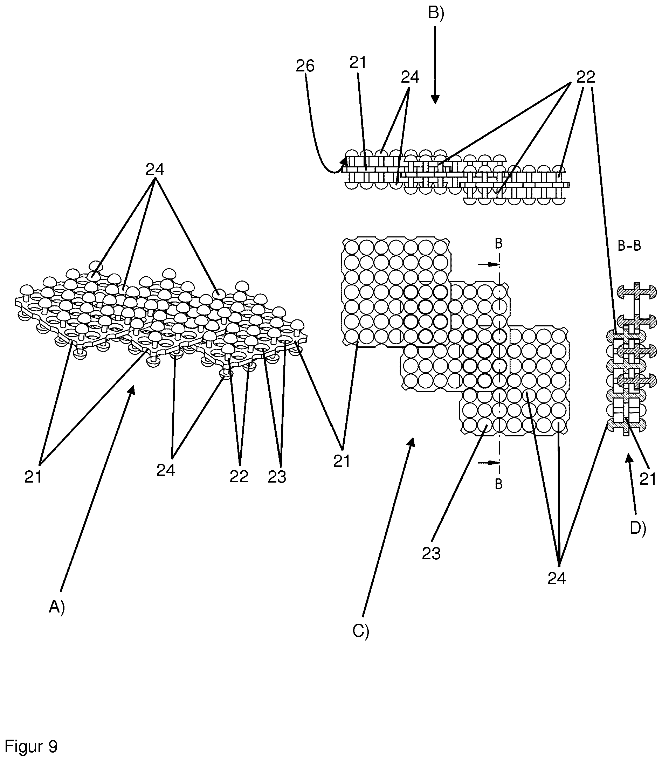

Figur 9 zeigt vier schematische Ansichten von miteinander verbundenen Platten eines dritten alternativen Knochenersatzmaterials. Dabei zeigt Figur 9 A) eine perspektivische Ansicht, Figur 9 B) eine Seitenansicht, Figur 9 C) eine Aufsicht auf die Oberseite und Figur 9 D) eine Querschnittansicht entlang des Schnitts B-B nach Figur 9 C).

Die Platten bestehen aus Titan oder einer Titanlegierung, Tantal oder einer Tantallegierung können aber auch aus Edelstahl, einem elastischen biokompatiblen Kunststoff oder aus einem Komposit solcher Materialien gefertigt sein. Die Platten werden mit einem CAM-Verfahren beziehungsweise einem 3D-Druckverfahren hergestellt, beispielsweise mit selektivem Elektronenstrahlschmelzen (EBM). Andere Rapid-Prototyping-Verfahren beziehungsweise computergestützte generative Herstellungsverfahren können auch zur Herstellung der Platten verwendet werden.

Die Platten weisen jeweils ein plattenförmiges Flächengebilde 21 auf, das die gesamten Platten trägt und in sich verbindet. Das Flächengebilde 21 ist flexibel und elastisch verformbar, so dass auch andere Flächen als Ebenen mit dem Flächengebilde 21 geformt werden können. Von dem Flächengebilde 21 erstrecken sich bei jeder Platte eine Vielzahl von Stiften 22 weg, die senkrecht von der Ebene der Flächengebilde 21 abstehen. In dem Flächengebilde 21 sind zwischen den Stiften 22 eine Vielzahl von durchgehenden Ausnehmungen 23 angeordnet, die, wenn die Platten miteinander zu einem Volumenkörper verbunden sind, eine offene Porosität des Volumenkörpers in einer Richtung senkrecht zu den Flächengebilden 21 bewirken können, wenn nicht die benachbarten Platten anliegen und dadurch die Ausnehmungen 23 abdecken.

In Figur 9 ist nur einer der beiden Typen von Platten dargestellt, nämlich Aufbauplatten, bei denen sich die Stifte 22 von beiden Seiten des Flächengebildes 21 aus erstrecken. Es können auch Grundplatten vorgesehen sein, bei denen eine flache Unterseite vorgesehen ist und bei denen sich die Stifte 22 nur auf einer Seite der Flächengebilde 21 von dem Flächengebilde 21 aus erstrecken. Solche Grundplatten können großflächig anliegend auf dem zu behandelnden Knochen befestigt werden. Die gezeigten Aufbauplatten können allerdings ebenfalls auf dem zu behandelnden Knochen befestigt werden, wenn auch mit einer geringeren Auflagefläche, so dass auf die Grundplatten verzichtet werden kann.

An den ansonsten zylindrischen Stiften 22 sind an dem den Flächengebilden 21 gegenüberliegenden Enden der Stifte 22 Pilze 24 als Verbindungselemente 24 vorgesehen. Die Pilze 24 sind nach außen (von dem Flächengebilde 21 weg) abgerundet und bilden Kugelabschnitte. Es sind aber auch andere Abrundungen, wie beispielsweise Ellipsenabschnitte möglich. An der zum Flächengebilde 21 hin ausgerichteten Seite bilden die Pilze 24 eine ebene Greiffläche 26, die zum Verhaken oder Rasten mit anderen Pilzen 24 eingreifender Platten oder mit den Ausnehmungen 23 eingreifender Platten geeignet sind.

Um ein erfindungsgemäßes Knochenersatzmaterial zu bilden, liegen die Platten aneinander anliegend aber nicht miteinander verhakt oder gerastet vor (also nicht so wie in Figur 9 gezeigt), so dass also die Pilze 24 der Stifte 22 benachbarter Platten noch nicht ineinander greifen. Zudem können die Platten mit einer Flüssigkeit benetzt vorliegen. In der Flüssigkeit ist bevorzugt zumindest eine pharmazeutisch wirksame Substanz enthalten, die zur Bekämpfung der Infektion oder zum Anregen des Knochenwachstums geeignet ist. Alternativ oder zusätzlich können die Platten mit einer solchen pharmazeutisch wirksamen Substanz beschichtet sein.

Das Knochenersatzmaterial kann geformt werden, indem die Platten über ihre Flächen ineinander gedrückt werden. Dadurch verhaken oder rasten die Platten miteinander und das Knochenersatzmaterial wird in der gewünschten Form verfestigt. Zuvor können die Platten auch durch elastische Verformung der Flächengebilde 21 verformt und an die Behandlungssituation angepasst werden. Nach dem Verhaken oder Rasten mit zumindest einer weiteren (meist dann ebenfalls verformten) Platte stabilisieren sich die beiden so miteinander verbundenen Platten gegenseitig, so dass die gewählte Form verfestigt ist.

Die Platten verbinden sich dabei untereinander in der Art, dass freie Zwischenräume zwischen den miteinander verbundenen Platten im Bereich der Stifte 22 und der Pilze 24 verbleiben, so dass der aus den Platten geformte Volumenköper in den Richtungen parallel zur Ebene der Platten offenporig ist. Die Platten haben einen Querschnitt beziehungsweise eine Dicke von etwa 3 mm, so dass die verbleibenden Poren einen freien Querschnitt im Bereich von etwa 0,3 mm aufweisen. Dieser Querschnitt reicht aus, damit sich in den Poren Knochenmaterial ausbilden beziehungsweise einwachsen kann. Der Volumenkörper mit seinen offenen Poren kann also als osteokonduktiv bezeichnet werden. Der aus den Platten geformte Volumenkörper ist daher als Knochenersatzmaterial gut geeignet.

Die Platten sollten fest ineinander gedrückt werden, so dass der Volumenkörper formstabil ist. Die Platten können dabei in einer ersten Stufe mit einander verhaken, indem die Pilze 24 die Stifte 22 verbundener Platten elastisch verformen und durch die elastische Rückstellkraft der Stifte 22 die Pilze 24 die Bewegung benachbarter Platten von dem Flächengebilde 21 weg begrenzen. Die Platten können durch weiteres ineinander drücken der Platten in einer zweiten Stufe mit einander verhaken oder rasten, indem die Pilze 24 durch die Ausnehmungen 23 der Flächengebilde 21 hindurchgedrückt werden, wie in Figur 9 gezeigt. Dabei kann es zu einem Verklemmen der Pilze 24 mit den Ausnehmungen 23 kommen, die eine Bewegung der Pilze 24 gegen die benachbarte Platte verhindert und damit die beiden Platten miteinander rastet. Es ist auch möglich, dass erstens die Kanten der Pilze 24 die Stifte 22 oder die Ausnehmungen 23 oder die Pilze 24 oder zweitens die Kanten der Ausnehmungen 23 die Pilze 24 benachbarter Platten in geringem Umfang plastisch verformen und dadurch eine Rastung der Platten miteinander erfolgt.

Zwei Platten können ferner miteinander verbunden werden, indem die Platten an manchen Bereichen über die Pilze 24 miteinander verhakt werden und in anderen Bereichen über die Pilze 24 und Ausnehmungen 23 miteinander gerastet werden.

Bevorzugt werden die Abmessungen der Pilze 24, die Tiefe der Ausnehmungen 23 (beziehungsweise die Dicke des Flächengebildes 21) und die Länge der Stifte 22 zwischen dem Flächengebilde 21 und den Pilzen 24 so aufeinander abgestimmt, dass bei einem Verbinden der Platten die von dem Flächengebilde 21 abgewandten Oberflächen der Pilze 24 an der Oberfläche der Flächengebilde 21 benachbarter Platten anliegen und/oder bei einem Verbinden der Platten die von dem Flächengebilde 21 abgewandten Oberflächen der Pilze 24 an der Greiffläche 26 der Pilze 24 der benachbarten Platte anliegen. Hierdurch wird erreicht, dass die verbundenen Platten nicht ohne Verformung gegeneinander beweglich sind.

Damit die Greifflächen 26 oder die gegenüberliegende Kappenoberseiten der Pilze 24 die Ausnehmungen 23 nicht komplett verschießen und damit die Ausnehmungen 23 von den Pilzen 24 leichter verformbar sind, können die Ausnehmungen 23 mehrere Schlitze aufweisen (nicht gezeigt), die über den Umfang der Ausnehmungen 23 verteilte sind. Die Breite der Schlitze sollte dazu ausreichen, dass sie osteokonduktiv wirken können.

Die Ausführungsform nach Figur 9 unterscheidet sich also von der nach den Figuren 1 bis 7 vor allem dadurch, dass die Stifte 22 und die Ausnehmungen 23 einen etwas größeren Abstand zueinander aufweisen. Zudem weisen die Ausnehmungen 23 keine zusätzlichen Schlitze auf.

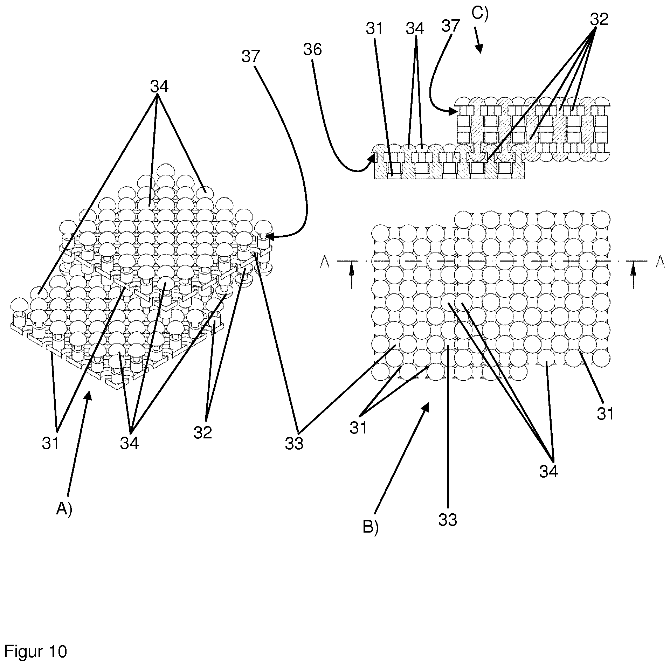

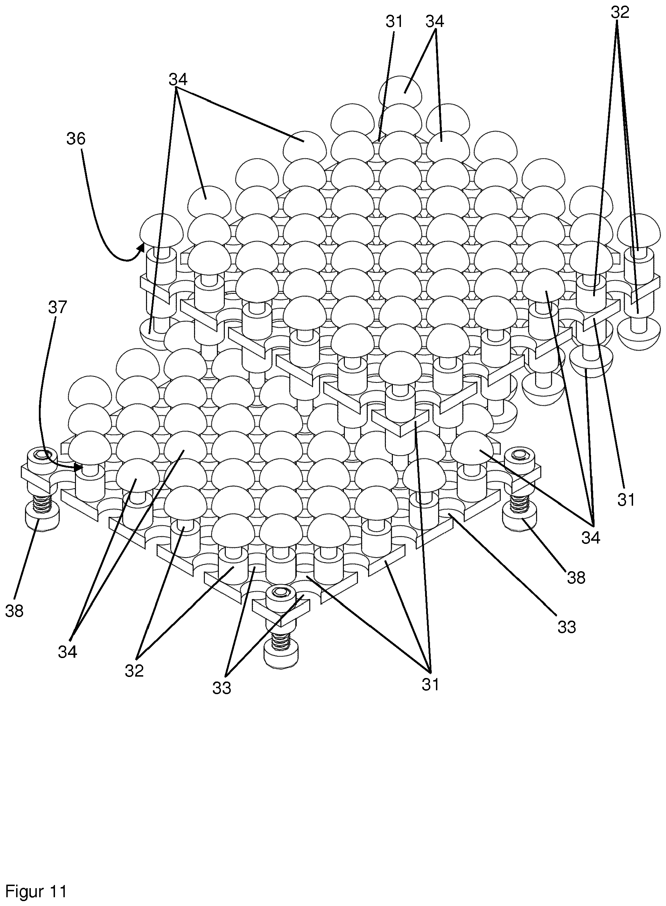

Figur 10 zeigt drei schematische Ansichten von zwei miteinander verbundenen Platten eines vierten alternativen Knochenersatzmaterials, A) perspektivische Ansicht, B) Aufsicht auf Oberseite, C) Querschnittansicht entlang des Schnitts A-A nach Figur 10 B), und die Figuren 11 bis 13 weitere Varianten des vierten alternativen Knochenersatzmaterials.

Die Platten bestehen aus einem biokompatiblen Metall, wie Edelstahl, Titan oder einer Titanlegierung, Tantal oder einer Tantallegierung können aber auch aus elastischem biokompatiblem Kunststoff oder aus einem Komposit solcher Materialien gefertigt sein. Die Platten werden mit einem CAM-Verfahren beziehungsweise einem 3D-Druckverfahren hergestellt, beispielsweise mit selektivem Elektronenstrahlschmelzen (EBM). Andere Rapid-Prototyping-Verfahren beziehungsweise computergestützte generative Herstellungsverfahren können auch zur Herstellung der Platten verwendet werden.

Die Platten weisen jeweils ein plattenförmiges Flächengebilde 31 auf, das die gesamten Platten trägt und in sich verbindet. Das Flächengebilde 31 ist flexibel und elastisch verformbar, so dass auch andere Flächen als Ebenen mit dem Flächengebilde 31 geformt werden können. Von dem Flächengebilde 31 erstrecken sich bei jeder Platte eine Vielzahl von Stiften 32 weg, die senkrecht von der Ebene der Flächengebilde 31 abstehen. In dem Flächengebilde 31 sind zwischen den Stiften 32 eine Vielzahl von durchgehenden Ausnehmungen 33 angeordnet, die, wenn die Platten miteinander zu einem Volumenkörper verbunden sind, eine offene Porosität des Volumenkörpers in einer Richtung senkrecht zu den Flächengebilden 31 bewirken können, wenn nicht die benachbarten Platten anliegen und dadurch die Ausnehmungen 33 abdecken.

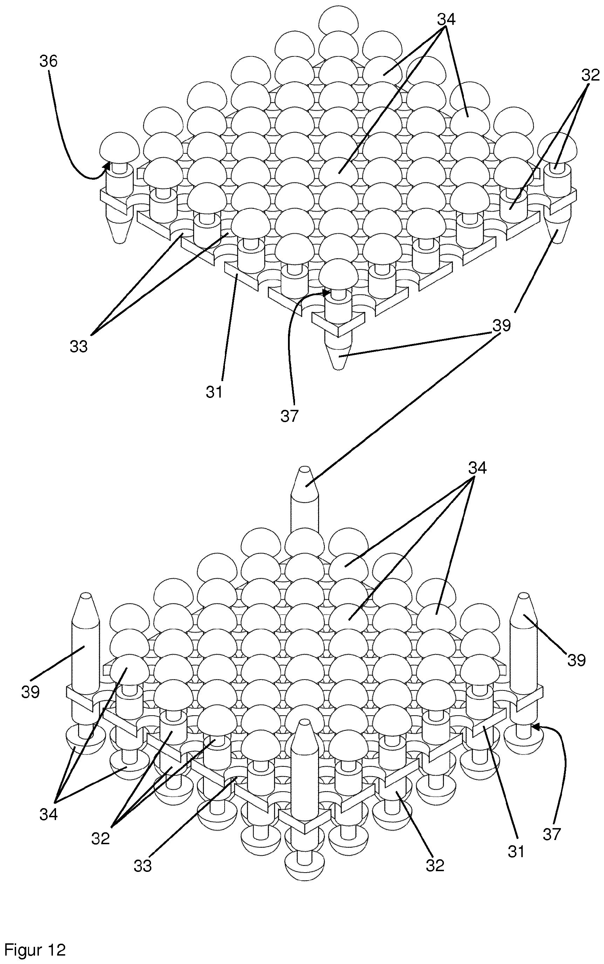

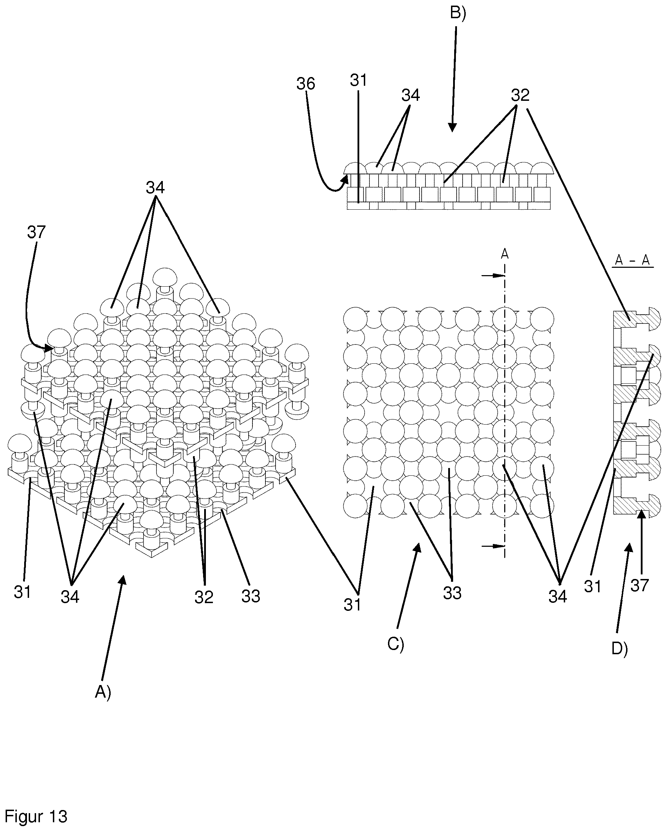

In den Figuren 10 bis 13 sind zwei verschiedene Typen von Platten dargestellt nämlich erstens Grundplatten, die eine flache Unterseite aufweisen und bei denen sich die Stifte 32 nur auf einer Seite der Flächengebilde 31 von dem Flächengebilde 31 aus erstrecken, und zweitens Aufbauplatten, bei denen sich die Stifte 32 von beiden Seiten des Flächengebildes 31 aus erstrecken. Die Grundplatten sind in Figur 10 unten beziehungsweise darunter, in Figur 11 unten, in Figur 12 oben und in Figur 13 A) unten und in Figur 13 B) bis D) gezeigt. Die Aufbauplatten sind in Figur 10 oben auf, in Figur 11 oben auf, in Figur 12 unten und in Figur 13 A) oben auf gezeigt. Die Grundplatten können großflächig anliegend auf dem zu behandelnden Knochen befestigt werden. Die Aufbauplatten können allerdings ebenfalls auf dem zu behandelnden Knochen befestigt werden, wenn auch mit einer geringeren Auflagefläche, so dass auf die Grundplatten verzichtet werden kann.

An den ansonsten zylindrischen Stiften 32 sind an dem den Flächengebilden 31 gegenüberliegenden Enden der Stifte 32 Pilze 34 als Verbindungselemente 34 vorgesehen. Die Pilze 34 sind nach außen (von dem Flächengebilde 31 weg) abgerundet und bilden Kugelabschnitte. Es sind aber auch andere Abrundungen, wie beispielsweise Ellipsenabschnitte möglich. An der zum Flächengebilde 31 hin ausgerichteten Seite bilden die Pilze 34 eine ebene Greiffläche 36, die zum Verhaken oder Rasten mit anderen Pilzen 34 eingreifender Platten oder mit den Ausnehmungen 33 eingreifender Platten geeignet sind.

In den Stiften 32 sind zudem angrenzend zu den Greifflächen 36 Nuten 37 als Verbindungselemente 37 vorgesehen, in die die Pilze 34 benachbarter Platten eingreifen beziehungsweise einrasten können. Hierzu können die Nuten 37 im Gegensatz zu den gezeigten Nuten 37, aber erfindungsgemäß bevorzugt, als Negativ der Form der Wölbung der Pilze 34 geformt sein, damit die Pilze 34 gut in die Nuten 37 passen.

Figur 11 zeigt eine schematische perspektivische Ansicht auf zwei über die Verbindungselemente 34 miteinander verbundene Platten des vierten erfindungsgemäßen Knochenersatzmaterials nach Art der Figur 10, Figur 12 eine schematische perspektivische Ansicht auf zwei nicht miteinander verbundene Platten des vierten erfindungsgemäßen Knochenersatzmaterials nach Art der Figuren 10 und 11 und Figur 13 vier schematische Ansichten von des vierten alternativen Knochenersatzmaterials in einer Ausführung mit Fehlstellen bei den Stiften 32 A) eine perspektivische Ansicht von zwei über die Verbindungselemente miteinander verbundenen Platten, B) eine Seitenansicht einer Grundplatte, C) eine Aufsicht auf die Oberseite der Grundplatte nach Figur 13 B) und D) eine Querschnittansicht entlang des Schnitts A-A nach Figur 13 C).

Um ein erfindungsgemäßes Knochenersatzmaterial zu bilden, liegen die Platten aneinander anliegend aber nicht miteinander verhakt oder gerastet vor (also nicht so wie in Figur 10 A) oder 11 oder 13A) gezeigt), so dass also die Pilze 34 der Stifte 32 benachbarter Platten noch nicht ineinander greifen. Zudem können die Platten mit einer Flüssigkeit benetzt vorliegen. In der Flüssigkeit ist bevorzugt zumindest eine pharmazeutisch wirksame Substanz enthalten, die zur Bekämpfung der Infektion oder zum Anregen des Knochenwachstums geeignet ist. Alternativ oder zusätzlich können die Platten mit einer solchen pharmazeutisch wirksamen Substanz beschichtet sein.

Das Knochenersatzmaterial kann geformt werden, indem die Platten über ihre Flächen ineinander gedrückt werden. Dadurch verhaken oder rasten die Platten miteinander und das Knochenersatzmaterial wird in der gewünschten Form verfestigt. Zuvor können die Platten auch durch elastische Verformung der Flächengebilde 31 verformt und an die Behandlungssituation angepasst werden. Nach dem Verhaken oder Rasten mit zumindest einer weiteren (meist dann ebenfalls verformten) Platte stabilisieren sich die beiden so miteinander verbundenen Platten gegenseitig, so dass die gewählte Form verfestigt ist.

Die Platten verbinden sich dabei untereinander in der Art, dass freie Zwischenräume zwischen den miteinander verbundenen Platten im Bereich der Stifte 32, der Pilze 34 und der Nuten 37 verbleiben, so dass der aus den Platten geformte Volumenköper in den Richtungen parallel zur Ebene der Platten offenporig ist. Die Platten haben einen Querschnitt beziehungsweise eine Dicke von etwa 6 mm, so dass die verbleibenden Poren einen freien Querschnitt im Bereich von etwa 0,6 mm aufweisen. Dieser Querschnitt reicht aus, damit sich in den Poren Knochenmaterial ausbilden beziehungsweise einwachsen kann. Der Volumenkörper mit seinen offenen Poren kann also als osteokonduktiv bezeichnet werden. Der aus den Platten geformte Volumenkörper ist daher als Knochenersatzmaterial gut geeignet.

Die Platten sollten fest ineinander gedrückt werden, so dass der Volumenkörper formstabil ist. Die Platten können dabei in einer ersten Stufe mit einander rasten, indem die Pilze 34 die Stifte 32 verbundener Platten elastisch verformen und durch die elastische Rückstellkraft der Stifte 32 die Pilze 34 beziehungsweise die Ränder der Pilze 34 in die Nuten 37 drücken und dadurch die Bewegung benachbarter Platten von dem Flächengebilde 31 weg begrenzen (siehe Figuren 10, 11 und 13 A). Die Platten können durch weiteres ineinander drücken der Platten in einer zweiten Stufe mit einander rasten, indem die Pilze 34 durch die Ausnehmungen 33 der Flächengebilde 31 hindurchgedrückt werden (nicht gezeigt). Dabei kann es zu einem Verklemmen der Pilze 34 mit den Ausnehmungen 33 kommen, die eine Bewegung der Pilze 34 gegen die benachbarte Platte verhindert und damit die beiden Platten miteinander rastet. Es ist auch möglich, dass erstens die Kanten der Pilze 34 die Stifte 32, die Nuten 37 oder die Ausnehmungen 33 oder die Pilze 34 oder zweitens die Kanten der Ausnehmungen 33 die Pilze 34 benachbarter Platten in geringem Umfang plastisch verformen und dadurch eine Rastung der Platten miteinander erfolgt.

Zwei Platten können ferner miteinander verbunden werden, indem die Platten an manchen Bereichen über die Pilze 34 und Nuten 37 miteinander gerastet werden und in anderen Bereichen über die Pilze 34 und Ausnehmungen 33 miteinander gerastet werden. Bevorzugt werden die Abmessungen der Pilze 34, die Tiefe der Ausnehmungen 33 (beziehungsweise die Dicke des Flächengebildes 31), die Form der Nuten 37 und die Länge der Stifte 32 zwischen dem Flächengebilde 31 und den Pilzen 34 so aufeinander abgestimmt, dass bei einem Verbinden der Platten die von dem Flächengebilde 31 abgewandten Oberflächen der Pilze 34 an der Oberfläche der Flächengebilde 31 benachbarter Platten anliegen und/oder bei einem Verbinden der Platten die von dem Flächengebilde 31 abgewandten Oberflächen der Pilze 34 an der Greiffläche 36 der Pilze 34 und bevorzugt entlang zumindest einer Linie oder besonders bevorzugt flächig an den Nuten 37 der Stifte 32 der benachbarten Platte anliegen. Hierdurch wird erreicht, dass die verbundenen Platten nicht ohne Verformung gegeneinander beweglich sind.

Die Nuten 37 verhindern auch, dass die Greifflächen 36 oder die gegenüberliegende Kappenoberseiten der Pilze 34 die Ausnehmungen 33 komplett abdecken. Damit die Ausnehmungen 33 von den Pilzen 34 leichter verformbar sind, können die Ausnehmungen 33 mehrere Schlitze aufweisen (nicht gezeigt), die über den Umfang der Ausnehmungen 33 verteilte sind. Die Breite der Schlitze sollte dazu ausreichen, dass sie osteokonduktiv wirken können.

Die vierte Ausführungsform nach den Figuren 10 bis 13 unterscheidet sich also von der nach den Figuren 1 bis 7 vor allem dadurch, dass die Stifte 32 dicker sind und Nuten 37 als zusätzliche Verbindungselemente aufweisen. Zudem weisen die Ausnehmungen 33 keine zusätzlichen Schlitze auf.

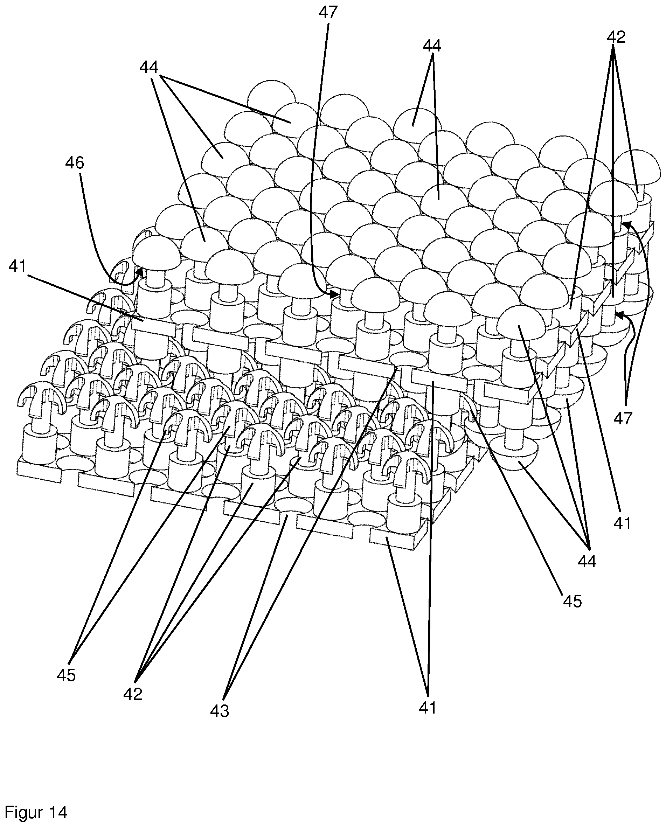

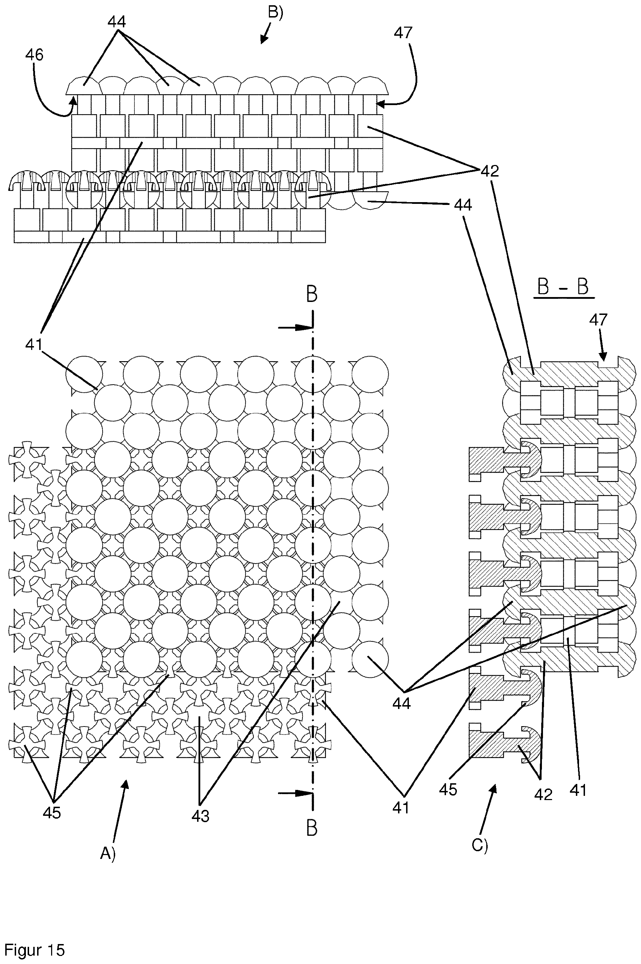

Figur 14 zeigt eine schematische perspektivische Ansicht auf zwei über die Verbindungselemente miteinander verbundene Platten eines fünften erfindungsgemäßen Knochenersatzmaterials. Figur 15 zeigt drei schematische Ansichten der zwei miteinander verbundenen Platten des fünften alternativen Knochenersatzmaterials nach Figur 14, und zwar Figur 15 A) eine Aufsicht auf die Oberseite, Figur 15 B) eine Seitenansicht und Figur 15 C) eine Querschnittansicht entlang des Schnitts B-B nach Figur 15 A).

Die Platten bestehen aus einem biokompatiblen Metall, insbesondere aus Edelstahl, Titan oder einer Titanlegierung, Tantal oder einer Tantallegierung können aber auch aus einem elastischen biokompatiblen Kunststoff oder aus einem Komposit solcher Materialien gefertigt sein. Die Platten werden mit einem CAM-Verfahren beziehungsweise einem 3D-Druckverfahren hergestellt, beispielsweise mit selektivem Elektronenstrahlschmelzen (EBM). Andere Rapid-Prototyping-Verfahren beziehungsweise computergestützte generative Herstellungsverfahren können auch zur Herstellung der Platten verwendet werden.

Die Platten weisen jeweils ein plattenförmiges Flächengebilde 41 auf, das die gesamten Platten trägt und in sich verbindet. Das Flächengebilde 41 ist flexibel und elastisch verformbar, so dass auch andere Flächen als Ebenen mit dem Flächengebilde 41 geformt werden können. Von dem Flächengebilde 41 erstrecken sich bei jeder Platte eine Vielzahl von Stiften 42 weg, die senkrecht von der Ebene der Flächengebilde 41 abstehen. In dem Flächengebilde 41 sind zwischen den Stiften 42 eine Vielzahl von durchgehenden Ausnehmungen 43 angeordnet, die, wenn die Platten miteinander zu einem Volumenkörper verbunden sind, eine offene Porosität des Volumenkörpers in einer Richtung senkrecht zu den Flächengebilden 41 bewirken können, wenn nicht die benachbarten Platten anliegen und dadurch die Ausnehmungen 43 abdecken.

In den Figuren 14 und 15 sind zwei verschiedene Typen von Platten dargestellt nämlich erstens Grundplatten, die eine flache Unterseite aufweisen und bei denen sich die Stifte 42 nur auf einer Seite der Flächengebilde 41 von dem Flächengebilde 41 aus erstrecken, und zweitens Aufbauplatten, bei denen sich die Stifte 42 von beiden Seiten des Flächengebildes 41 aus erstrecken. Die Grundplatten sind in Figur 14 unten beziehungsweise darunter, in Figur 15 A) unten (in der Bildebene), in Figur 15 B) unten und in Figur 15 C) links gezeigt. Die Aufbauplatten sind in Figur 14, in Figur 15 A) oben (aus der Bildebene), in Figur 15 B) oben und in Figur 15 C) rechts gezeigt. Die Grundplatten können großflächig anliegend auf dem zu behandelnden Knochen befestigt werden. Die Aufbauplatten können allerdings ebenfalls auf dem zu behandelnden Knochen befestigt werden, wenn auch mit einer geringeren Auflagefläche, so dass auf die Grundplatten verzichtet werden kann.

An den ansonsten zylindrischen Stiften 42 sind an dem den Flächengebilden 41 gegenüberliegenden Enden der Stifte 42 Pilze 44 oder Gruppen von jeweils vier Haken 45 als Verbindungselemente 44, 45 vorgesehen. Die Pilze 44 sind nach außen (von dem Flächengebilde 41 weg) abgerundet und bilden Kugelabschnitte. Es sind aber auch andere Abrundungen, wie beispielsweise Ellipsenabschnitte möglich. Die Haken 45 sind in gleicher Weise nach außen abgerundet. An der zum Flächengebilde 41 hin ausgerichteten Seite bilden die Pilze 44 eine ebene Greiffläche 46, die zum Verhaken oder Rasten mit anderen Pilzen 44 und Haken 45 eingreifender Platten oder mit den Ausnehmungen 43 eingreifender Platten geeignet sind. Dementsprechend bilden die Haken 45 an der zum Flächengebilde 41 hin ausgerichteten Seite Hinterschnitte, die zum Verhaken oder Rasten mit anderen Pilzen 44 und Haken 45 eingreifender Platten oder mit den Ausnehmungen 43 eingreifender Platten geeignet sind.

In den Stiften 42 sind zudem angrenzend zu den Greifflächen 46 und angrenzend zu den Haken 45 Nuten 47 als Verbindungselemente 47 vorgesehen, in die die Pilze 44 und Haken 45 benachbarter Platten eingreifen beziehungsweise einrasten können.

Hierzu können die Nuten 47 im Gegensatz zu den gezeigten Nuten 47, aber erfindungsgemäß bevorzugt, als Negativ der Form der Wölbung der Pilze 44 beziehungsweise der Haken 45 geformt sein, damit die Pilze 44 und Haken 45 gut in die Nuten 47 passen.

Bei der vorliegenden fünften Ausführungsform sind an der Grundplatte ausschließlich Haken 45 als Verbindungselemente 45 vorgesehen und an der Aufbauplatte ausschließlich Pilze 44 als Verbindungselemente 44 vorgesehen. Das kann auch umgekehrt sein und die Haken 45 und Pilze 44 können auch gemischt vorliegen.

Um ein erfindungsgemäßes Knochenersatzmaterial zu bilden, liegen die Platten aneinander anliegend aber nicht miteinander verhakt oder gerastet vor (also nicht so wie in Figur 14 oder 15 gezeigt), so dass also die Pilze 44 und Haken 45 der Stifte 42 benachbarter Platten noch nicht ineinander greifen. Zudem können die Platten mit einer Flüssigkeit benetzt vorliegen. In der Flüssigkeit ist bevorzugt zumindest eine pharmazeutisch wirksame Substanz enthalten, die zur Bekämpfung der Infektion oder zum Anregen des Knochenwachstums geeignet ist. Alternativ oder zusätzlich können die Platten mit einer solchen pharmazeutisch wirksamen Substanz beschichtet sein.

Das Knochenersatzmaterial kann geformt werden, indem die Platten über ihre Flächen ineinander gedrückt werden. Dadurch verhaken oder rasten die Platten miteinander und das Knochenersatzmaterial wird in der gewünschten Form verfestigt. Zuvor können die Platten auch durch elastische Verformung der Flächengebilde 41 verformt und an die Behandlungssituation angepasst werden. Nach dem Verhaken oder Rasten mit zumindest einer weiteren (meist dann ebenfalls verformten) Platte stabilisieren sich die beiden so miteinander verbundenen Platten gegenseitig, so dass die gewählte Form verfestigt ist.

Die Platten verbinden sich dabei untereinander in der Art, dass freie Zwischenräume zwischen den miteinander verbundenen Platten im Bereich der Stifte 42, der Pilze 44, der Haken 45 und der Nuten 47 verbleiben, so dass der aus den Platten geformte Volumenköper in den Richtungen parallel zur Ebene der Platten offenporig ist. Die Platten haben einen Querschnitt beziehungsweise eine Dicke von etwa 7 mm, so dass die verbleibenden Poren einen freien Querschnitt im Bereich von etwa 0,7 mm aufweisen. Dieser Querschnitt reicht aus, damit sich in den Poren Knochenmaterial ausbilden beziehungsweise einwachsen kann. Der Volumenkörper mit seinen offenen Poren kann also als osteokonduktiv bezeichnet werden. Der aus den Platten geformte Volumenkörper ist daher als Knochenersatzmaterial gut geeignet.

Die Platten sollten fest ineinander gedrückt werden, so dass der Volumenkörper formstabil ist. Die Platten können dabei in einer ersten Stufe mit einander rasten, indem die Pilze 44 und Haken 45 die Stifte 42 verbundener Platten elastisch verformen und durch die elastische Rückstellkraft der Stifte 42 die Pilze 44 und Haken 45 beziehungsweise die Ränder der Pilze 44 und Spitzen der Haken 45 in die Nuten 47 drücken und dadurch die Bewegung benachbarter Platten von dem Flächengebilde 41 weg begrenzen (siehe Figuren 14 und 15). Die Platten können durch weiteres ineinander drücken der Platten in einer zweiten Stufe mit einander rasten, indem die Pilze 44 und Haken 45 durch die Ausnehmungen 43 der Flächengebilde 41 hindurchgedrückt werden (nicht gezeigt). Dabei kann es zu einem Verklemmen der Pilze 44 und Haken 45 mit den Ausnehmungen 43 kommen, die eine Bewegung der Pilze 44 und Haken 45 gegen die benachbarte Platte verhindert und damit die beiden Platten miteinander rastet. Es ist auch möglich, dass erstens die Kanten der Pilze 44 und/oder die Spitzen der Haken 45 die Stifte 42, die Nuten 47 oder die Ausnehmungen 43 oder die Pilze 44 oder zweitens die Kanten der Ausnehmungen 43 die Pilze 44 und/oder Haken 45 benachbarter Platten in geringem Umfang plastisch verformen und dadurch eine Rastung der Platten miteinander erfolgt.

Zwei Platten können ferner miteinander verbunden werden, indem die Platten an manchen Bereichen über die Pilze 44, Haken 45 und Nuten 47 miteinander gerastet werden und in anderen Bereichen über die Pilze 44 und Haken 45 mit den Ausnehmungen 43 gerastet werden. Bevorzugt werden die Abmessungen der Pilze 44, der Haken 45, die Tiefe der Ausnehmungen 43 (beziehungsweise die Dicke des Flächengebildes 41), die Form der Nuten 47 und die Länge der Stifte 42 zwischen dem Flächengebilde 41 und den Pilzen 44 oder Haken 45 so aufeinander abgestimmt, dass bei einem Verbinden der Platten die von dem Flächengebilde 41 abgewandten Oberflächen der Pilze 44 und Haken 45 an der Oberfläche der Flächengebilde 41 benachbarter Platten anliegen und/oder bei einem Verbinden der Platten die von dem Flächengebilde 41 abgewandten Oberflächen der Pilze 44 und Haken 45 an der Greiffläche 46 der Pilze 44 und bevorzugt entlang zumindest einer Linie oder besonders bevorzugt flächig an den Nuten 47 der Stifte 42 der benachbarten Platte anliegen. Hierdurch wird erreicht, dass die verbundenen Platten nicht ohne Verformung gegeneinander beweglich sind.

Die Nuten 47 verhindern auch, dass die Greifflächen 46 oder die gegenüberliegende Kappenoberseiten der Pilze 44 oder die Haken 45 die Ausnehmungen 43 komplett abdecken. Damit die Ausnehmungen 43 von den Pilzen 44 und Haken 45 leichter verformbar sind, können die Ausnehmungen 43 mehrere Schlitze aufweisen (nicht gezeigt), die über den Umfang der Ausnehmungen 43 verteilte sind. Die Breite der Schlitze sollte dazu ausreichen, dass sie osteokonduktiv wirken können.

Die fünfte Ausführungsform nach den Figuren 14 und 15 unterscheidet sich also von der nach den Figuren 1 bis 7 vor allem dadurch, dass die Stifte 42 dicker sind und Nuten 47 aufweisen sowie dadurch, dass Haken 45 als Verbindungselemente 45 vorgesehen sind. Zudem weisen die Ausnehmungen 43 keine zusätzlichen Schlitze auf.

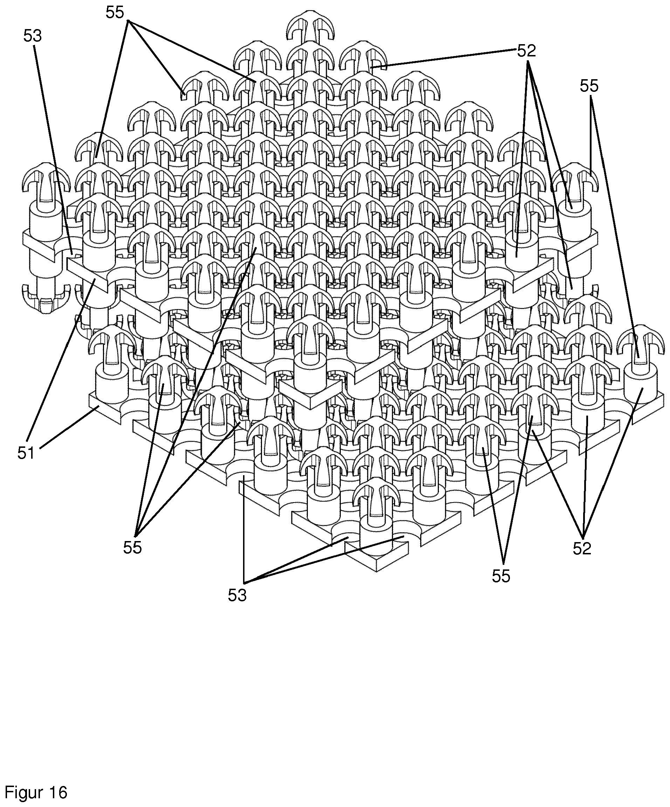

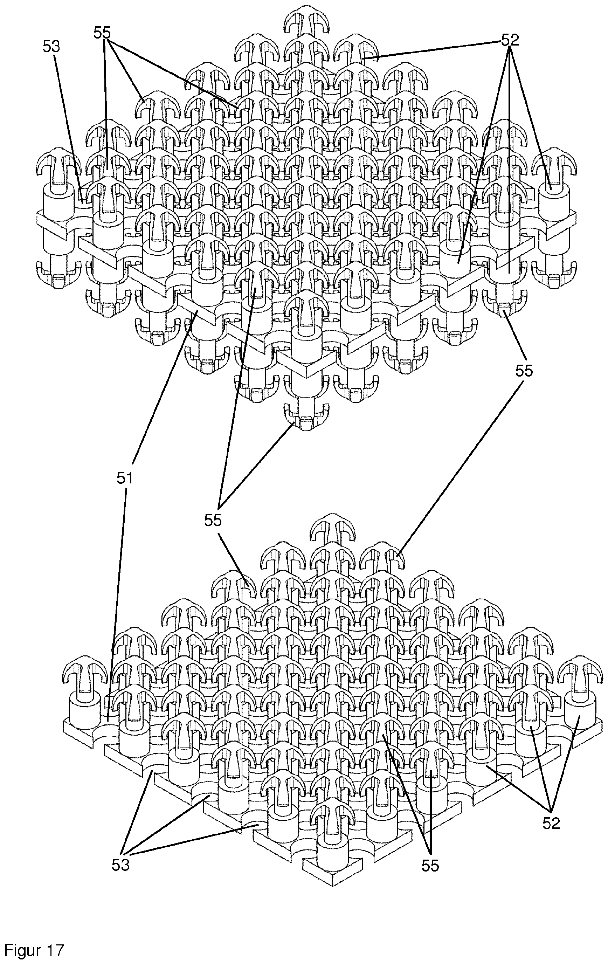

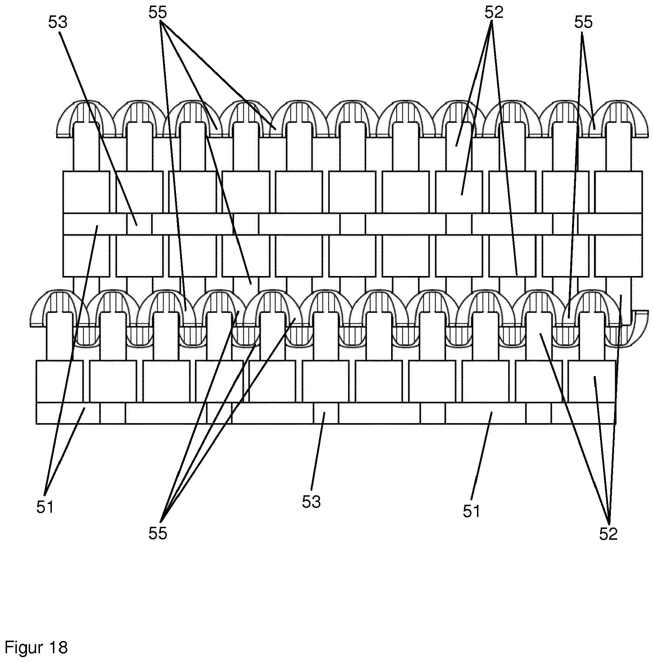

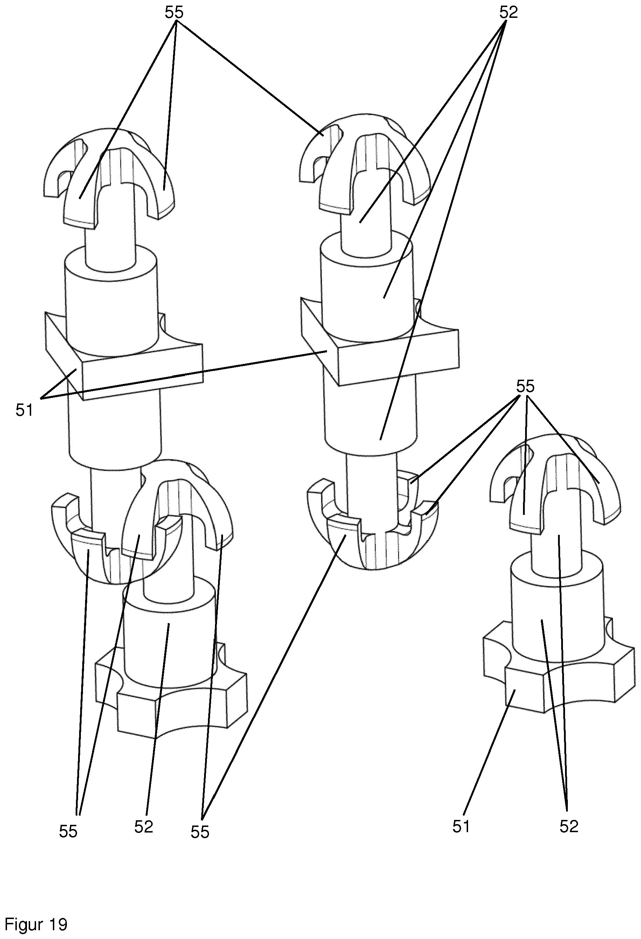

Figur 16 zeigt eine schematische perspektivische Ansicht auf zwei miteinander verhakte Platten eines sechsten erfindungsgemäßen Knochenersatzmaterials und Figur 17 eine schematische perspektivische Ansicht auf zwei nicht miteinander verhakte Platten des sechsten erfindungsgemäßen Knochenersatzmaterials nach Figur 16. Figur 18 zeigt eine schematische Seitenansicht auf miteinander verhakte Platten des sechsten erfindungsgemäßen Knochenersatzmaterials nach Figur 16. Schließlich zeigt Figur 19 eine schematische perspektivische Detailansicht auf zwei miteinander verhakte Haken (links) zwei nicht miteinander verhakte Haken (rechts) des sechsten erfindungsgemäßen Knochenersatzmaterials nach den Figuren 16 bis 18.

Die Platten bestehen aus einem biokompatiblen Material, insbesondere aus Edelstahl, Titan oder einer Titanlegierung, Tantal oder einer Tantallegierung oder aus einem elastischen biokompatiblen Kunststoff. Sie können aber auch aus einem Komposit solcher Materialien gefertigt sein. Die Platten werden mit einem CAM-Verfahren beziehungsweise einem 3D-Druckverfahren hergestellt. Die zu den anderen Ausführungsbeispielen genannten Rapid-Prototyping-Verfahren beziehungsweise computergestützten generativen Herstellungsverfahren können zur Herstellung der Platten verwendet werden.

Die Platten weisen jeweils ein plattenförmiges Flächengebilde 51 auf, das die gesamten Platten trägt und in sich verbindet. Das Flächengebilde 51 ist flexibel und elastisch verformbar, so dass auch andere Flächen als Ebenen mit dem Flächengebilde 51 geformt werden können. Von dem Flächengebilde 51 erstrecken sich bei jeder Platte eine Vielzahl von Stiften 52 weg, die senkrecht von der Ebene der Flächengebilde 51 abstehen. In dem Flächengebilde 51 sind zwischen den Stiften 52 eine Vielzahl von durchgehenden Ausnehmungen 53 angeordnet, die, wenn die Platten miteinander zu einem Volumenkörper verbunden sind, eine offene Porosität des Volumenkörpers in einer Richtung senkrecht zu den Flächengebilden 51 bewirken.

In den Figuren 16 bis 18 sind zwei verschiedene Typen von Platten dargestellt nämlich erstens Grundplatten, die eine flache Unterseite aufweisen und bei denen sich die Stifte 52 nur auf einer Seite der Flächengebilde 51 von dem Flächengebilde 51 aus erstrecken, und zweitens Aufbauplatten, bei denen sich die Stifte 52 von beiden Seiten des Flächengebildes 51 aus erstrecken. Die Grundplatten sind in Figur 16 unten beziehungsweise darunter, in Figur 17 und 18 unten gezeigt. Die Aufbauplatten sind in Figur 16 oben auf und in den Figuren 17 und 18 oben auf gezeigt. Die Grundplatten können großflächig anliegend auf dem zu behandelnden Knochen befestigt werden. Die Aufbauplatten können allerdings ebenfalls auf dem zu behandelnden Knochen befestigt werden, wenn auch mit einer geringeren Auflagefläche, so dass auf die Grundplatten verzichtet werden kann.

An den ansonsten zylindrischen Stiften 52 sind an dem den Flächengebilden 51 gegenüberliegenden Enden der Stifte 52 Gruppen von jeweils vier Haken 55 als Verbindungselemente 55 vorgesehen. Die Haken 55 sind nach außen (von dem Flächengebilde 51 weg) abgerundet und bilden Teile von Kugeloberflächen. Es sind aber auch andere Abrundungen, wie beispielsweise Ellipsenabschnitte möglich. An der zum Flächengebilde 51 hin ausgerichteten Seite bilden die Haken 55 Hinterschnitte, die zum Verhaken oder Rasten mit anderen Haken 55 eingreifender Platten oder mit den Ausnehmungen 53 eingreifender Platten geeignet sind.

Die Stifte 52 sind im Bereich angrenzend zu den Haken 55 beziehungsweise den Hinterschnitten der Haken 55 dünner beziehungsweise mit einem geringeren Querschnitt ausgeformt. In die dünneren Bereiche können die Haken 55 benachbarter Platten leichter eingreifen beziehungsweise einrasten.

Bei der vorliegenden sechsten Ausführungsform sind ausschließlich Haken 55 als Verbindungselemente 55 vorgesehen. Um ein erfindungsgemäßes Knochenersatzmaterial zu bilden, liegen die Platten aneinander anliegend aber nicht miteinander verhakt oder gerastet vor (also nicht so wie in Figur 16 oder 18 gezeigt), so dass also die Haken 55 der Stifte 52 benachbarter Platten noch nicht ineinander greifen. Zudem können die Platten mit einer Flüssigkeit benetzt vorliegen. In der Flüssigkeit ist bevorzugt zumindest eine pharmazeutisch wirksame Substanz enthalten, die zur Bekämpfung der Infektion oder zum Anregen des Knochenwachstums geeignet ist. Alternativ oder zusätzlich können die Platten mit einer solchen pharmazeutisch wirksamen Substanz beschichtet sein.

Das Knochenersatzmaterial kann geformt werden, indem die Platten über ihre Flächen ineinander gedrückt werden. Dadurch verhaken oder rasten die Platten miteinander und das Knochenersatzmaterial wird in der gewünschten Form verfestigt. Zuvor können die Platten auch durch elastische Verformung der Flächengebilde 51 verformt und an die Behandlungssituation angepasst werden. Nach dem Verhaken oder Rasten mit zumindest einer weiteren (meist dann ebenfalls verformten) Platte stabilisieren sich die beiden so miteinander verbundenen Platten gegenseitig, so dass die gewählte Form verfestigt ist. Das Verhaken ist in Figur 19 im Detail dargestellt.

Die Platten verbinden sich dabei untereinander in der Art, dass freie Zwischenräume zwischen den miteinander verbundenen Platten im Bereich der Stifte 52 und der Haken 55 verbleiben, so dass der aus den Platten geformte Volumenköper in den Richtungen parallel zur Ebene der Platten offenporig ist. Die Platten haben einen Querschnitt beziehungsweise eine Dicke von etwa 5 mm, so dass die verbleibenden Poren einen freien Querschnitt im Bereich von etwa 0,5 mm aufweisen. Dieser Querschnitt reicht aus, damit sich in den Poren Knochenmaterial ausbilden beziehungsweise einwachsen kann. Der Volumenkörper mit seinen offenen Poren kann also als osteokonduktiv bezeichnet werden. Der aus den Platten geformte Volumenkörper ist daher als Knochenersatzmaterial gut geeignet.

Die Platten sollten fest ineinander gedrückt werden, so dass der Volumenkörper formstabil ist. Die Platten können dabei in einer ersten Stufe mit einander rasten, indem die Haken 55 die Stifte 52 verbundener Platten elastisch verformen und durch die elastische Rückstellkraft der Stifte 52 die Haken 55 beziehungsweise Spitzen der Haken 55 ineinander drücken und dadurch die Bewegung benachbarter Platten von dem Flächengebilde 51 weg begrenzen (siehe Figuren 16, 18 und 19). Die Platten können durch weiteres ineinander drücken der Platten in einer zweiten Stufe mit einander rasten, indem die Haken 55 durch die Ausnehmungen 53 der Flächengebilde 51 hindurchgedrückt werden (nicht gezeigt). Dabei kann es zu einem Verklemmen der Haken 55 mit den Ausnehmungen 53 kommen, die eine Bewegung der Haken 55 gegen die benachbarte Platte verhindert und damit die beiden Platten miteinander rastet. Es ist auch möglich, dass erstens die Spitzen der Haken 55 die Stifte 52 oder die Ausnehmungen 53 oder zweitens die Kanten der Ausnehmungen 53 die Haken 55 benachbarter Platten in geringem Umfang plastisch verformen und dadurch eine Rastung der Platten miteinander erfolgt.

Zwei Platten können ferner miteinander verbunden werden, indem die Platten an manchen Bereichen über die Haken 55 miteinander gerastet werden und in anderen Bereichen über die Haken 55 mit den Ausnehmungen 53 gerastet werden. Bevorzugt werden die Abmessungen der Haken 55, die Tiefe der Ausnehmungen 53 (beziehungsweise die Dicke des Flächengebildes 51) und die Länge der Stifte 52 zwischen dem Flächengebilde 51 und den Haken 55 so aufeinander abgestimmt, dass bei einem Verbinden der Platten die von dem Flächengebilde 51 abgewandten Seiten der Haken 55 an der Oberfläche der Flächengebilde 51 benachbarter Platten anliegen und/oder bei einem Verbinden der Platten die von dem Flächengebilde 51 abgewandten Seiten der Haken 55 in den Hinterschnitten der Haken 55 anliegen. Hierdurch wird erreicht, dass die verbundenen Platten nicht ohne Verformung gegeneinander beweglich sind.

Die Form der Haken 55 verhindert, dass diese die Ausnehmungen 53 vollständig abdecken. Damit die Ausnehmungen 53 von den Haken 55 leichter verformbar sind, können die Ausnehmungen 53 mehrere Schlitze aufweisen (nicht gezeigt), die über den Umfang der Ausnehmungen 53 verteilte sind.

Die sechste Ausführungsform nach den Figuren 16 bis 19 unterscheidet sich also von der nach den Figuren 1 bis 7 vor allem dadurch, dass die Stifte 52 bereichsweise dicker sind und vor allem dadurch, dass Haken 55 als Verbindungselemente 55 vorgesehen sind. Zudem weisen die Ausnehmungen 53 keine zusätzlichen Schlitze auf.

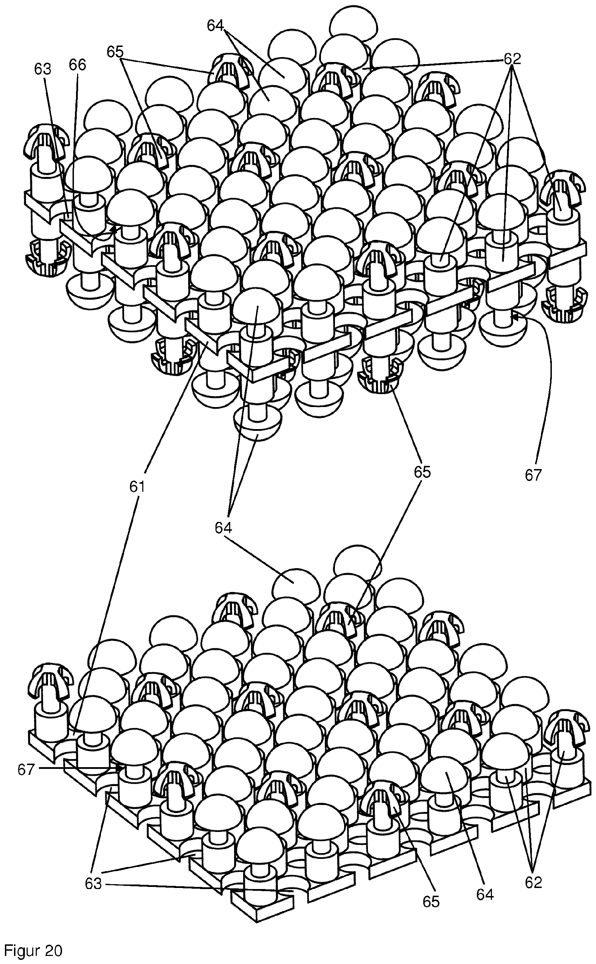

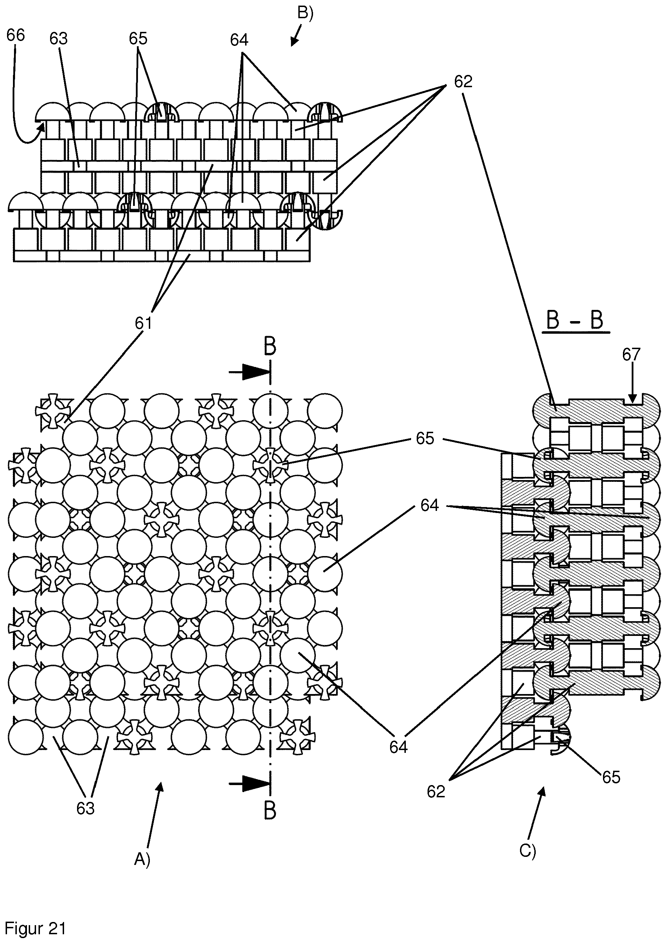

Figur 20 zeigt eine schematische perspektivische Ansicht auf zwei nicht miteinander verhakte Platten eines siebten erfindungsgemäßen Knochenersatzmaterials und Figur 21 drei schematische Ansichten von zwei miteinander verbundenen Platten des siebten alternativen Knochenersatzmaterials A) als Aufsicht auf Oberseite, B) als Seitenansicht und C) als Querschnittansicht entlang des Schnitts B-B nach Figur 21 A).

Die Platten bestehen aus einem biokompatiblen Metall, insbesondere aus Edelstahl, Titan oder einer Titanlegierung, Tantal oder einer Tantallegierung können aber auch aus einem elastischen biokompatiblen Kunststoff oder aus einem Komposit solcher Materialien gefertigt sein. Die Platten werden mit einem CAM-Verfahren beziehungsweise einem 3D-Druckverfahren hergestellt, beispielsweise mit selektivem Elektronenstrahlschmelzen (EBM). Andere Rapid-Prototyping-Verfahren beziehungsweise computergestützte generative Herstellungsverfahren können auch zur Herstellung der Platten verwendet werden.

Die Platten weisen jeweils ein plattenförmiges Flächengebilde 61 auf, das die gesamten Platten trägt und in sich verbindet. Das Flächengebilde 61 ist flexibel und elastisch verformbar, so dass auch andere Flächen als Ebenen mit dem Flächengebilde 61 geformt werden können. Von dem Flächengebilde 61 erstrecken sich bei jeder Platte eine Vielzahl von Stiften 62 weg, die senkrecht von der Ebene der Flächengebilde 61 abstehen. In dem Flächengebilde 61 sind zwischen den Stiften 62 eine Vielzahl von durchgehenden Ausnehmungen 63 angeordnet, die, wenn die Platten miteinander zu einem Volumenkörper verbunden sind, eine offene Porosität des Volumenkörpers in einer Richtung senkrecht zu den Flächengebilden 61 bewirken können, wenn nicht die benachbarten Platten anliegen und dadurch die Ausnehmungen 63 abdecken.

In den Figuren 20 und 21 sind zwei verschiedene Typen von Platten dargestellt nämlich erstens Grundplatten, die eine flache Unterseite aufweisen und bei denen sich die Stifte 62 nur auf einer Seite der Flächengebilde 61 von dem Flächengebilde 61 aus erstrecken, und zweitens Aufbauplatten, bei denen sich die Stifte 62 von beiden Seiten des Flächengebildes 61 aus erstrecken. Die Grundplatten sind in Figur 20 unten, in Figur 21 A) unten (in der Bildebene), in Figur 21 B) unten und in Figur 21 C) links gezeigt. Die Aufbauplatten sind in Figur 20 oben und in Figur 21 oben auf, also in Figur 21 A) oben (aus der Bildebene), in Figur 21 B) oben und in Figur 21 C) rechts gezeigt. Die Grundplatten können großflächig anliegend auf dem zu behandelnden Knochen befestigt werden. Die Aufbauplatten können allerdings ebenfalls auf dem zu behandelnden Knochen befestigt werden, wenn auch mit einer geringeren Auflagefläche, so dass auf die Grundplatten verzichtet werden kann.

An den ansonsten zylindrischen Stiften 62 sind an dem den Flächengebilden 61 gegenüberliegenden Enden der Stifte 62 Pilze 64 oder Gruppen von jeweils vier Haken 65 als Verbindungselemente 64, 65 vorgesehen. Die Pilze 64 sind nach außen (von dem Flächengebilde 61 weg) abgerundet und bilden Kugelabschnitte. Es sind aber auch andere Abrundungen, wie beispielsweise Ellipsenabschnitte möglich. Die Haken 65 sind in gleicher Weise nach außen abgerundet. An der zum Flächengebilde 61 hin ausgerichteten Seite bilden die Pilze 64 eine ebene Greiffläche 66, die zum Verhaken oder Rasten mit anderen Pilzen 64 und Haken 65 eingreifender Platten oder mit den Ausnehmungen 63 eingreifender Platten geeignet sind. Dementsprechend bilden die Haken 65 an der zum Flächengebilde 61 hin ausgerichteten Seite Hinterschnitte, die zum Verhaken oder Rasten mit anderen Pilzen 64 und Haken 65 eingreifender Platten oder mit den Ausnehmungen 63 eingreifender Platten geeignet sind.

In den Stiften 62 sind zudem angrenzend zu den Greifflächen 66 und angrenzend zu den Haken 65 Nuten 67 als Verbindungselemente 67 vorgesehen, in die die Pilze 64 und Haken 65 benachbarter Platten eingreifen beziehungsweise einrasten können. Hierzu können die Nuten 67 im Gegensatz zu den gezeigten Nuten 67, aber erfindungsgemäß bevorzugt, als Negativ der Form der Wölbung der Pilze 64 beziehungsweise der Haken 65 geformt sein, damit die Pilze 64 und Haken 65 gut in die Nuten 67 passen.

Bei der vorliegenden siebten Ausführungsform sind an der an den Platten Pilze 64 und Haken 65 gemischt als Verbindungselemente 64, 65 vorgesehen, wobei zwei von elf Verbindungselementen 64, 65 Haken 65 sind und der Rest Pilze 64. Das kann auch umgekehrt sein und die Haken 65 und Pilze 64 können auch in einem anderen Mischungsverhältnis vorliegen.

Um ein erfindungsgemäßes Knochenersatzmaterial zu bilden, liegen die Platten aneinander anliegend aber nicht miteinander verhakt oder gerastet vor (also nicht so wie in Figur 21 gezeigt), so dass also die Pilze 64 und Haken 65 der Stifte 62 benachbarter Platten noch nicht ineinander greifen. Zudem können die Platten mit einer Flüssigkeit benetzt vorliegen. In der Flüssigkeit ist bevorzugt zumindest eine pharmazeutisch wirksame Substanz enthalten, die zur Bekämpfung der Infektion oder zum Anregen des Knochenwachstums geeignet ist. Alternativ oder zusätzlich können die Platten mit einer solchen pharmazeutisch wirksamen Substanz beschichtet sein.

Das Knochenersatzmaterial kann geformt werden, indem die Platten über ihre Flächen ineinander gedrückt werden. Dadurch verhaken oder rasten die Platten miteinander und das Knochenersatzmaterial wird in der gewünschten Form verfestigt. Zuvor können die Platten auch durch elastische Verformung der Flächengebilde 61 verformt und an die Behandlungssituation angepasst werden. Nach dem Verhaken oder Rasten mit zumindest einer weiteren (meist dann ebenfalls verformten) Platte stabilisieren sich die beiden so miteinander verbundenen Platten gegenseitig, so dass die gewählte Form verfestigt ist.

Die Platten verbinden sich dabei untereinander in der Art, dass freie Zwischenräume zwischen den miteinander verbundenen Platten im Bereich der Stifte 62, der Pilze 64, der Haken 65 und der Nuten 67 verbleiben, so dass der aus den Platten geformte Volumenköper in den Richtungen parallel zur Ebene der Platten offenporig ist. Die Platten haben einen Querschnitt beziehungsweise eine Dicke von etwa 9 mm, so dass die verbleibenden Poren einen freien Querschnitt im Bereich von etwa 0,9 mm aufweisen. Dieser Querschnitt reicht aus, damit sich in den Poren Knochenmaterial ausbilden beziehungsweise einwachsen kann. Der Volumenkörper mit seinen offenen Poren kann also als osteokonduktiv bezeichnet werden. Der aus den Platten geformte Volumenkörper ist daher als Knochenersatzmaterial gut geeignet.

Die Platten sollten fest ineinander gedrückt werden, so dass der Volumenkörper formstabil ist. Die Platten können dabei in einer ersten Stufe mit einander rasten, indem die Pilze 64 und Haken 65 die Stifte 62 verbundener Platten elastisch verformen und durch die elastische Rückstellkraft der Stifte 62 die Pilze 64 und Haken 65 beziehungsweise die Ränder der Pilze 64 und Spitzen der Haken 65 in die Nuten 67 drücken und dadurch die Bewegung benachbarter Platten von dem Flächengebilde 61 weg begrenzen (siehe Figur 21). Die Platten können durch weiteres ineinander drücken der Platten in einer zweiten Stufe mit einander rasten, indem die Pilze 64 und Haken 65 durch die Ausnehmungen 63 der Flächengebilde 61 hindurchgedrückt werden (nicht gezeigt). Dabei kann es zu einem Verklemmen der Pilze 64 und Haken 65 mit den Ausnehmungen 63 kommen, die eine Bewegung der Pilze 64 und Haken 65 gegen die benachbarte Platte verhindert und damit die beiden Platten miteinander rastet. Es ist auch möglich, dass erstens die Kanten der Pilze 64 und/oder die Spitzen der Haken 65 die Stifte 62, die Nuten 67 oder die Ausnehmungen 63 oder die Pilze 64 oder zweitens die Kanten der Ausnehmungen 63 die Pilze 64 und/oder Haken 65 benachbarter Platten in geringem Umfang plastisch verformen und dadurch eine Rastung der Platten miteinander erfolgt.

Zwei Platten können ferner miteinander verbunden werden, indem die Platten an manchen Bereichen über die Pilze 64, Haken 65 und Nuten 67 miteinander gerastet werden und in anderen Bereichen über die Pilze 64 und/oder Haken 65 mit den Ausnehmungen 63 gerastet werden. Bevorzugt werden die Abmessungen der Pilze 64, der Haken 65, die Tiefe der Ausnehmungen 63 (beziehungsweise die Dicke des Flächengebildes 61), die Form der Nuten 67 und die Länge der Stifte 62 zwischen dem Flächengebilde 61 und den Pilzen 64 oder Haken 65 so aufeinander abgestimmt, dass bei einem Verbinden der Platten die von dem Flächengebilde 61 abgewandten Oberflächen der Pilze 64 und Haken 65 an der Oberfläche der Flächengebilde 61 benachbarter Platten anliegen und/oder bei einem Verbinden der Platten die von dem Flächengebilde 61 abgewandten Oberflächen der Pilze 64 und Haken 65 an der Greiffläche 66 der Pilze 64 und bevorzugt entlang zumindest einer Linie oder besonders bevorzugt flächig an den Nuten 67 der Stifte 62 der benachbarten Platte anliegen. Hierdurch wird erreicht, dass die verbundenen Platten nicht ohne Verformung gegeneinander beweglich sind.

Die Nuten 67 verhindern auch, dass die Greifflächen 66 oder die gegenüberliegende Kappenoberseiten der Pilze 64 oder die Haken 65 die Ausnehmungen 63 komplett abdecken. Damit die Ausnehmungen 63 von den Pilzen 64 und Haken 65 leichter verformbar sind, können die Ausnehmungen 63 mehrere Schlitze aufweisen (nicht gezeigt), die über den Umfang der Ausnehmungen 63 verteilte sind. Die Breite der Schlitze sollte dazu ausreichen, dass sie osteokonduktiv wirken können.

Die siebte Ausführungsform nach den Figuren 20 und 21 unterscheidet sich also von der nach den Figuren 1 bis 7 vor allem dadurch, dass die Stifte 62 dicker sind und Nuten 67 aufweisen sowie dadurch, dass Haken 65 als Verbindungselemente 65 vorgesehen sind. Zudem weisen die Ausnehmungen 63 keine zusätzlichen Schlitze auf.

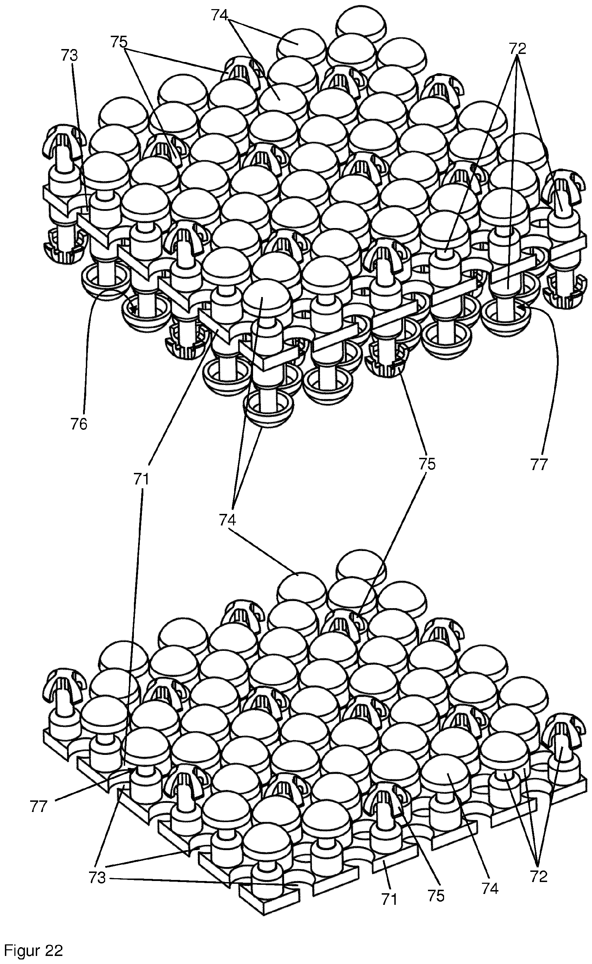

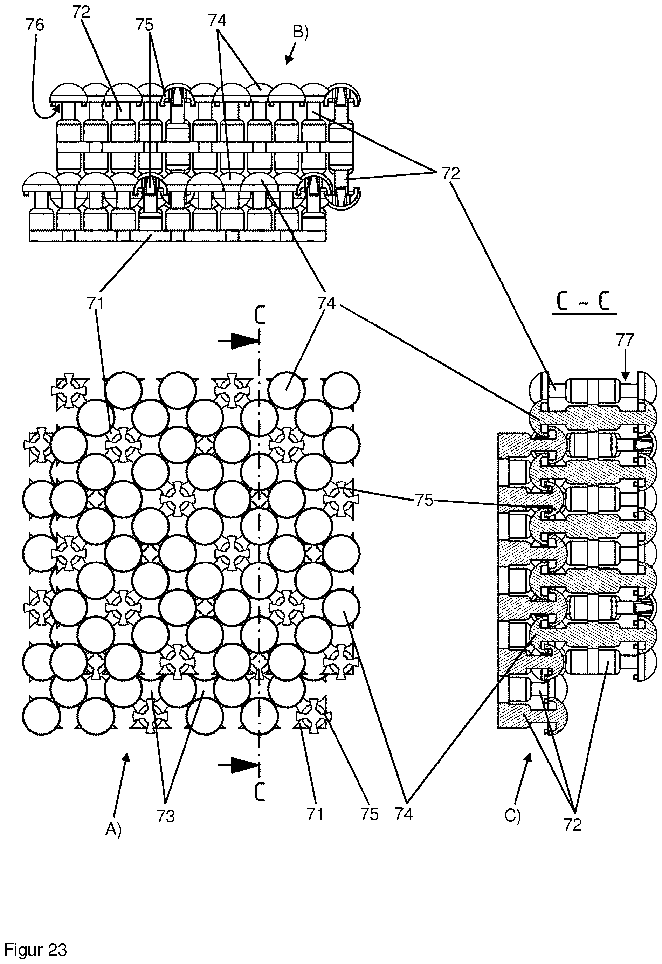

Figur 22 zeigt eine schematische perspektivische Ansicht auf zwei nicht miteinander verhakte Platten eines achten erfindungsgemäßen Knochenersatzmaterials und Figur 23 drei schematische Ansichten von zwei miteinander verbundenen Platten des achten alternativen Knochenersatzmaterials - Figur 23 A) eine Aufsicht auf Oberseite, Figur 23 B) eine Seitenansicht und Figur 23 C) eine Querschnittansicht entlang des Schnitts C-C nach Figur 23 A).

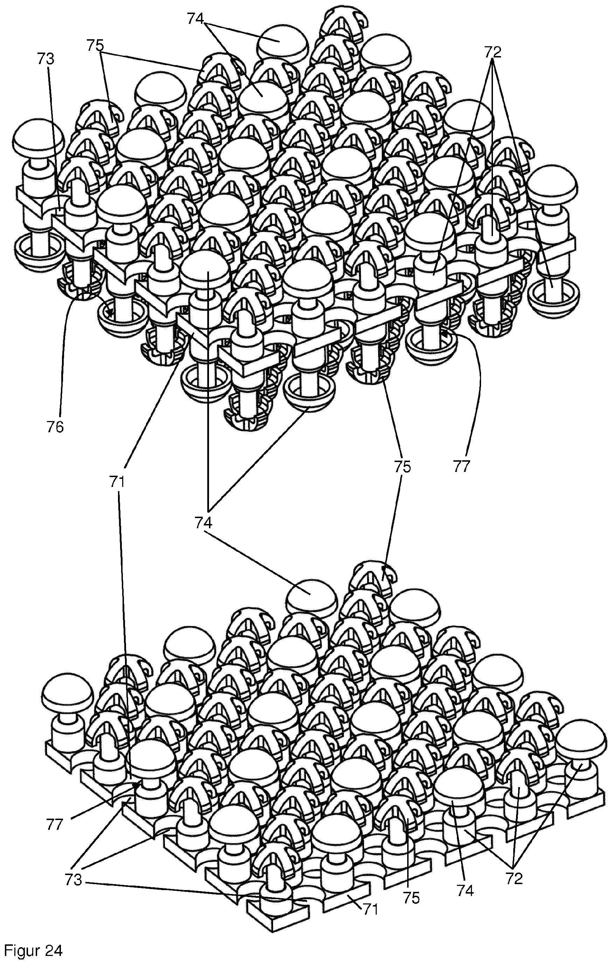

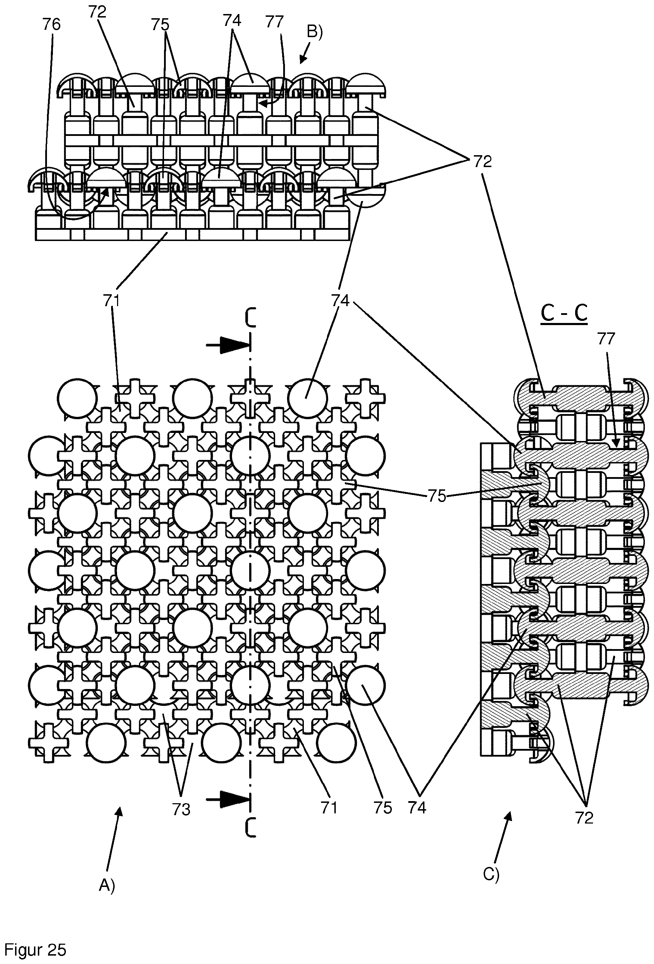

Figur 24 zeigt eine schematische perspektivische Ansicht auf zwei nicht miteinander verhakte Platten einer Modifikation des achten erfindungsgemäßen Knochenersatzmaterials und Figur 25 drei schematische Ansichten von zwei miteinander verbundenen Platten der Modifikation des achten alternativen Knochenersatzmaterials - Figur 25 A) eine Aufsicht auf Oberseite, Figur 25 B) eine Seitenansicht und Figur 25 C) eine Querschnittansicht entlang des Schnitts C-C nach Figur 25 A).

Die Platten bestehen aus einem Titan oder einer Titanlegierung, Tantal oder einer Tantallegierung können aber auch aus einem anderen elastischen biokompatiblen Material gefertigt sein. Die Platten werden mit einem CAM-Verfahren beziehungsweise einem 3D-Druckverfahren hergestellt, beispielsweise mit selektivem Elektronenstrahlschmelzen (EBM). Andere Rapid-Prototyping-Verfahren beziehungsweise computergestützte generative Herstellungsverfahren können auch zur Herstellung der Platten verwendet werden.

Die Platten weisen jeweils ein plattenförmiges Flächengebilde 71 auf, das die gesamten Platten trägt und in sich verbindet. Das Flächengebilde 71 ist flexibel und elastisch verformbar, so dass auch andere Flächen als Ebenen mit dem Flächengebilde 71 geformt werden können. Von dem Flächengebilde 71 erstrecken sich bei jeder Platte eine Vielzahl von Stiften 72 weg, die senkrecht von der Ebene der Flächengebilde 71 abstehen. In dem Flächengebilde 71 sind zwischen den Stiften 72 eine Vielzahl von durchgehenden Ausnehmungen 73 angeordnet, die, wenn die Platten miteinander zu einem Volumenkörper verbunden sind, eine offene Porosität des Volumenkörpers in einer Richtung senkrecht zu den Flächengebilden 71 bewirken können, wenn nicht die benachbarten Platten anliegen und dadurch die Ausnehmungen 73 abdecken.

In den Figuren 22 bis 25 sind zwei verschiedene Typen von Platten dargestellt nämlich erstens Grundplatten, die eine flache Unterseite aufweisen und bei denen sich die Stifte 72 nur auf einer Seite der Flächengebilde 71 von dem Flächengebilde 71 aus erstrecken, und zweitens Aufbauplatten, bei denen sich die Stifte 72 von beiden Seiten des Flächengebildes 71 aus erstrecken. Die Grundplatten sind in Figur 22 unten, in Figur 23 A) unten (in der Bildebene), in Figur 23 B) unten und in Figur 23 C) links gezeigt sowie in Figur 24 unten, in Figur 25 A) unten (in der Bildebene), in Figur 25 B) unten und in Figur 25 C) links gezeigt. Die Aufbauplatten sind in Figur 22 und 24 oben und in Figur 23 oben auf, also in Figur 23 A) oben (aus der Bildebene), in Figur 23 B) oben und in Figur 23 C) rechts beziehungsweise in Figur 25 A) oben (aus der Bildebene), in Figur 25 B) oben und in Figur 25 C) rechts gezeigt. Die Grundplatten können großflächig anliegend auf dem zu behandelnden Knochen befestigt werden. Die Aufbauplatten können allerdings ebenfalls auf dem zu behandelnden Knochen befestigt werden, wenn auch mit einer geringeren Auflagefläche, so dass auf die Grundplatten verzichtet werden kann.

An den ansonsten zylindrischen Stiften 72 sind an dem den Flächengebilden 71 gegenüberliegenden Enden der Stifte 72 Pilze 74 oder Gruppen von jeweils vier Haken 75 als Verbindungselemente 74, 75 vorgesehen. Die Pilze 74 sind nach außen (von dem Flächengebilde 71 weg) abgerundet und bilden Kugelabschnitte. Es sind aber auch andere Abrundungen, wie beispielsweise Ellipsenabschnitte möglich. Die Haken 75 sind in gleicher Weise nach außen abgerundet. An der zum Flächengebilde 71 hin ausgerichteten Seite weisen die Pilze 74 Hinterschnitte 76 auf, der zum Verhaken oder Rasten mit Haken 75 eingreifender Platten geeignet sind. Dementsprechend bilden die Haken 75 an der zum Flächengebilde 71 hin ausgerichteten Seite Hinterschnitte, die zum Verhaken oder Rasten mit anderen Pilzen 74 und Haken 75 eingreifender Platten oder mit den Ausnehmungen 73 eingreifender Platten geeignet sind.

In den Stiften 72 sind zudem angrenzend zu den Hinterschnitten 76 und angrenzend zu den Haken 75 Nuten 77 als Verbindungselemente 77 vorgesehen, in die die Pilze 74 und Haken 75 benachbarter Platten eingreifen beziehungsweise einrasten können. Hierzu sind die dem Flächengebilde 71 zugewandten Kanten der Nuten 77 abgerundet geformt, damit die Pilze 74 und Haken 75 gut in die Nuten 77 passen beziehungsweise gleiten.

Bei der vorliegenden achten Ausführungsform sind an der an den Platten Pilze 74 und Haken 75 gemischt als Verbindungselemente 74, 75 vorgesehen. Bei der Variante nach den Figuren 22 und 23 sind zwei von elf Verbindungselementen 74, 75 Haken 75 und der Rest Pilze 74. Bei der Variante nach den Figuren 24 und 25 sind acht von elf Verbindungselementen 74, 75 Haken 75 und der Rest Pilze 74. Das kann auch umgekehrt sein und die Haken 75 und Pilze 74 können auch in einem anderen Mischungsverhältnis vorliegen.

Um ein erfindungsgemäßes Knochenersatzmaterial zu bilden, liegen die Platten aneinander anliegend aber nicht miteinander verhakt oder gerastet vor (also nicht so wie in den Figur 23 und 25 gezeigt), so dass also die Pilze 74 und Haken 75 der Stifte 72 benachbarter Platten noch nicht ineinander greifen. Zudem können die Platten mit einer Flüssigkeit benetzt vorliegen. In der Flüssigkeit ist bevorzugt zumindest eine pharmazeutisch wirksame Substanz enthalten, die zur Bekämpfung der Infektion oder zum Anregen des Knochenwachstums geeignet ist. Alternativ oder zusätzlich können die Platten mit einer solchen pharmazeutisch wirksamen Substanz beschichtet sein.

Das Knochenersatzmaterial kann geformt werden, indem die Platten über ihre Flächen ineinander gedrückt werden. Dadurch verhaken oder rasten die Platten miteinander und das Knochenersatzmaterial wird in der gewünschten Form verfestigt. Zuvor können die Platten auch durch elastische Verformung der Flächengebilde 71 verformt und an die Behandlungssituation angepasst werden. Nach dem Verhaken oder Rasten mit zumindest einer weiteren (meist dann ebenfalls verformten) Platte stabilisieren sich die beiden so miteinander verbundenen Platten gegenseitig, so dass die gewählte Form verfestigt ist.

Die Platten verbinden sich dabei untereinander in der Art, dass freie Zwischenräume zwischen den miteinander verbundenen Platten im Bereich der Stifte 72, der Pilze 74, der Haken 75 und der Nuten 77 verbleiben, so dass der aus den Platten geformte Volumenköper in den Richtungen parallel zur Ebene der Platten offenporig ist. Die Platten haben einen Querschnitt beziehungsweise eine Dicke zwischen 5 mm und 10 mm, so dass die verbleibenden Poren einen freien Querschnitt im Bereich zwischen etwa 0,5 mm und 1 mm aufweisen. Dieser Querschnitt reicht aus, damit sich in den Poren Knochenmaterial ausbilden beziehungsweise einwachsen kann. Der Volumenkörper mit seinen offenen Poren kann also als osteokonduktiv bezeichnet werden. Der aus den Platten geformte Volumenkörper ist daher als Knochenersatzmaterial gut geeignet.

Die Platten sollten fest ineinander gedrückt werden, so dass der Volumenkörper formstabil ist. Die Platten können dabei in einer ersten Stufe mit einander rasten, indem die Pilze 74 und Haken 75 die Stifte 72 verbundener Platten elastisch verformen und durch die elastische Rückstellkraft der Stifte 72 die Pilze 74 und Haken 75 beziehungsweise die Ränder der Pilze 74 und Spitzen der Haken 75 in die Nuten 77 drücken und dadurch die Bewegung benachbarter Platten von dem Flächengebilde 71 weg begrenzen (siehe Figuren 23 und 25). Die Platten können durch weiteres ineinander drücken der Platten in einer zweiten Stufe mit einander rasten, indem die Pilze 74 und Haken 75 durch die Ausnehmungen 73 der Flächengebilde 71 hindurchgedrückt werden (nicht gezeigt). Dabei kann es zu einem Verklemmen der Pilze 74 und Haken 75 mit den Ausnehmungen 73 kommen, die eine Bewegung der Pilze 74 und Haken 75 gegen die benachbarte Platte verhindert und damit die beiden Platten miteinander rastet. Es ist auch möglich, dass erstens die Kanten der Pilze 74 und/oder die Spitzen der Haken 75 die Stifte 72, die Nuten 77 oder die Ausnehmungen 73 oder die Pilze 74 oder zweitens die Kanten der Ausnehmungen 73 die Pilze 74 und/oder Haken 75 benachbarter Platten in geringem Umfang plastisch verformen und dadurch eine Rastung der Platten miteinander erfolgt.

Zwei Platten können ferner miteinander verbunden werden, indem die Platten an manchen Bereichen über die Pilze 74, Haken 75 und Nuten 77 miteinander gerastet werden und in anderen Bereichen über die Pilze 74 und/oder Haken 75 mit den Ausnehmungen 73 gerastet werden. Bevorzugt werden die Abmessungen der Pilze 74, der Haken 75, die Tiefe der Ausnehmungen 73 (beziehungsweise die Dicke des Flächengebildes 71), die Form der Nuten 77 und die Länge der Stifte 72 zwischen dem Flächengebilde 71 und den Pilzen 74 oder Haken 75 so aufeinander abgestimmt, dass bei einem Verbinden der Platten die von dem Flächengebilde 71 abgewandten Oberflächen der Pilze 74 und Haken 75 an der Oberfläche der Flächengebilde 71 benachbarter Platten anliegen und/oder bei einem Verbinden der Platten die von dem Flächengebilde 71 abgewandten Oberflächen der Pilze 74 und Haken 75 in die Hinterschnitten 76 der Pilze 74 und bevorzugt entlang zumindest einer Linie an den Nuten 77 der Stifte 72 der benachbarten Platte anliegen. Hierdurch wird erreicht, dass die verbundenen Platten nicht ohne Verformung gegeneinander beweglich sind.

Die Nuten 77 verhindern auch, dass die Greifflächen 76 oder die gegenüberliegende Kappenoberseiten der Pilze 74 oder die Haken 75 die Ausnehmungen 73 komplett abdecken. Damit die Ausnehmungen 73 von den Pilzen 74 und Haken 75 leichter verformbar sind, können die Ausnehmungen 73 mehrere Schlitze aufweisen (nicht gezeigt), die über den Umfang der Ausnehmungen 73 verteilte sind. Die Breite der Schlitze sollte dazu ausreichen, dass sie osteokonduktiv wirken können.

Die achte Ausführungsform nach den Figuren 22 bis 25 unterscheidet sich also von der nach den Figuren 20 und 21 dadurch, dass die Pilze 74 Hinterschnitte 76 aufweisen, in die die Haken 75 angrenzender Platten greifen können.

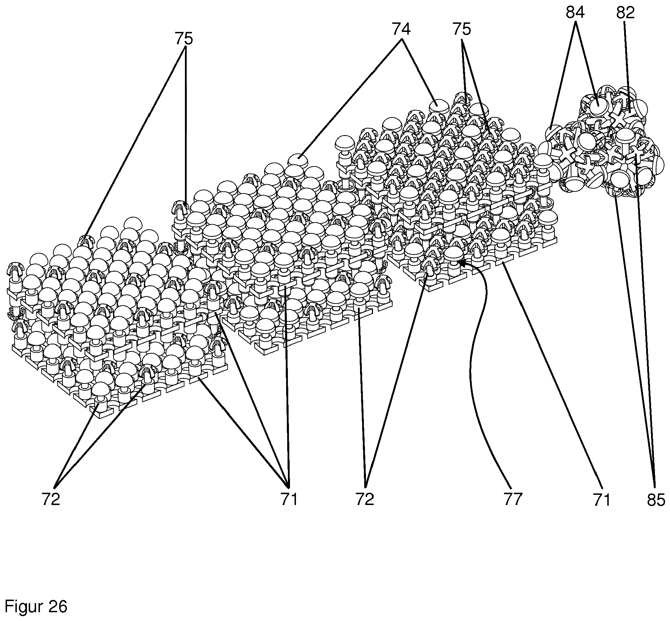

Figur 26 zeigt eine schematische perspektivische Ansicht dreier und sechs paarweise miteinander verbundener Platten des achten alternativen Knochenersatzmaterials und von drei Partikeln eines Knochenersatzmaterials, wobei die Platten mit den Partikeln verbunden werden können.

Die Partikel sind aufgebaut aus einem Kern, der in dem geometrischen Zentrum des Partikels angeordnet ist, sowie zwanzig Stiften 82, die sich in verschiedene Richtungen radial vom Kern weg erstrecken. An den Enden der Stifte 82 sind entweder Pilze 84 oder eine Gruppe von jeweils vier Haken 85 als Verbindungselemente 84, 85 an den ansonsten zylindrischen Stiften 82 angeordnet. Die Pilze 84 und Haken 85 entsprechen den Pilzen 74 und Haken 75 der Platten und sind ähnlich dimensioniert. Die Pilze 84 und die Haken 85 sind dementsprechend nach außen (vom Kern weg) kugelförmig abgerundet geformt. Es sind auch andere Abrundungen, wie beispielsweise Ellipsenabschnitte möglich. An der zum Kern hin ausgerichteten Seite haben die Pilze 84 Hinterschnitte. Ebenso weisen die Haken 85 Hinterschnitte auf. Die Hinterschnitte der Pilze 84 und die Hinterschnitte der Haken 85 sind zum Verhaken und/oder Rasten mit anderen Pilzen 84 und Haken 85 eingreifender Partikel oder zum Verhaken und/oder Rasten mit den Pilzen 74 und Haken 75 eingreifender Platten geeignet.

Bevorzugt werden die Stifte 72 und Verbindungselemente 74, 75 der Platten auf die Stifte 82 und Verbindungselemente 84, 85 der Partikel abgestimmt, damit eine gleichmäßige Stabilität erreicht werden kann. Die Materialien, aus denen die Partikel hergestellt werden, können die gleichen sein, wie die der Platten, ebenso können die gleichen Herstellungsverfahren verwendet werden.

Die Platten können über Befestigungsmittel (nicht gezeigt) in Form von Spitzen oder Schrauben mit dem Knochen eines Patienten verbunden werden. Anschließend können weitere Platten des erfindungsgemäßen Knochenersatzmaterials oder die Partikel auf den Platten befestigt werden. Die Partikel und die Platten verbinden sich dabei untereinander in der Art, dass freie Zwischenräume zwischen den miteinander verbundenen Partikeln und Platten verbleiben, so dass der aus den Partikeln und Platten geformte und verfestigte Volumenköper offenporig ist. Die freien Querschnitte der offenporigen Struktur muss noch ausreichen, damit sich in den Poren Knochenmaterial ausbilden beziehungsweise einwachsen kann. Der aus den Platten und Partikeln geformte Körper mit seinen offenen Poren kann also als osteokonduktiv bezeichnet werden. Zur Förderung der Osteokonduktivität kann auch die Oberfläche der Partikel mit einer das Knochenwachstum fördernden Substanz beschichtet sein. Der aus den Partikeln und Platten geformte Volumenkörper ist daher als Knochenersatzmaterial gut geeignet.

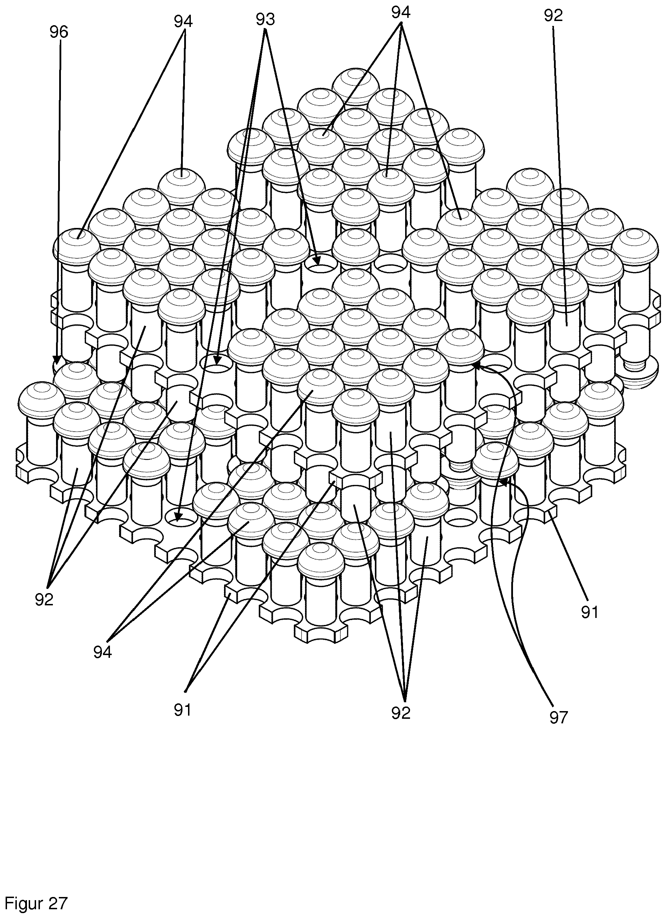

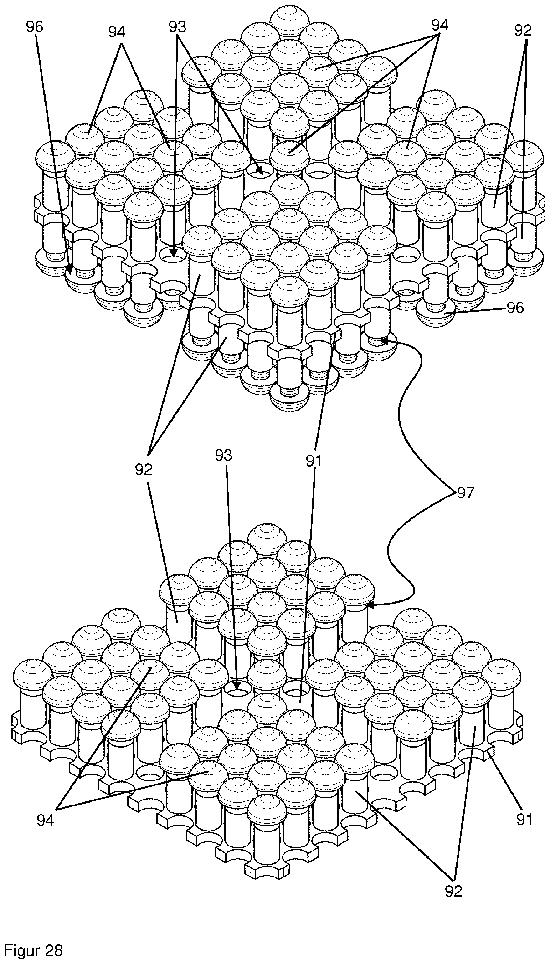

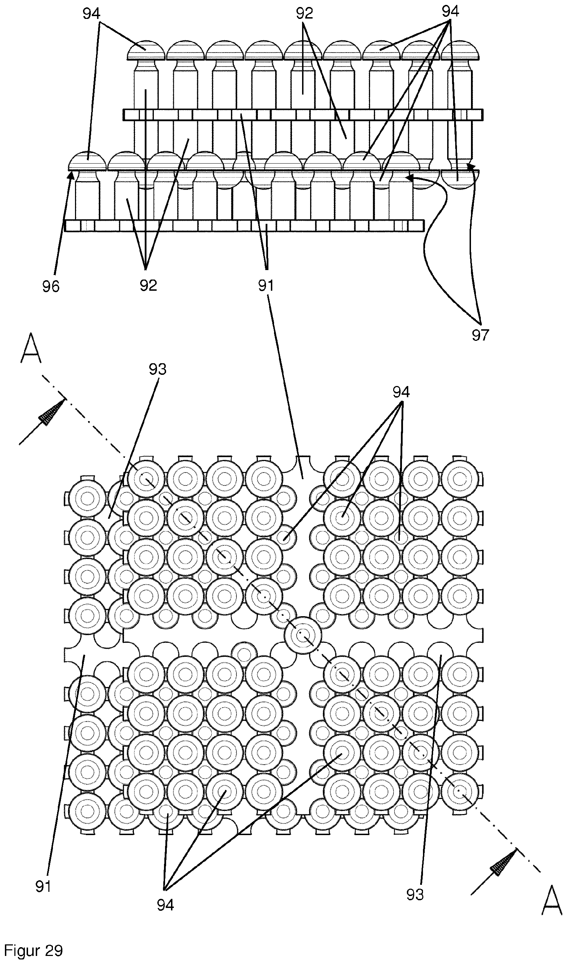

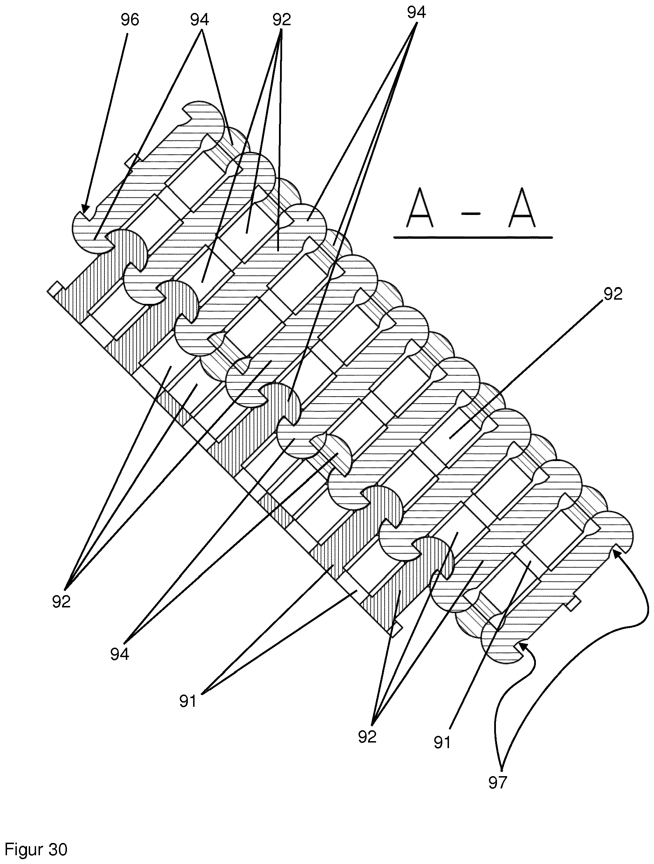

Die Figuren 27 bis 30 zeigen Platten eines neunten alternativen erfindungsgemäßen Knochenersatzmaterials, das erfindungsgemäß besonders bevorzugt ist. Dabei zeigt Figur 27 eine schematische perspektivische Ansicht auf zwei miteinander gerastete Platten, Figur 28 eine schematische perspektivische Ansicht auf die zwei nicht miteinander gerasteten Platten nach Figur 27, Figur 29 zwei schematische Ansichten der zwei miteinander gerasteten Platten nach Figur 27 in einer Aufsicht auf die Oberseite (Figur 29 unten) und in einer Seitenansicht (Figur 29 oben) und Figur 30 eine schematische Querschnittansicht des Schnitts A-A gemäß Figur 29 unten der mit einander gerasteten Platten nach den Figuren 27 und 29.

Die Platten bestehen aus einem Titan oder einer Titanlegierung, Tantal oder einer Tantallegierung können aber auch aus einem anderen elastischen biokompatiblen Material gefertigt sein. Die Platten werden mit einem CAM-Verfahren beziehungsweise einem 3D-Druckverfahren hergestellt, beispielsweise mit selektivem Elektronenstrahlschmelzen (EBM). Andere Rapid-Prototyping-Verfahren beziehungsweise computergestützte generative Herstellungsverfahren können auch zur Herstellung der Platten verwendet werden.

Die Platten weisen jeweils ein plattenförmiges Flächengebilde 91 auf, das die gesamten Platten trägt und in sich verbindet. Das Flächengebilde 91 ist flexibel und elastisch verformbar, so dass auch andere Flächen als Ebenen mit dem Flächengebilde 91 geformt werden können. Von dem Flächengebilde 91 erstrecken sich bei jeder Platte eine Vielzahl von Stiften 92 weg, die senkrecht von der Ebene der Flächengebilde 91 abstehen. In dem Flächengebilde 91 sind zwischen den Stiften 92 eine Vielzahl von durchgehenden Ausnehmungen 93 angeordnet, die, wenn die Platten miteinander zu einem Volumenkörper verbunden sind, eine offene Porosität des Volumenkörpers in einer Richtung senkrecht zu den Flächengebilden 91 bewirken können, wenn nicht die benachbarten Platten anliegen und dadurch die Ausnehmungen 93 abdecken.

In den Figuren 27 bis 30 sind zwei verschiedene Typen von Platten dargestellt nämlich erstens Grundplatten, die eine flache Unterseite aufweisen und bei denen sich die Stifte 92 nur auf einer Seite der Flächengebilde 91 von dem Flächengebilde 91 aus erstrecken, und zweitens Aufbauplatten, bei denen sich die Stifte 92 von beiden Seiten des Flächengebildes 91 aus erstrecken. Die Grundplatten sind in Figur 27 unten beziehungsweise darunter, in Figur 28 und in der Seitenansicht nach Figur 29 unten, in der Aufsicht nach Figur 29 unterhalb (in der Bildebene) und in Figur 30 links unten gezeigt. Die Aufbauplatten sind in Figur 27 oben auf, in Figur 28 und in der Seitenansicht nach Figur 29 oben, in der Aufsicht nach Figur 29 oberhalb (aus der Bildebene) und in Figur 30 rechts oben gezeigt. Die Grundplatten können großflächig anliegend auf dem zu behandelnden Knochen befestigt werden. Die Aufbauplatten können allerdings ebenfalls auf dem zu behandelnden Knochen befestigt werden, wenn auch mit einer geringeren Auflagefläche, so dass auf die Grundplatten verzichtet werden kann.

An den ansonsten zylindrischen Stiften 92 sind an dem den Flächengebilden 91 gegenüberliegenden Enden der Stifte 92 Pilze 94 als Verbindungselemente 94 vorgesehen. Die Pilze 94 sind nach außen (von dem Flächengebilde 91 weg) abgerundet und bilden Kugelabschnitte. Es sind aber auch andere Abrundungen, wie beispielsweise Ellipsenabschnitte möglich. An der zum Flächengebilde 91 hin ausgerichteten Seite bilden die Pilze 94 eine ebene Greiffläche 96, die zum Verhaken oder Rasten mit anderen Pilzen 94 eingreifender Platten oder mit den Ausnehmungen 93 eingreifender Platten geeignet sind.

In den Stiften 92 sind zudem angrenzend zu den Pilzen 94 beziehungsweise den Greifflächen 96 Nuten 97 als Verbindungselemente 97 vorgesehen, in die die Pilze 94 benachbarter Platten eingreifen beziehungsweise einrasten können. Hierzu sind die dem Flächengebilde 91 zugewandten Kanten der Nuten 97 abgerundet geformt, damit die Pilze 94 gut in die Nuten 97 passen beziehungsweise gleiten. Die Form der Nuten 97 entspricht einem Negativ der Form der Oberfläche der Pilze 94, so dass sich diese entlang einer Linie in einer der Nuten 97 anlegen können. Die Pilze 94 bilden so Rastmittel 94 und die Nuten 97 die passenden Gegenrastmittel 97. Ein weiteres Einschieben der Platte wird durch diesen Aufbau verhindert.

Die Stifte 92 mit den Pilzen 94 sind in Gruppen beziehungsweise Inseln von jeweils sechzehn Stiften 92 beziehungsweise Pilzen 94 angeordnet. Dadurch können sich die am Rand der Gruppen beziehungsweise Inseln angeordneten Stifte 92 leichter nach außen verbiegen, wenn die Pilze 94 einer anderen Platte aufgedrückt werden. Dadurch sind die Platten leichter miteinander verbindbar, da sich die elastischen Verformungen der Stifte 92 beim Einrasten der Pilze 94 in die Nuten 97 nicht gegenseitig behindern.

Bei der vorliegenden neunten Ausführungsform sind an der an den Platten ausschließlich Pilze 94 und Nuten 97 als Verbindungselemente 94, 97 vorgesehen. Alternativ oder zusätzlich könnten auch Haken (nicht gezeigt) als Verbindungselemente an den Stiften 92 vorgesehen sein.

Um ein erfindungsgemäßes Knochenersatzmaterial zu bilden, liegen die Platten aneinander anliegend aber nicht miteinander verhakt oder gerastet vor (also nicht so wie in den Figur 27, 29 und 30 gezeigt), so dass also die Pilze 94 der Stifte 92 benachbarter Platten noch nicht ineinander greifen. Zudem können die Platten mit einer Flüssigkeit benetzt vorliegen. In der Flüssigkeit ist bevorzugt zumindest eine pharmazeutisch wirksame Substanz enthalten, die zur Bekämpfung der Infektion oder zum Anregen des Knochenwachstums geeignet ist. Alternativ oder zusätzlich können die Platten mit einer solchen pharmazeutisch wirksamen Substanz beschichtet sein.

Das Knochenersatzmaterial kann geformt werden, indem die Platten über ihre Flächen ineinander gedrückt werden. Dadurch verhaken oder rasten die Platten miteinander und das Knochenersatzmaterial wird in der gewünschten Form verfestigt. Zuvor können die Platten auch durch elastische Verformung der Flächengebilde 91 verformt und an die Behandlungssituation angepasst werden. Nach dem Verhaken oder Rasten mit zumindest einer weiteren (meist dann ebenfalls verformten) Platte stabilisieren sich die beiden so miteinander verbundenen Platten gegenseitig, so dass die gewählte Form verfestigt ist.

Die Platten verbinden sich dabei untereinander in der Art, dass freie Zwischenräume zwischen den miteinander verbundenen Platten im Bereich der Stifte 92, der Pilze 94 und der Nuten 97 verbleiben, so dass der aus den Platten geformte Volumenköper in den Richtungen parallel zur Ebene der Platten offenporig ist. Die Platten haben einen Querschnitt beziehungsweise eine Dicke zwischen 5 mm und 10 mm, so dass die verbleibenden Poren einen freien Querschnitt im Bereich zwischen etwa 0,5 mm und 1 mm aufweisen. Dieser Querschnitt reicht aus, damit sich in den Poren Knochenmaterial ausbilden beziehungsweise einwachsen kann. Der Volumenkörper mit seinen offenen Poren kann also als osteokonduktiv bezeichnet werden. Der aus den Platten geformte Volumenkörper ist daher als Knochenersatzmaterial gut geeignet.

Die Platten sollten fest ineinander gedrückt werden, so dass der Volumenkörper formstabil ist. Die Platten können dabei mit einander rasten, indem die Pilze 94 die Stifte 92 verbundener Platten elastisch verformen und durch die elastische Rückstellkraft der Stifte 92 die Pilze 94 beziehungsweise die Ränder der Pilze 94 in die Nuten 97 drücken und dadurch die Bewegung benachbarter Platten von dem Flächengebilde 91 weg begrenzen (siehe Figuren 27, 29 und 30). Durch die angepasste Form der Nuten 97 an die Pilze 94 ist eine weitere Bewegung der Pilze 94 blockiert, insbesondere dann, wenn eine große Zahl von Pilzen 94 in einer großen Zahl von Nuten 97 eingerastet ist.

Bevorzugt werden die Abmessungen der Pilze 94, die Dicke des Flächengebildes 91, die Form der Nuten 97 und die Länge der Stifte 92 zwischen dem Flächengebilde 91 und den Pilzen 94 so aufeinander abgestimmt, dass bei einem Verbinden der Platten die von dem Flächengebilde 91 abgewandten Oberflächen der Pilze 94 an der Oberfläche der Nuten 97 benachbarter Platten anliegen und/oder bei einem Verbinden der Platten die Greifflächen 96 der Pilze 94 in die Greifflächen 96 der Pilze 94 der benachbarten Platte anliegen. Hierdurch wird erreicht, dass die verbundenen Platten nicht ohne große Krafteinwirkung gegeneinander beweglich sind.

Die Nuten 97 verhindern auch, dass die Greifflächen 96 oder die gegenüberliegende Kappenoberseiten der Pilze 94 die Ausnehmungen 93 komplett abdecken. Damit die Ausnehmungen 93 noch schlechter von den Pilzen 94 abgedeckt werden können, können die Ausnehmungen 93 mehrere Schlitze aufweisen (nicht gezeigt), die über den Umfang der Ausnehmungen 93 verteilte sind.

Die neunte Ausführungsform nach den Figuren 27 bis 30 unterscheidet sich also von der nach den Figuren 1 bis 7 vor allem dadurch, dass die Pilze 94 passende Nuten 97 als zusätzliche Verbindungselemente aufweisen, in die die Pilze 94 angrenzender Platten rasten können, sowie dadurch, dass die Stifte 92 dicker sind. Zudem weisen die Ausnehmungen 93 keine zusätzlichen Schlitze auf.

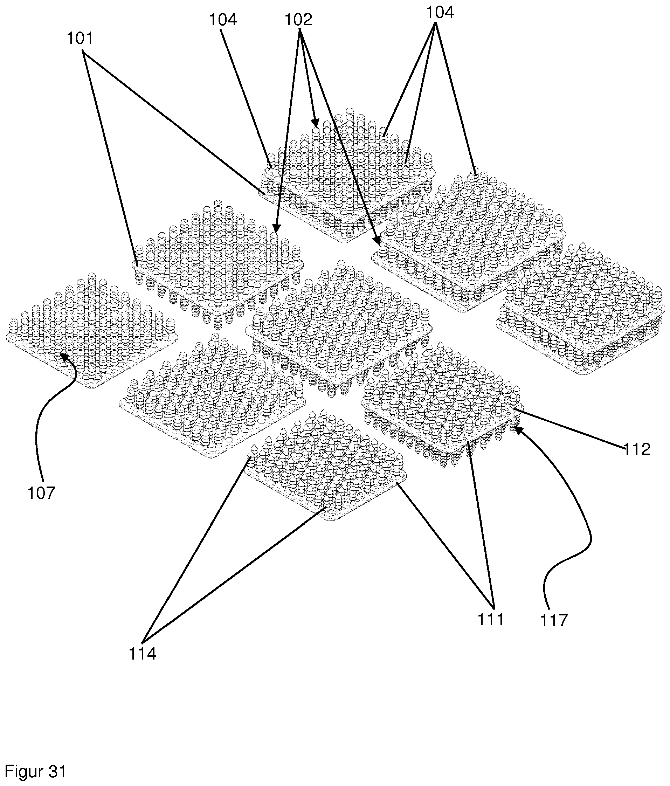

Figur 31 zeigt eine schematische perspektivische Ansicht auf mehrere Platten eines zehnten und elften erfindungsgemäßen Knochenersatzmaterials, die teilweise über ihre Verbindungselemente 104, 114 miteinander verhakt sind.

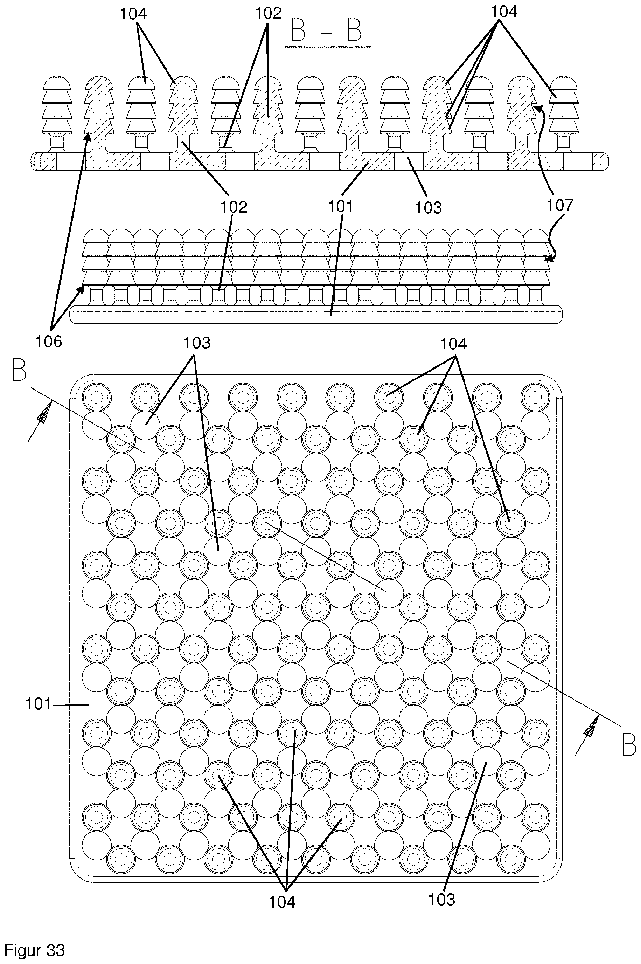

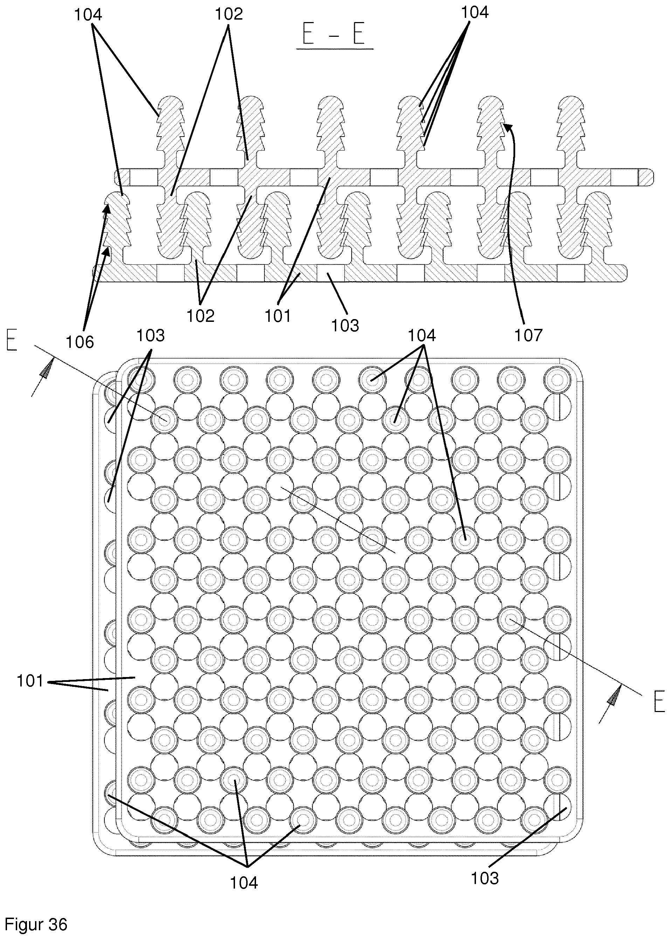

Die Figuren 31, 33 und 36 zeigen Platten eines zehnten alternativen erfindungsgemäßen Knochenersatzmaterials, das erfindungsgemäß besonders bevorzugt ist. Dabei zeigt Figur 33 drei schematische Ansichten von einer Platte des zehnten alternativen Knochenersatzmaterials, und zwar Figur 33 Unten eine Aufsicht auf die Oberseite, Figur 33 Mitte eine Seitenansicht und Figur 33 Oben einen Querschnittansicht entlang des Schnitts B-B nach Figur 33 Unten. Figur 36 zeigt zwei schematische Ansichten von zwei miteinander verbundenen Platten des zehnten alternativen Knochenersatzmaterials und zwar in Figur 36 Unten eine Aufsicht auf die Oberseite und Figur 36 Oben eine Querschnittansicht entlang des Schnitts E-E nach Figur 36 Unten.

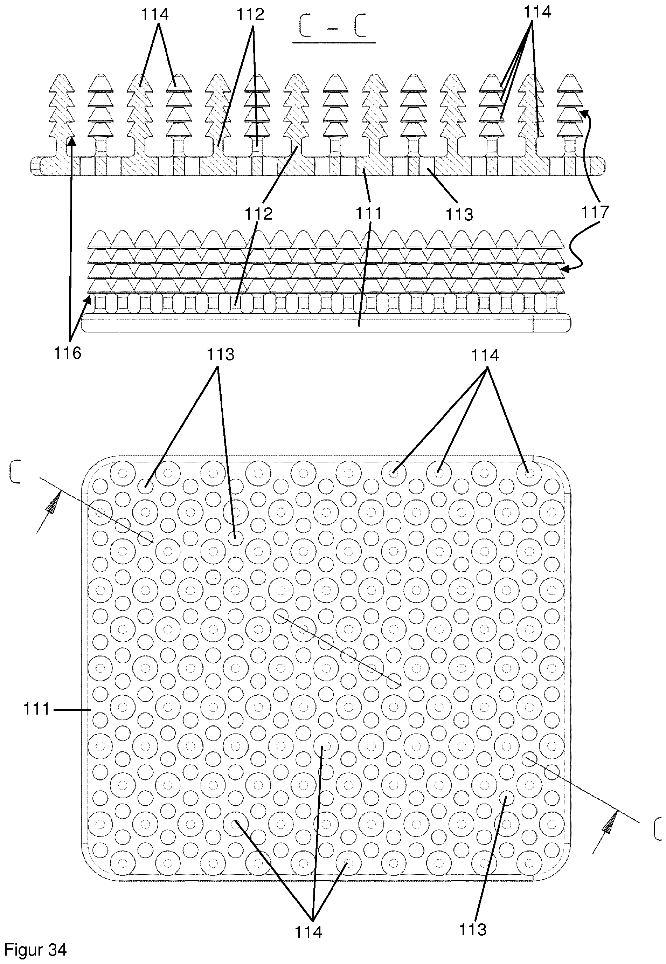

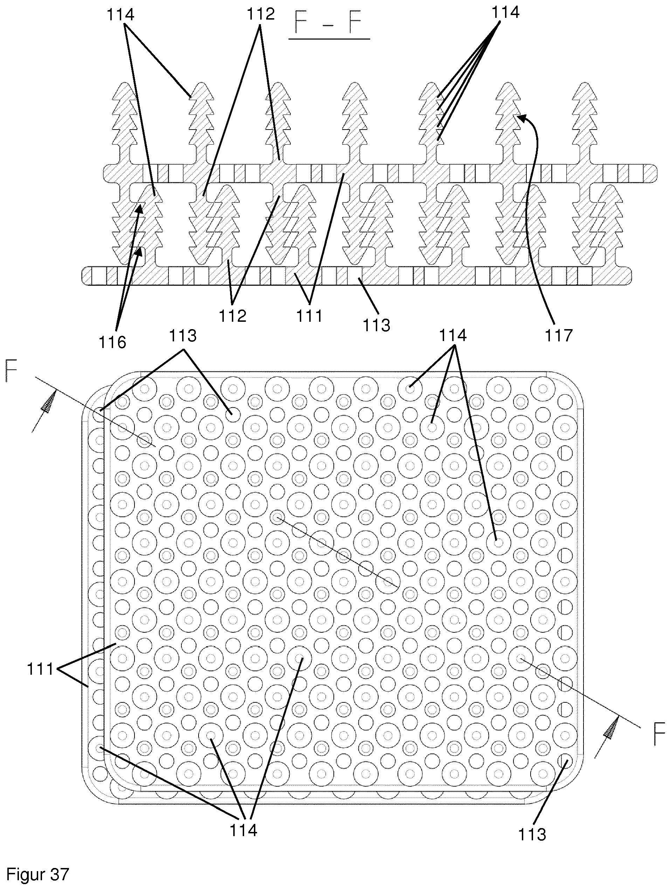

Die Figuren 31, 34 und 37 zeigen Platten eines elften alternativen erfindungsgemäßen Knochenersatzmaterials, das erfindungsgemäß besonders bevorzugt ist. Dabei zeigt Figur 34 drei schematische Ansichten von einer Platte des zehnten alternativen Knochenersatzmaterials, und zwar Figur 34 Unten eine Aufsicht auf die Oberseite, Figur 34 Mitte eine Seitenansicht und Figur 34 Oben einen Querschnittansicht entlang des Schnitts C-C nach Figur 34 Unten. Figur 37 zeigt zwei schematische Ansichten von zwei miteinander verbundenen Platten des elften alternativen Knochenersatzmaterials und zwar in Figur 37 Unten eine Aufsicht auf die Oberseite und Figur 37 Oben eine Querschnittansicht entlang des Schnitts F-F nach Figur 37 Unten.

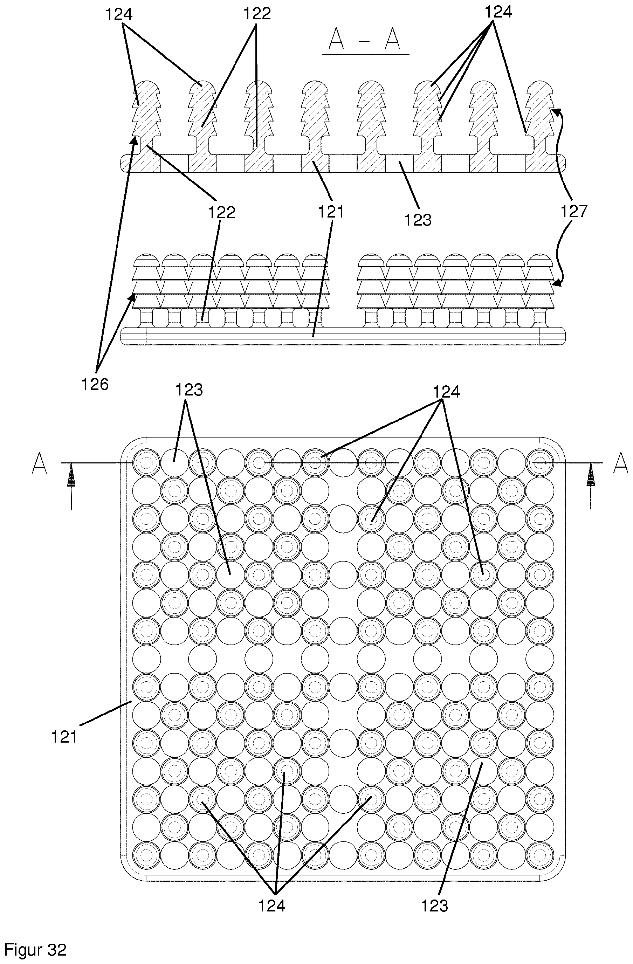

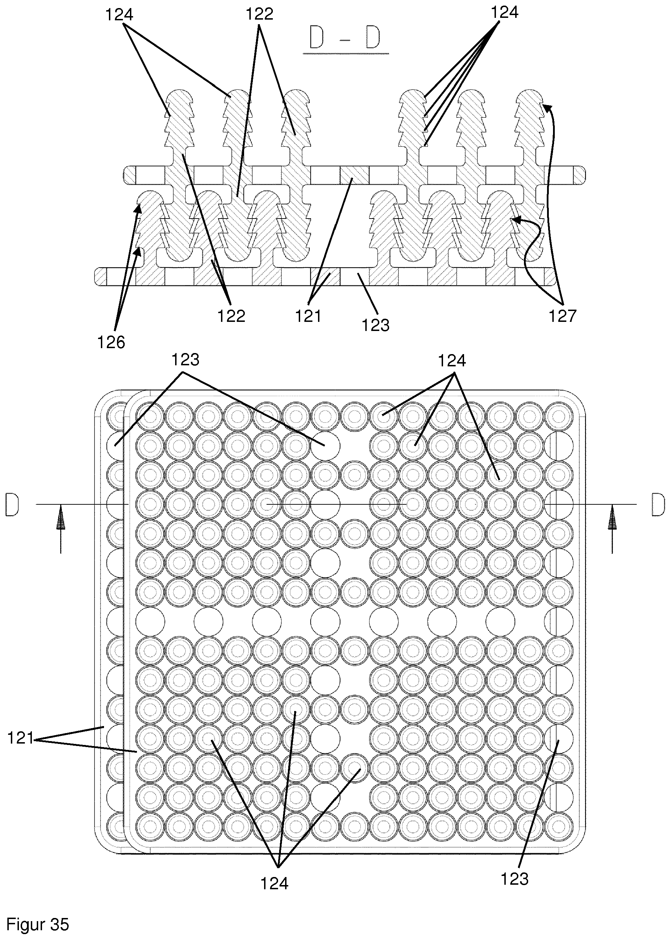

Die Figuren 32 und 35 zeigen Platten eines zwölften alternativen erfindungsgemäßen Knochenersatzmaterials, das erfindungsgemäß ebenfalls besonders bevorzugt ist. Dabei zeigt Figur 32 drei schematische Ansichten von einer Platte des zwölften alternativen Knochenersatzmaterials, und zwar Figur 32 Unten eine Aufsicht auf die Oberseite, Figur 32 Mitte eine Seitenansicht und Figur 32 Oben einen Querschnittansicht entlang des Schnitts A-A nach Figur 32 Unten. Figur 35 zeigt zwei schematische Ansichten von zwei miteinander verbundenen Platten des zehnten alternativen Knochenersatzmaterials und zwar in Figur 35 Unten eine Aufsicht auf die Oberseite und Figur 35 Oben eine Querschnittansicht entlang des Schnitts D-D nach Figur 35 Unten.

Diese drei Ausführungsformen zehn, elf und zwölf ähneln sich untereinander sehr stark und können daher im Folgenden gemeinsam beschrieben werden.

Die Platten bestehen aus einem Titan oder einer Titanlegierung, Tantal oder einer Tantallegierung können aber auch aus einem anderen elastischen biokompatiblen Material gefertigt sein. Die Platten werden mit einem CAM-Verfahren beziehungsweise einem 3D-Druckverfahren hergestellt, beispielsweise mit selektivem Elektronenstrahlschmelzen (EBM). Andere Rapid-Prototyping-Verfahren beziehungsweise computergestützte generative Herstellungsverfahren können auch zur Herstellung der Platten verwendet werden.

Die Platten weisen jeweils ein plattenförmiges Flächengebilde 101, 111, 121 auf, das die gesamten Platten trägt und in sich verbindet. Das Flächengebilde 101, 111, 121 ist flexibel und elastisch verformbar, so dass auch andere Flächen als Ebenen mit dem Flächengebilde 101, 111, 121 geformt werden können. Von dem Flächengebilde 101, 111, 121 erstrecken sich bei jeder Platte eine Vielzahl von Stiften 102, 112, 122 weg, die senkrecht von der Ebene der Flächengebilde 101, 111, 121 abstehen. In dem Flächengebilde 101, 111, 121 sind zwischen den Stiften 102, 112, 122 eine Vielzahl von durchgehenden Ausnehmungen 103, 113, 123 angeordnet, die, wenn die Platten miteinander zu einem Volumenkörper verbunden sind, eine offene Porosität des Volumenkörpers in einer Richtung senkrecht zu den Flächengebilden 101, 111, 121 bewirken können, wenn nicht die benachbarten Platten anliegen und dadurch die Ausnehmungen 103, 113, 123 abdecken.

In den Figuren 31 bis 37 sind zu der zehnten, elften und zwölften Ausführungsform jeweils zwei verschiedene Typen von Platten dargestellt nämlich erstens Grundplatten, die eine flache Unterseite aufweisen und bei denen sich die Stifte 102, 112, 122 nur auf einer Seite der Flächengebilde 101, 111, 121 von dem Flächengebilde 101, 111, 121 aus erstrecken, und zweitens Aufbauplatten, bei denen sich die Stifte 102, 112, 122 von beiden Seiten des Flächengebildes 101, 111, 121 aus erstrecken. Die Grundplatten sind in Figur 31 links unten, in den Figuren 32, 33 und 34 sowie in den Figuren 35, 36 und 37 jeweils als untere der beiden Platten dargestellt. Die Aufbauplatten sind in Figur 31 rechts oben und in den Figuren 35, 36, 37 jeweils als obere der beiden Platten gezeigt. Die Grundplatten können großflächig anliegend auf dem zu behandelnden Knochen befestigt werden. Die Aufbauplatten können allerdings ebenfalls auf dem zu behandelnden Knochen befestigt werden, wenn auch mit einer geringeren Auflagefläche, so dass auf die Grundplatten verzichtet werden kann.

An den ansonsten zylindrischen Stiften 102, 112, 122 sind an dem den Flächengebilden 101, 111, 121 gegenüberliegenden Enden der Stifte 102, 112, 122 jeweils vier übereinander angeordnete Pilze 104, 114, 124 als Verbindungselemente 104, 114, 124 vorgesehen. Die Pilze 104, 114, 124 an den Spitzen der Stifte 102, 112, 122 sind nach außen (von dem Flächengebilde 101, 111, 121 weg) abgerundet. Bei der zehnten Ausführungsform (Figuren 31, 33 und 36) und der zwölften Ausführungsform (Figuren 32 und 35) bilden die Pilze 104, 124 an den Spitzen der Stifte 102, 122 Kugelabschnitte, während bei der elften Ausführungsform die Pilze 114 an den Spitzen der Stifte 112 etwas spitzer gestaltet sind. Es sind aber auch andere Abrundungen, wie beispielsweise Ellipsenabschnitte möglich. Die Pilze 104, 114, 124 die unterhalb der Pilze 104, 114, 124 an der Spitze der Stifte 102, 112, 122 angeordnet sind, die also auf den Stiften 102, 112, 122 zwischen dem Flächengebilde 101, 111, 121 und den Pilzen 104, 114, 124 die an der von dem Flächengebilde 101, 111, 121 abgewandten Seite der Stifte 102, 112, 122 angeordnet sind, haben die Form einer Kegelstumpfs beziehungsweise sind stumpf-kegelförmig. An der zum Flächengebilde 101, 111, 121 hin ausgerichteten Seite bilden die Pilze 104, 114, 124 eine ebene Greiffläche 106, 116, 126, die zum Verhaken oder Rasten mit anderen Pilzen 104, 114, 124 eingreifender Platten oder mit den Ausnehmungen 103, 113, 123 eingreifender Platten geeignet sind. Die Stifte 112 und Pilze 114 der elften Ausführungsform (Figuren 31, 34 und 37) weisen einen etwas geringeren Durchmesser auf als die der zehnten und zwölften Ausführungsform, wobei die Greifflächen 116 der elften Ausführungsform etwas tiefer beziehungsweise großflächiger ausgestaltet sind als die Greifflächen 106, 126 der zehnten und zwölften Ausführungsform.

In den Stiften 102, 112, 122 sind zudem angrenzend zu den Pilzen 104, 114, 124 beziehungsweise den Greifflächen 106, 116, 126 Nuten 107, 117, 127 als Verbindungselemente 107, 117, 127 vorgesehen, in die die Pilze 104, 114, 124 benachbarter Platten eingreifen beziehungsweise einrasten können. Die Form der Nuten 107, 117, 127 entspricht in etwa einem Negativ der Form der Oberfläche der Pilze 104, 114, 124, so dass sich diese entlang einer Linie in einer der Nuten 107, 117, 127 anlegen können. Die Pilze 104, 114, 124 bilden so Rastmittel 104, 114, 124 und die Nuten 107, 117, 127 die passenden Gegenrastmittel 107, 117, 127. Ein weiteres Einschieben der Platten ist bei der der zehnten, elften und zwölften Ausführungsform möglich, indem die Stifte 102, 112, 122 mit den Pilzen 104, 114, 124 in die beziehungsweise durch die Ausnehmungen 103, 113, 123 gedrückt werden.

Bei der zwölften Ausführungsform sind die Stifte 122 mit den Pilzen 124 sind in Gruppen beziehungsweise Inseln von jeweils sechsundvierzig Stiften 122 angeordnet. Dadurch können sich die am Rand der Gruppen beziehungsweise Inseln angeordneten Stifte 122 leichter nach außen verbiegen, wenn die Pilze 124 einer anderen Platte aufgedrückt werden. Dadurch sind die Platten leichter miteinander verbindbar, da sich die elastischen Verformungen der Stifte 122 beim Einrasten der Pilze 124 in die Nuten 127 nicht gegenseitig behindern.

Um ein erfindungsgemäßes Knochenersatzmaterial zu bilden, liegen die Platten aneinander anliegend aber nicht miteinander verhakt oder gerastet vor, so dass also die Pilze 104, 114, 124 der Stifte 102, 112, 122 benachbarter Platten noch nicht ineinander greifen. Zudem können die Platten mit einer Flüssigkeit benetzt vorliegen. In der Flüssigkeit ist bevorzugt zumindest eine pharmazeutisch wirksame Substanz enthalten, die zur Bekämpfung der Infektion oder zum Anregen des Knochenwachstums geeignet ist. Alternativ oder zusätzlich können die Platten mit einer solchen pharmazeutisch wirksamen Substanz beschichtet sein.

Das Knochenersatzmaterial kann geformt werden, indem die Platten über ihre Flächen ineinander gedrückt werden. Dadurch verhaken oder rasten die Platten miteinander und das Knochenersatzmaterial wird in der gewünschten Form verfestigt. Zuvor können die Platten auch durch elastische Verformung der Flächengebilde 101, 111, 121 verformt und an die Behandlungssituation angepasst werden. Nach dem Verhaken oder Rasten mit zumindest einer weiteren (meist dann ebenfalls verformten) Platte stabilisieren sich die beiden so miteinander verbundenen Platten gegenseitig, so dass die gewählte Form verfestigt ist.

Die Platten verbinden sich dabei untereinander in der Art, dass freie Zwischenräume zwischen den miteinander verbundenen Platten im Bereich der Stifte 102, 112, 122, der Pilze 104, 114, 124 und der Nuten 107, 117, 127 verbleiben, so dass der aus den Platten geformte Volumenköper in den Richtungen parallel zur Ebene der Platten offenporig ist. Die Platten haben einen Querschnitt beziehungsweise eine Dicke zwischen 5 mm und 10 mm, so dass die verbleibenden Poren einen freien Querschnitt im Bereich zwischen etwa 0,5 mm und 1 mm aufweisen. Dieser Querschnitt reicht aus, damit sich in den Poren Knochenmaterial ausbilden beziehungsweise einwachsen kann. Der Volumenkörper mit seinen offenen Poren kann also als osteokonduktiv bezeichnet werden. Der aus den Platten geformte Volumenkörper ist daher als Knochenersatzmaterial gut geeignet.

Die Stifte 102, 112, 122 sind zwischen den Pilzen 104, 114, 124 und den Flächengebilden 101, 111, 121 am dünnsten, so dass die Stifte 102, 112, 122 im Bereich der Verbindung zu den Flächengebilden 101, 111, 121 an leichtesten umgebogen werden können, beziehungsweise dort am beweglichsten sind, um den Rastvorgang oder das Verhaken der Pilze 104, 114, 124 mit den Nuten 107, 117, 127 zwischen den Pilzen 104, 114, 124 zu ermöglichen.

Die Platten sollten fest ineinander gedrückt werden, so dass der Volumenkörper formstabil ist. Die Platten können dabei in einer ersten Stufe mit einander verhaken, indem die Pilze 104, 114, 124 die Stifte 102, 112, 122 verbundener Platten elastisch verformen und durch die elastische Rückstellkraft der Stifte 102, 112, 122 die Pilze 104, 114, 124 beziehungsweise die Ränder der Pilze 104, 114, 124 in die Nuten 107, 117, 127 drücken und dadurch die Bewegung benachbarter Platten von dem Flächengebilde 101, 111, 121 weg begrenzen (siehe Figuren 35 bis 37). Die Platten können durch weiteres ineinander drücken der Platten in einer zweiten Stufe mit einander rasten, indem die Pilze 104, 114, 124 durch die Ausnehmungen 103, 113, 123 der Flächengebilde 101, 111, 121 hindurchgedrückt werden (nicht gezeigt). Dabei kann es zu einem Verklemmen der Pilze 104, 114, 124 mit den Ausnehmungen 103, 113, 123 kommen, die eine Bewegung der Pilze 104, 114, 124 gegen die benachbarte Platte verhindert und damit die beiden Platten miteinander rastet. Es ist auch möglich, dass erstens die Kanten der Pilze 104, 114, 124 die Stifte 102, 112, 122, die Nuten 107, 117, 127 oder die Ausnehmungen 103, 113, 123 oder die Pilze 104, 114, 124 oder zweitens die Kanten der Ausnehmungen 103, 113, 123 die Pilze 104, 114, 124 benachbarter Platten in geringem Umfang plastisch verformen und dadurch eine Rastung der Platten miteinander erfolgt.

Eine erste Verhakung findet auch bereits dann statt, wenn die äußeren Pilze 104, 114, 124 an den Spitzen der Stifte 102, 112, 122 benachbarter Platten ineinander greifen. Zwei Platten können ferner miteinander verbunden werden, indem die Platten an manchen Bereichen über die Pilze 104, 114, 124 miteinander verhakt werden und in anderen Bereichen über die Pilze 104, 114, 124 und Ausnehmungen 103, 113, 123 miteinander gerastet werden. Bevorzugt werden die Abmessungen der Pilze 104, 114, 124, die Tiefe der Ausnehmungen 103, 113, 123 (beziehungsweise die Dicke der Flächengebilde 101, 111, 121) und die Länge der Stifte 102, 112, 122 zwischen dem Flächengebilde 101, 111, 121 und den Pilzen 104, 114, 124 und zwischen den inneren und äußeren Pilzen 104, 114, 124 so aufeinander abgestimmt, dass bei einem Verbinden der Platten die von dem Flächengebilde 101, 111, 121 abgewandten Oberflächen der Pilze 104, 114, 124 an der Oberfläche der Flächengebilde 101, 111, 121 benachbarter Platten anliegen und/oder bei einem Verbinden der Platten die von dem Flächengebilde 101, 111, 121 abgewandten Oberflächen der Pilze 104, 114, 124 an der Greiffläche 106, 116, 126 der Pilze 104, 114, 124 der benachbarten Platte anliegen. Hierdurch wird erreicht, dass die verbundenen Platten nicht ohne Verformung gegeneinander beweglich sind.

Die zehnte, elfte und zwölfte Ausführungsform nach den Figuren 31 bis 37 unterscheidet sich also von der nach den Figuren 1 bis 7 vor allem dadurch, dass durch die Vielzahl der übereinander angeordneten Pilze 104, 114, 124 diese miteinander in verschiedenen Positionen verhaken können, so dass ein daraus gefertigtes Knochenersatzmaterial besonders genau und mit geringen Abweichungen in der Dicke geformt werden kann. Zudem greifen bei abnehmenden Abstand der Platten zueinander immer mehr Pilze 104, 114, 124 in die Nuten 107, 117, 127 benachbarter Platten, so dass ein immer stabilerer Verbund entsteht.

Die in der voranstehenden Beschreibung, sowie den Ansprüchen, Figuren und Ausführungsbeispielen offenbarten Merkmale der Erfindung können sowohl einzeln, als auch in jeder beliebigen Kombination für die Verwirklichung der Erfindung in ihren verschiedenen Ausführungsformen wesentlich sein.

### Bezugszeichenliste

- 1, 11, 21, 31, 41, 51, 61, 71, 91, 101, 111, 121: Flächengebilde
- 2, 12, 22, 32, 42, 52, 62, 72, 92, 102, 112, 122: Stift
- 3, 13, 23, 33, 43, 53, 63, 73, 93, 103, 112, 123: Ausnehmung
- 4, 14, 24, 34, 44, 64, 74, 94, 104, 114, 124: Pilz / Verbindungselement
- 6, 16, 26, 36, 46, 66, 96, 106, 116, 126: Greiffläche
- 37, 47, 67, 77, 97, 107, 117, 127: Nut / Verbindungselement
- 38: Schraube / Befestigungsmittel
- 39: Spitze / Befestigungsmittel
- 45, 55, 65, 75: Haken / Verbindungselement
- 76: Hinterschnitt
- 82: Stift
- 84: Pilz / Verbindungselement
- 85: Haken / Verbindungselement

## Patentansprüche

1. Flächiges alloplastisches Knochenersatzmaterial aufweisend zumindest eine Platte, bevorzugt aufweisend mehrere Platten, zur Augmentation von Knochendefekten, wobei das Knochenersatzmaterial aus einem biokompatiblen Kunststoff, einem biokompatiblen Metall und/oder einer biokompatiblen Metalllegierung besteht, wobei die zumindest eine Platte ein Flächengebilde (1, 11, 21, 31, 41, 51, 61, 71, 91, 101, 111, 121) aufweist,
**dadurch gekennzeichnet, dass** die zumindest eine Platte eine Mehrzahl von sich aus dem Flächengebilde (1, 11, 21, 31, 41, 51, 61, 71, 91, 101, 111, 121) der zumindest einen Platte heraus erstreckende Stifte (2, 12, 22, 32, 42, 52, 62, 72, 92, 102, 112, 122) aufweist, wobei die Stifte (2, 12, 22, 32, 42, 52, 62, 72, 92, 102, 112, 122) jeweils mindestens ein Verbindungselement (4, 14, 24, 34, 37, 44, 45, 47, 55, 64, 65, 67, 74, 75, 76, 77, 94, 97, 104, 107, 114, 117, 124, 127) aufweisen, wobei die Stifte (2, 12, 22, 32, 42, 52, 62, 72, 92, 102, 112, 122) elastisch verformbar sind und derart dicht zueinander angeordnet sind, so dass durch Aufeinanderpressen der Flächen mehrerer Platten die gleichen Verbindungselemente (4, 14, 24, 34, 37, 44, 45, 47, 55, 64, 65, 67, 74, 75, 76, 77, 94, 97, 104, 107, 114, 117, 124, 127) verschiedener Platten miteinander verhaken und/oder rasten und die miteinander verhakten und/oder gerasteten Platten einen offenporigen Körper aus miteinander verhakten und/oder gerasteten Platten bilden.

2. Knochenersatzmaterial nach Anspruch 1, **dadurch gekennzeichnet, dass**
die Verbindungselemente (4, 14, 24, 34, 37, 44, 45, 47, 55, 64, 65, 67, 74, 75, 76, 77, 94, 97,104, 107, 114, 117, 124, 127) Pilze (4, 14, 24, 34, 44, 64, 74, 94, 104, 114, 124), Haken (45, 55, 65, 75), Hinterschnitte (76) und/oder Rastelemente (34, 37, 44, 45, 47, 64, 65, 67, 74, 75, 77, 94, 97, 104, 107, 114, 117, 124, 127) sind, vorzugsweise Pilze (4, 14, 24, 34, 44, 64, 74, 94, 104, 114, 124), Haken (45, 55, 65, 75), Hinterschnitte (76), Rastungen (34, 44, 45, 64, 65, 74, 75, 94, 104, 114, 124) und/oder Gegenrastungen (37, 47, 67, 77, 97, 107, 117, 127) sind.

3. Knochenersatzmaterial nach einem der vorangehenden Ansprüche, **dadurch gekennzeichnet, dass**
die Verbindungselemente (4, 14, 24, 34, 37, 44, 45, 47, 55, 64, 65, 67, 74, 75, 76, 77, 94, 97, 104, 107, 114, 117, 124, 127) an der Mantelfläche der Stifte (2, 12, 22, 32, 42, 52, 62, 72, 92, 102, 112, 122) ausgebildet sind.

4. Knochenersatzmaterial nach einem der vorangehenden Ansprüche, **dadurch gekennzeichnet, dass**
zumindest eines der zumindest einen Verbindungselemente (4, 14, 24, 34, 37, 44, 45, 47, 55, 64, 65, 67, 74, 75, 76, 77, 94, 97, 104, 107, 114, 117, 124, 127) pro Stift stumpf-kegelförmig ausgebildet ist, wobei die Längsachsen der Stifte (2, 12, 22, 32, 42, 52, 62, 72, 92, 102, 112, 122) die Längsachsen der Kegel bilden und wobei der Mantel der Kegel nach der zu dem Flächengebilde (1, 11, 21, 31, 41, 51, 61, 71, 91, 101, 111, 121) der zumindest einen Platte abgewandten Außenseite gerichtet ist.

5. Knochenersatzmaterial nach einem der vorangehenden Ansprüche, **dadurch gekennzeichnet, dass**
die Stifte (2, 12, 22, 32, 42, 52, 62, 72, 92, 102, 112, 122) zwischen dem Flächengebilde (1, 11, 21, 31, 41, 51, 61, 71, 91, 101, 111, 121) einer Platte und zumindest einem der zumindest einen Verbindungselemente (4, 14, 24, 34, 44, 45, 55, 64, 65, 74, 75, 94, 97, 104, 107, 114, 117, 124, 127)eine umlaufende Nut (37, 47, 67, 77, 97, 107, 117, 127) als zusätzliches Verbindungselement (37, 47, 67, 77, 97, 107, 117, 127) enthalten, in welche Verbindungselemente (4, 14, 24, 34, 44, 45, 55, 64, 65, 74, 75, 94, 104, 114, 124) anderer Platten einhaken oder einrasten können, bevorzugt derart einrasten, dass keine weitere Bewegung der Verbindungselemente (4, 14, 24, 34, 37, 44, 45, 47, 55, 64, 65, 67, 74, 75, 76, 77, 94, 97, 104, 107, 114, 117, 124, 127) entlang der Stifte (2, 12, 22, 32, 42, 52, 62, 72, 92, 102, 112, 122) möglich ist.

6. Knochenersatzmaterial nach einem der vorangehenden Ansprüche, **dadurch gekennzeichnet, dass**
zumindest zwei Verbindungselemente (4, 14, 24, 34, 37, 44, 45, 47, 55, 64, 65, 67, 74, 75, 76, 77, 94, 97, 104, 107, 114, 117, 124, 127) hintereinander auf der Mantelfläche der Stifte (2, 12, 22, 32, 42, 52, 62, 72, 92, 102, 112, 122) angeordnet sind, besonders bevorzugt zumindest drei Verbindungselemente (4, 14, 24, 34, 37, 44, 45, 47, 55, 64, 65, 67, 74, 75, 76, 77, 94, 97, 104, 107, 114, 117, 124, 127) hintereinander auf der Mantelfläche der Stifte (2, 12, 22, 32, 42, 52, 62, 72, 92, 102, 112, 122) angeordnet sind.

7. Knochenersatzmaterial nach einem der vorangehenden Ansprüche, **dadurch gekennzeichnet, dass**
in dem Flächengebilde (1, 11, 21, 31, 41, 51, 61, 71, 91, 101, 111, 121) der zumindest einen Platte durchgehende Poren (3, 13, 23, 33, 43, 53, 63, 73, 93, 103, 113, 123) enthalten sind, wobei die Tiefe der Poren (3, 13, 23, 33, 43, 53, 63, 73, 93, 103, 113, 123) senkrecht zu dem Flächengebilde (1, 11, 21, 31, 41, 51, 61, 71, 91, 101, 111, 121) der zumindest einen Platte zumindest 0,25 mm beträgt, bevorzugt zumindest 0,4 mm beträgt.

8. Knochenersatzmaterial nach einem der vorangehenden Ansprüche, **dadurch gekennzeichnet, dass**
benachbarte Stifte (2, 12, 22, 32, 42, 52, 62, 72, 92, 102, 112, 122), die auf der gleichen Seite einer ersten Platte der zumindest einen Platte angeordnet sind, einen derartigen Abstand zueinander aufweisen, dass nach einer elastischer Deformation aufgrund einer Verhakung und/oder Rastung mit einem Verbindungselement (4, 14, 24, 34, 37, 44, 45, 47, 55, 64, 65, 67, 74, 75, 76, 77, 94, 97, 104, 107, 114, 117, 124, 127) einer zweiten Platte der zumindest einen Platte die Stifte (2, 12, 22, 32, 42, 52, 62, 72, 92, 102, 112, 122) der erste Platte mindestens zwei Verhakungen und/oder Rastungen mit zumindest zwei weiteren Verbindungselementen (4, 14, 24, 34, 37, 44, 45, 47, 55, 64, 65, 67, 74, 75, 76, 77, 94, 97, 104, 107, 114, 117, 124, 127) der zweiten Platte ermöglichen, bevorzugt mindestens drei Verhakungen und/oder Rastungen mit drei weiteren Verbindungselementen (4, 14, 24, 34, 37, 44, 45, 47, 55, 64, 65, 67, 74, 75, 76, 77, 94, 97, 104, 107, 114, 117, 124, 127) der zweiten Platte ermöglichen.

9. Knochenersatzmaterial nach einem der vorangehenden Ansprüche, **dadurch gekennzeichnet, dass**
die Platte oder wenigstens eine der Platten nur auf einer Seite Stifte (2, 12, 22, 32, 42, 52, 62, 72, 92, 102, 112, 122) aufweist und auf der anderen Seite flächig an einem Knochen zu befestigen ist, wobei vorzugsweise hierzu scharfe Spitzen (39) vorgesehen sind, die in den Knochen eingedrückt werden können, oder in dem Flächengebilde (1, 11, 21, 31, 41, 51, 61, 71, 91, 101, 111, 121) der zumindest einen Platte Ösen oder Bohrungen vorgesehen sind, durch die die zumindest eine Platte auf einen Knochen geschraubt oder anders befestigt werden kann.

10. Knochenersatzmaterial nach einem der vorangehenden Ansprüche, **dadurch gekennzeichnet, dass**
die zumindest eine Platte oder wenigstens eine der zumindest einen Platten auf beiden Seite Stifte (2, 12, 22, 32, 42, 52, 62, 72, 92, 102, 112, 122) aufweist.

11. Knochenersatzmaterial nach einem der vorangehenden Ansprüche, **dadurch gekennzeichnet, dass**
die Poren (3, 13, 23, 33, 43, 53, 63, 73, 93, 103, 113, 123) des aus mehreren Platten gebildeten offenporigen Körpers interkonnektierend und osteokonduktiv sind, wobei vorzugsweise die Poren (3, 13, 23, 33, 43, 53, 63, 73, 93,103,113, 123) einen freien Querschnitt zwischen 0,1 mm und 1 mm aufweisen, besonders bevorzugt zwischen 0,25 mm und 0,9 mm.

12. Knochenersatzmaterial nach einem der vorangehenden Ansprüche, **dadurch gekennzeichnet, dass**
die Stifte (2, 12, 22, 32, 42, 52, 62, 72, 92, 102, 112, 122) mit Verbindungselementen (4, 14, 24, 34, 37, 44, 45, 47, 55, 64, 65, 67, 74, 75, 76, 77, 94, 97, 104, 107, 114, 117, 124, 127) in Reihen von jeweils drei oder mehreren Stiften (2, 12, 22, 32, 42, 52, 62, 72, 92, 102, 112, 122) angeordnet sind und dass zwischen diesen jeweils drei oder mehrere Reihen ein Streifen von unbesetzter Oberfläche der zumindest einen Platte verbleibt oder dass eine gruppierte oder nestförmige Anordnung von Stiften (2, 12, 22, 32, 42, 52, 62, 72, 92, 102, 112, 122) mit Verbindungselementen (4, 14, 24, 34, 37, 44, 45, 47, 55, 64, 65, 67, 74, 75, 76, 77, 94, 97, 104, 107, 114, 117, 124, 127) vorgesehen ist.

13. Knochenersatzmaterial nach einem der vorangehenden Ansprüche, **dadurch gekennzeichnet, dass**
das Knochenersatzmaterial neben der zumindest einen Platte zusätzlich zumindest einen Partikel aufweist, wobei der zumindest eine Partikel einen Kern und mindestens sechs sich vom Kern erstreckende Stifte (82) aufweist, wobei die Stifte (82) jeweils mindestens ein Verbindungselement (84, 85) aufweisen, das analog der Verbindungselemente (4, 14, 24, 34, 37, 44, 45, 47, 55, 64, 65, 67, 74, 75, 76, 77, 94, 97, 104, 107, 114, 117, 124, 127) der zumindest einen Platten ausgebildet ist, so dass die zumindest eine Platte mit dem zumindest einen Partikel durch Aufeinanderpressen die Verbindungselemente (4, 14, 24, 34, 37, 44, 45, 47, 55, 64, 65, 67, 74, 75, 76, 77, 84, 85, 94, 97, 104, 107, 114, 117, 124, 127) der zumindest einen Platte und des zumindest einen Partikels miteinander verhaken und/oder rasten und die miteinander verhakten und/oder gerasteten Platte(n) und Partikel einen offenporigen Körper aus miteinander verhakten und/oder gerasteten Platte(n) und Partikel(n) bilden.

14. Verfahren zum Formen eines Körpers aus einem flächigen alloplastischen Knochenersatzmaterial mit mehreren Platten nach einem der vorangehenden Ansprüche, bei dem mehrere Platten gegeneinander gedrückt werden, wobei die Platten miteinander verhaken und/oder rasten und einen offenporigen Körper ausbilden.

15. Verfahren nach Anspruch 14, **dadurch gekennzeichnet, dass**
zumindest eine der Platten mit einem porösen Volumenkörper eines zweiten Knochenersatzmaterials verbunden werden, indem die Verbindungselemente (4, 14, 24, 34, 37, 44, 45, 47, 55, 64, 65, 67, 74, 75, 76, 77, 94, 97, 104, 107, 114, 117, 124, 127) mit den Poren des zweiten Knochenersatzmaterials rasten und/oder verhaken, und/oder zumindest eine der Platten mit einem partikulären dritten Knochenersatzmaterial aufweisend mehrere Partikel verbunden werden, wobei die Partikel des dritten Knochenersatzmaterials eine Mehrzahl von sich von einem Kern der Partikel erstreckenden Stiften (82) mit Verbindungelementen (84, 85) aufweist, wobei bevorzugt die Stifte (82) und die Verbindungselemente (84, 85) der Partikel des dritten Knochenersatzmaterials die Merkmale der Stifte (2, 12, 22, 32, 42, 52, 62, 72, 92, 102, 112, 122) und Verbindungselemente (4, 14, 24, 34, 37, 44, 45, 47, 55, 64, 65, 67, 74, 75, 76, 77, 94, 97, 104, 107, 114, 117, 124, 127) der zumindest einen Platte des Knochenersatzmaterials nach einem der Ansprüche 1 bis 13 aufweisen.

## Claims

1. Planar alloplastic bone replacement material comprising at least one plate, preferably comprising multiple plates, for augmentation of bone defects, whereby the bone replacement material consists of a biocompatible plastic material, a biocompatible metal and/or a biocompatible metal alloy, whereby the at least one plate comprises a planar structure (1, 11, 21, 31, 41, 51, 61, 71, 91, 101, 111, 121) **characterized in that**
the at least one plate comprises a plurality of pins (2, 12, 22, 32, 42, 52, 62, 72, 92, 102, 112, 122) extending outwards from the planar structure (1, 11, 21, 31, 41, 51, 61, 71, 91, 101, 111, 121) of the at least one plate, whereby the pins (2, 12, 22, 32, 42, 52, 62, 72, 92, 102, 112, 122) each comprise at least one connecting element (4, 14, 24, 34, 37, 44, 45, 47, 55, 64, 65, 67, 74, 75, 76, 77, 94, 97, 104, 107, 114, 117, 124, 127), whereby the pins (2, 12, 22, 32, 42, 52, 62, 72, 92, 102, 112, 122) are deformable elastically and are arranged sufficiently close to each other such that pressing the surfaces of multiple plates onto each other causes the same connecting elements (4, 14, 24, 34, 37, 44, 45, 47, 55, 64, 65, 67, 74, 75, 76, 77, 94, 97, 104, 107, 114, 117, 124, 127) of different plates to interlock with and/or snap into each other and the interlocked and/or snapped-in plates form an open-pored body of mutually interlocked and/or snapped-in plates.

2. Bone replacement material according to claim 1, **characterised in that**
the connecting elements (4, 14, 24, 34, 37, 44, 45, 47, 55, 64, 65, 67, 74, 75, 76, 77, 94, 97, 104, 107, 114, 117, 124, 127) are mushrooms (4, 14, 24, 34, 44, 64, 74, 94, 104, 114, 124), hooks (45, 55, 65, 75), undercuts (76) and/or snap-in elements (34, 37, 44, 45, 47, 64, 65, 67, 74, 75, 77, 94, 97, 104, 107, 114, 117, 124, 127), preferably mushrooms (4, 14, 24, 34, 44, 64, 74, 94, 104, 114, 124), hooks (45, 55, 65, 75), undercuts (76), snap-in means (34, 44, 45, 64, 65, 74, 75, 94, 104, 114, 124) and/or opposite snap-in means (37, 47, 67, 77, 97, 107, 117, 127).

3. Bone replacement material according to any one of the preceding claims, **characterised in that**
the connecting elements (4, 14, 24, 34, 37, 44, 45, 47, 55, 64, 65, 67, 74, 75, 76, 77, 94, 97, 104, 107, 114, 117, 124, 127) are provided on the jacket surface of the pins (2, 12, 22, 32, 42, 52, 62, 72, 92, 102, 112, 122).

4. Bone replacement material according to any one of the preceding claims, **characterised in that**
at least one of the at least one connecting elements (4, 14, 24, 34, 37, 44, 45, 47, 55, 64, 65, 67, 74, 75, 76, 77, 94, 97, 104, 107, 114, 117, 124, 127) per pin has a truncated cone shape, whereby the longitudinal axes of the pins (2, 12, 22, 32, 42, 52, 62, 72, 92, 102, 112, 122) form the longitudinal axes of the cones and whereby the jacket of the cones faces toward the outer side that faces away from the planar structure (1, 11, 21, 31, 41, 51, 61, 71, 91, 101, 111, 121) of the at least one plate.

5. Bone replacement material according to any one of the preceding claims, **characterised in that**
the pins (2, 12, 22, 32, 42, 52, 62, 72, 92, 102, 112, 122) contain a circumferential groove (37, 47, 67, 77, 97, 107, 117, 127) as additional connecting element (37, 47, 67, 77, 97, 107, 117, 127) between the planar structure (1, 11, 21, 31, 41, 51, 61, 71, 91, 101, 111, 121) of one plate and at least one of the at least one connecting elements (4, 14, 24, 34, 44, 45, 55, 64, 65, 74, 75, 94, 97,104, 107,114, 117,124, 127), whereby connecting elements (4, 14, 24, 34, 44, 45, 55, 64, 65, 74, 75, 94) of other plates can interlock with or snap into said groove, preferably snap-in appropriately such that no further motion of the connecting elements (4, 14, 24, 34, 37, 44, 45, 47, 55, 64, 65, 67, 74, 75, 76, 77, 94, 97, 104, 107, 114, 117, 124, 127) along the pins (2, 12, 22, 32, 42, 52, 62, 72, 92, 102, 112, 122) is possible.

6. Bone replacement material according to any one of the preceding claims, **characterised in that**
at least two connecting elements (4, 14, 24, 34, 37, 44, 45, 47, 55, 64, 65, 67, 74, 75, 76, 77, 94, 97, 104, 107, 114, 117, 124, 127) are arranged in succession on the jacket surface of the pins (2, 12, 22, 32, 42, 52, 62, 72, 92, 102, 112, 122), particularly preferably at least three connecting elements (4, 14, 24, 34, 37, 44, 45, 47, 55, 64, 65, 67, 74, 75, 76, 77, 94, 97, 104, 107, 114, 117, 124, 127) are arranged in succession on the jacket surface of the pins (2, 12, 22, 32, 42, 52, 62, 72, 92, 102, 112, 122).

7. Bone replacement material according to any one of the preceding claims, **characterised in that**
the planar structure (1, 11, 21, 31, 41, 51, 61, 71, 91, 101, 111, 121) of the at least one plate contains through-going pores (3, 13, 23, 33, 43, 53, 63, 73, 93, 103, 113, 123), whereby the depth of the pores (3, 13, 23, 33, 43, 53, 63, 73, 93, 103, 113, 123) perpendicular to the planar structure (1, 11, 21, 31, 41, 51, 61, 71, 91, 101, 111, 121) of the at least one plate is at least 0.25 mm, preferably at least 0.4 mm.

8. Bone replacement material according to any one of the preceding claims, **characterised in that**
neighbouring pins (2, 12, 22, 32, 42, 52, 62, 72, 92, 102, 112, 122) that are arranged on the same side of a first plate of the at least one plate are situated at an appropriate distance from each other such that the pins (2, 12, 22, 32, 42, 52, 62, 72, 92, 102, 112, 122) of the first plate, after elastic deformation due to interlocking and/or snapping into a connecting element (4, 14, 24, 34, 37, 44, 45, 47, 55, 64, 65, 67, 74, 75, 76, 77, 94, 97, 104, 107, 114, 117, 124, 127) of a second plate of the at least one plate, enable at least two interlocks and/or snap-in connections to at least two further connecting elements (4, 14, 24, 34, 37, 44, 45, 47, 55, 64, 65, 67, 74, 75, 76, 77, 94, 97, 104, 107, 114, 117, 124, 127) of the second plate, preferably enable at least three interlocks and/or snap-in connections to three further connecting elements (4, 14, 24, 34, 37, 44, 45, 47, 55, 64, 65, 67, 74, 75, 76, 77, 94, 97, 104, 107, 114, 117, 124, 127) of the second plate.

9. Bone replacement material according to any one of the preceding claims, **characterised in that**
the plate or at least one of the plates comprises pins (2, 12, 22, 32, 42, 52, 62, 72, 92, 102, 112, 122) just on one side and is attachable in planar manner to a bone on the other side, whereby, preferably, sharp tips (39) are provided for this purpose that can be pushed into the bone or eyelets or boreholes are provided in the planar structure (1, 11, 21, 31, 41, 51, 61, 71, 91, 101, 111, 121) of the at least one plate by means of which the at least one plate can be screwed to a bone or attached by other means.

10. Bone replacement material according to any one of the preceding claims, **characterised in that**
the at least one plate or at least one of the at least one plates comprises pins (2, 12, 22, 32, 42, 52, 62, 72, 92, 102, 112, 122) on both sides.

11. Bone replacement material according to any one of the preceding claims, **characterised in that**
the pores (3, 13, 23, 33, 43, 53, 63, 73, 93, 103, 113, 123) of the open-pored body formed from multiple plates to be interconnecting and osteoconductive, whereby the pores (3, 13, 23, 33, 43, 53, 63, 73, 93, 103, 113, 123) preferably have a free cross-section between 0.1 mm and 1 mm, particularly preferably between 0.25 mm and 0.9 mm.

12. Bone replacement material according to any one of the preceding claims,
the pins (2, 12, 22, 32, 42, 52, 62, 72, 92, 102, 112, 122) having connecting elements (4, 14, 24, 34, 37, 44, 45, 47, 55, 64, 65, 67, 74, 75, 76, 77, 94, 97, 104, 107, 114, 117, 124, 127) are arranged in rows of three or more pins (2, 12, 22, 32, 42, 52, 62, 72, 92, 102, 112, 122) each and in that a strip of unoccupied surface of the at least one plate remains between these three or more rows each or in that a grouped or nested arrangement of pins (2, 12, 22, 32, 42, 52, 62, 72, 92, 102, 112, 122) having connecting elements (4, 14, 24, 34, 37, 44, 45, 47, 55, 64, 65, 67, 74, 75, 76, 77, 94, 97, 104, 107, 114, 117, 124, 127) is provided.

13. Bone replacement material according to any one of the preceding claims, **characterised in that**
the bone replacement material comprises at least one particle aside from the at least one plate, whereby the at least one particle comprises a core and at least six pins (82) extending from the core, whereby the pins (82) each comprise at least one connecting element (84, 85) that is designed in analogous manner to the connecting elements (4, 14, 24, 34, 37, 44, 45, 47, 55, 64, 65, 67, 74, 75, 76, 77, 94, 97, 104, 107, 114, 117, 124, 127) of the at least one plates such that the at least one plate and the at least one particle interlock with and/or snap into each other by pressing the connecting elements (4, 14, 24, 34, 37, 44, 45, 47, 55, 64, 65, 67, 74, 75, 76, 77, 94, 97, 104, 107, 114, 117, 124, 127) of the at least one plate and of the at least one particle onto each other, and whereby the mutually interlocked and/or snapped-in plate(s) and particle(s) form an open-pored body of mutually interlocked and/or snapped-in plate(s) and particle(s).

14. Method for forming a body made of a planar alloplastic bone replacement material comprising multiple plates according to any one of the preceding claims, in which multiple plates are pushed against each other, whereby the plates interlock with and/or snap into each other and form an open-pored body.

15. Method according to claim 14, **characterised in that**
at least one of the plates is being connected to a porous three-dimensional body of a second bone replacement material by snapping-in and/or interlocking the connecting elements (4, 14, 24, 34, 37, 44, 45, 47, 55, 64, 65, 67, 74, 75, 76, 77, 94, 97, 104, 107, 114, 117, 124, 127) with the pores of the second bone replacement material, and/or at least one of the plates are being connected to a particulate third bone replacement material comprising multiple particles, whereby the particles of the third bone replacement material comprise a plurality of pins (82) that extend from a core of the particles and have connecting elements (84, 85), whereby, preferably, the pins (82) and the connecting elements (84, 85) of the particles of the third bone replacement material comprise the features of the pins 2, 12, 22, 32, 42, 52, 62, 72, 92, 102, 112, 122) and connecting elements (4, 14, 24, 34, 37, 44, 45, 47, 55, 64, 65, 67, 74, 75, 76, 77, 94, 97, 104, 107, 114, 117, 124, 127) of the at least one plate of the bone replacement material according to any one of the claims 1 to 13.

## Revendications

1. Matériau de substitution d'os alloplastique plan présentant au moins une plaque, présentant de préférence plusieurs plaques, pour l'augmentation de défauts osseux, le matériau de substitution d'os étant constitué d'une matière plastique biocompatible, d'un métal biocompatible et/ou d'un alliage métallique biocompatible, où au moins une plaque présente une structure plane (1, 11, 21, 31, 41, 51, 61, 71, 91, 101, 111, 121),
**caractérisé en ce que** l'au moins une plaque présente une multiplicité de broches (2, 12, 22, 32, 42, 52, 62, 72, 92, 102, 112, 122) s'étendant hors de la structure plane (1, 11, 21, 31, 41, 51, 61, 71, 91, 101, 111, 121) de l'au moins une plaque, où les broches (2, 12, 22, 32, 42, 52, 62, 72, 92, 102, 112, 122) présentent respectivement au moins un élément de liaison (4, 14, 24, 34, 37, 44,45, 47, 55, 64,65, 67, 74,75, 76, 77, 94,97, 104, 107, 114, 117, 124, 127), où les broches (2, 12, 22, 32, 42, 52, 62, 72, 92, 102, 112, 122) sont déformables de manière élastique et sont disposées de manière si dense les unes par rapport aux autres que par la pression des surfaces les unes sur les autres de plusieurs plaques, les mêmes éléments de liaison (4, 14, 24, 34, 37, 44, 45, 47, 55, 64, 65, 67, 74, 75, 76, 77, 94, 97, 104, 107, 114, 117, 124, 127) de différentes plaques s'ancrent les uns dans les autres et/ou se verrouillent et les plaques ancrées les unes dans les autres et/ou verrouillées forment un corps à pores ouvertes à base de plaques ancrées les unes dans les autres et/ou verrouillées.

2. Matériau de substitution d'os selon la revendication 1, **caractérisé en ce que** les éléments de liaison (4, 14, 24, 34, 37, 44, 45, 47, 55, 64, 65, 67, 74, 75, 76, 77, 94, 97, 104, 107, 114, 117, 124, 127) sont des champignons (4, 14, 24, 34, 44, 64, 74, 94, 104, 114, 124), des crochets (45, 55, 65, 75), des renfoncements (76) et/ou des éléments de verrouillage (34, 37, 44, 45, 47, 64, 65, 67, 74, 75, 77, 94, 97, 104, 107, 114, 117, 124, 127), sont de préférence des champignons (4, 14, 24, 34, 44, 64, 74, 94, 104, 114, 124), des crochets (45, 55, 65, 75), des renfoncements (76) et/ou des éléments de verrouillage (34, 44, 45, 64, 65, 74, 75, 94, 97, 104,, 104, 114, 124) et/ou des éléments de verrouillage complémentaires (37, 47, 67, 77, 97, 107, 117, 127).

3. Matériau de substitution d'os selon l'une des revendications précédentes, **caractérisé en ce que**
les éléments de liaison (4, 14, 24, 34, 37, 44, 45, 47, 55, 64, 65, 67, 74, 75, 76, 77, 94, 97, 104, 107, 114, 117, 124, 127) sont formés sur la surface d'enveloppe des broches (2, 12, 22, 32, 42, 52, 62, 72, 92, 102, 112, 122).

4. Matériau de substitution d'os selon l'une des revendications précédentes, **caractérisé en ce**
**qu'**au moins un des éléments de liaison (4, 14, 24, 34, 37, 44, 45, 47, 55, 64, 65, 67, 74, 75, 76, 77, 94, 97, 104, 107, 114, 117, 124, 127) est conçu de forme tronconique par broche, où les axes longitudinaux des broches (2, 12, 22, 32, 42, 52, 62, 72, 92, 102, 112, 122) forment les axes longitudinaux des cônes et où l'enveloppe et où l'enveloppe des cônes est orientée vers le côté extérieur opposé à structure plane (1, 11, 21, 31, 41, 51, 61, 71, 91, 101, 111, 121) de l'au moins une plaque.

5. Matériau de substitution d'os selon l'une des revendications précédentes, **caractérisé en ce que**
les broches (2, 12, 22, 32, 42, 52, 62, 72, 92, 102, 112, 122) contiennent, entre la structure plane (1, 11, 21, 31, 41, 51, 61, 71, 91, 101, 111, 121) d'une plaque et au moins un des au moins un élément de liaison (4, 14, 24, 34, 44, 45, 55, 64, 65, 74, 75, 76, 94, 97, 104, 107, 114, 117, 124, 127), une rainure (37, 47, 67, 77, 97, 107, 117, 127) circonférentielle servant d'élément de liaison (37, 47, 67, 77, 97, 107, 117, 127) supplémentaire, dans laquelle des éléments de liaison (4, 14, 24, 34, 44, 45, 55, 64, 65, 74, 75, 76, 94, 97, 114, 124) d'autres plaques peuvent s'ancrer ou se verrouiller, de préférence, se verrouiller de telle manière qu'aucun autre mouvement des éléments de liaison (4, 14, 24, 34, 37, 44, 45, 47, 55, 64, 65, 67, 74, 75, 76, 77, 94, 97, 104, 107, 114, 117, 124, 127) le long des broches (2, 12, 22, 32, 42, 52, 62, 72, 92, 102, 112, 122) n'est possible.

6. Matériau de substitution d'os selon l'une des revendications précédentes, **caractérisé en ce**
**qu'**au moins deux éléments de liaison (4, 14, 24, 34, 37, 44, 45, 47, 55, 64, 65, 67, 74, 75, 76, 77, 94, 97, 104, 107, 114, 117, 124, 127) sont disposés l'un derrière l'autre sur la surface d'enveloppe des broches (2, 12, 22, 32, 42, 52, 62, 72, 92, 102, 112, 122), de manière particulièrement préférée qu'au moins trois éléments de liaison (4, 14, 24, 34, 37, 44, 45, 47, 55, 64, 65, 67, 74, 75, 76, 77, 94, 97, 104, 107, 114, 117, 124, 127) sont disposés les uns derrière les autres sur la surface d'enveloppe des broches (2, 12, 22, 32, 42, 52, 62, 72, 92, 102, 112, 122).

7. Matériau de substitution d'os selon l'une des revendications précédentes, **caractérisé en ce que**
des pores (3, 13, 23, 33, 43, 53, 63, 73, 93, 103, 113, 123) traversants sont contenus dans la structure plane (1, 11, 21, 31, 41, 51, 61, 71, 91, 101, 111, 121) de l'au moins une plaque, où la profondeur des pores (3, 13, 23, 33, 43, 53, 63, 73, 93, 103, 113, 123) perpendiculairement par rapport à la structure plane (1, 11, 21, 31, 41, 51, 61, 71, 91, 101, 111, 121) de l'au moins une plaque se situe à au moins 0,25 mm, de préférence à au moins 0,4 mm.

8. Matériau de substitution d'os selon l'une des revendications précédentes, **caractérisé en ce que**
des broches (2, 12, 22, 32, 42, 52, 62, 72, 92, 102, 112, 122) voisines qui sont disposées sur le même côté d'une première plaque de l'au moins une plaque présentent une distance entre elles telle qu'après une déformation élastique en raison d'un ancrage et/ou d'un verrouillage avec un élément de liaison (4, 14, 24, 34, 37, 44, 45, 47, 55, 64, 65, 67, 74, 75, 76, 77, 94, 97, 104, 107, 114, 117, 124, 127) d'une deuxième plaque de l'au moins une plaque, les broches (2, 12, 22, 32, 42, 52, 62, 72, 92, 102, 112, 122) de la première plaque permettent au moins deux ancrages et/ou verrouillages avec au moins deux autres éléments de liaison (4, 14, 24, 34, 37, 44, 45, 47, 55, 64, 65, 67, 74, 75, 76, 77, 94, 97, 104, 107, 114, 117, 124, 127) de la deuxième plaque, de préférence, au moins trois ancrages et/ou verrouillages avec trois autres éléments de liaison (4, 14, 24, 34, 37, 44, 45, 47, 55, 64, 65, 67, 74, 75, 76, 77, 94, 97, 104, 107, 114, 117, 124, 127) de la deuxième plaque.

9. Matériau de substitution d'os selon l'une des revendications précédentes, **caractérisé en ce que**
la plaque, ou au moins l'une des plaques, présente des broches (2, 12, 22, 32, 42, 52, 62, 72, 92, 102, 112, 122) uniquement sur un côté et peut être fixée à plat sur l'autre côté sur un os, où, de préférence, des pointes (39) acérées sont prévues à cet effet qui peut être pressées à l'intérieur dans l'os ou dans la structure plane (1, 11, 21, 31, 41, 51, 61, 71, 91, 101, 111, 121) de l'au moins une plaque, des oeillets ou des perçages sont prévus par lesquels l'au moins une plaque peut être vissée ou autrement fixée sur un os.

10. Matériau de substitution d'os selon l'une des revendications précédentes, **caractérisé en ce que**
l'au moins une plaque ou au moins une de l'au moins une plaque présente des broches (2, 12, 22, 32, 42, 52, 62, 72, 92, 102, 112, 122) sur les deux côtés.

11. Matériau de substitution d'os selon l'une des revendications précédentes, **caractérisé en ce que**
les pores (3, 13, 23, 33, 43, 53, 63, 73, 93, 103, 113, 123) du corps à pores ouverts formé à partir de plusieurs plaques sont interconnectés et ostéoconducteurs, où de préférences les pores (3, 13, 23, 33, 43, 53, 63, 73, 93, 103, 113, 123) présentent une section transversale libre entre 0,1 mm et 1 mm, de manière particulièrement préférée, entre 0,25 mm et 0,9 mm.

12. Matériau de substitution d'os selon l'une des revendications précédentes, **caractérisé en ce que**
les broches (2, 12, 22, 32, 42, 52, 62, 72, 92, 102, 112, 122) sont disposées avec des éléments de liaison (4, 14, 24, 34, 37, 44, 45, 47, 55, 64, 65, 67, 74, 75, 76, 77, 94, 97, 104, 107, 114, 117, 124, 127) en rangées de respectivement trois ou plusieurs broches (2, 12, 22, 32, 42, 52, 62, 72, 92, 102, 112, 122) et qu'entre celles-ci trois ou plusieurs rangées il reste une bande de surface non occupée de l'au moins une plaque et qu'un agencement groupé ou en forme de nid de broches (2, 12, 22, 32, 42, 52, 62, 72, 92, 102, 112, 122) est prévu avec des éléments de liaison (4, 14, 24, 34, 37, 44, 45, 47, 55, 64, 65, 67, 74, 75, 76, 77, 94, 97, 104, 107, 114, 117, 124, 127).

13. Matériau de substitution d'os selon l'une des revendications précédentes, **caractérisé en ce que**
le matériau de substitution d'os présente de manière complémentaire, outre l'au moins une plaque, au moins une particule, l'au moins une particule présentant un noyau et au moins six broches (82) s'étendant à partir du noyau, les broches (82) présentant respectivement au moins un élément de liaison (84, 85) qui est conçu de manière analogue aux éléments de liaison (4, 14, 24, 34, 37, 44, 45, 47, 55, 64, 65, 67, 74, 75, 76, 77, 94, 97, 104, 107, 114, 117, 124, 127) de l'au moins une plaque de sorte que l'au moins une plaque et l'au moins une particule s'ancrent l'une dans l'autre et/ou se verrouillent par une pression des éléments de liaison (4, 14, 24, 34, 37, 44, 45, 47, 55, 64, 65, 67, 74, 75, 76, 77, 94, 97, 104, 107, 114, 117, 124, 127) les uns sur les autres de l'au moins une plaque et de l'au moins une particule, et les plaque(s) et particule ancrées les unes dans les autres et/ou verrouillées forment un corps à pores ouverts à base de plaque(s) et de particule(s) ancrées les unes dans les autres et/ou verrouillées.

14. Procédé de façonnage d'un corps à partir d'un matériau de substitution d'os plan alloplastique avec plusieurs plaques selon l'une des revendications précédentes, chez lequel plusieurs plaques sont pressées les unes contre les autres, où les plaques s'ancrent les unes dans les autres et/ou se verrouillent et forment un corps à pores ouverts.

15. Procédé selon la revendication 14, **caractérisé en ce**
**qu'**au moins une des plaques est liée avec un corps volumique poreux d'un deuxième matériau de substitution d'os **en ce que** les éléments de liaison (4, 14, 24, 34, 37, 44, 45, 47, 55, 64, 65, 67, 74, 75, 76, 77, 94, 97, 104, 107, 114, 117, 124, 127) s'ancrent dans les pores et/ou se verrouillent avec les pores du deuxième matériau de substitution d'os, et/ou au moins une des plaques est liée avec un troisième matériau de substitution d'os particulaire présentant plusieurs particules, où la particule du troisième matériau de substitution d'os présente une multiplicité de broches (82) s'étendant à partir d'un noyau de la particule avec des éléments de liaison (84, 85), où de préférence les broches (82) et les éléments de liaison (8, 85) de la particule du troisième matériau de substitution d'os présentent les caractéristiques des broches (2, 12, 22, 32, 42, 52, 62, 72, 92, 102, 112, 122) et des éléments de liaison (4, 14, 24, 34, 37, 44, 45, 47, 55, 64, 65, 67, 74, 75, 76, 77, 94, 97, 104, 107, 114, 117, 124, 127) de l'au moins une plaque du matériau de substitution d'os selon l'une des revendications 1 à 13.
